(19) Europäisches Patentamt
European Patent Office
Office européen des brevets

(11) EP 4 201 990 A1

(12) **EUROPEAN PATENT APPLICATION**
published in accordance with Art. 153(4) EPC

(43) Date of publication:
**28.06.2023 Bulletin 2023/26**

(21) Application number: **21858387.0**

(22) Date of filing: **19.08.2021**

(51) International Patent Classification (IPC):
***C08K 5/3492*** (2006.01) ***C08L 23/00*** (2006.01)
***C08L 101/00*** (2006.01) ***C07D 251/30*** (2006.01)

(52) Cooperative Patent Classification (CPC):
**C07D 251/30; C08K 5/3492; C08L 23/00; C08L 101/00**

(86) International application number:
**PCT/JP2021/030479**

(87) International publication number:
**WO 2022/039244 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2020 JP 2020140377**

(71) Applicant: **ADEKA CORPORATION**
**Arakawa-ku**
**Tokyo**
**116-8554 (JP)**

(72) Inventors:
- **MUTO Takashi**
  **Saitama-shi, Saitama 336-0022 (JP)**
- **FUKUI Satoshi**
  **Saitama-shi, Saitama 336-0022 (JP)**
- **UEDA Naoto**
  **Saitama-shi, Saitama 336-0022 (JP)**

(74) Representative: **Gill Jennings & Every LLP**
**The Broadgate Tower**
**20 Primrose Street**
**London EC2A 2ES (GB)**

(54) **COMPOUND, ADDITIVE FOR SYNTHETIC RESIN, ADDITIVE COMPOSITION FOR SYNTHETIC RESIN, RESIN COMPOSITION, AND MOLDED ARTICLE OF SAME**

(57) Provided are a compound, an additive for synthetic resins, an additive composition for synthetic resins, a resin composition, and a molded article thereof, which can improve the properties of synthetic resins. A compound containing a monovalent group represented by general formula (1) can improve the properties of synthetic resins.

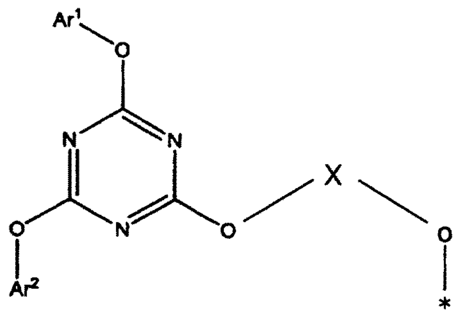

(1)

In general formula (1), X represents a divalent group, $Ar^1$ and $Ar^2$ each independently represent an unsubstituted or substituted phenyl group, and * represents a site bonding to another atom.

EP 4 201 990 A1

## Description

TECHNICAL FIELD

The present invention relates to a compound, an additive for synthetic resins, an additive composition for synthetic resins, a resin composition, and a molded article thereof, and in particular, to a compound, an additive for synthetic resins, an additive composition for synthetic resins, a resin composition, and a molded article thereof, which can improve the properties of synthetic resins.

BACKGROUND ART

A variety of compounds are used as additives for synthetic resins to improve the properties of synthetic resins. For example, in Patent Document 1, a triphenyloxytriazine is proposed as a compound used as an additive for synthetic resins, and such compounds are indicated to be a fluidity modifier for thermoplastic resins.

RELATED ART DOCUMENTS

PATENT DOCUMENT

[PATENT DOCUMENT 1] JP S61-14261

SUMMARY OF THE INVENTION

PROBLEMS TO BE SOLVED BY THE INVENTION

However, the compounds described in Patent Document 1 may not sufficiently improve the properties of synthetic resins, and there was room for further improvement.

Accordingly, an object of the present invention is to provide a compound, an additive for synthetic resins, an additive composition for synthetic resins, a resin composition, and a molded article thereof, which can improve the properties of synthetic resins.

MEANS FOR SOLVING THE PROBLEMS

The present inventors intensely studied to solve the above-described problem, and found that a compound having a specific structure can solve the problem, thereby completing the present invention.

Specifically, the compound of the present invention is a compound containing a monovalent group represented by general formula (1):

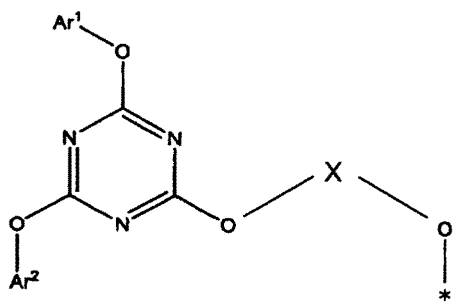

(1)

(where X represents a divalent group, $Ar^1$ and $Ar^2$ each independently represent an unsubstituted or substituted phenyl group, and * represents a site bonding to another atom).

The compound of the present invention is preferably a compound represented by general formula (2):

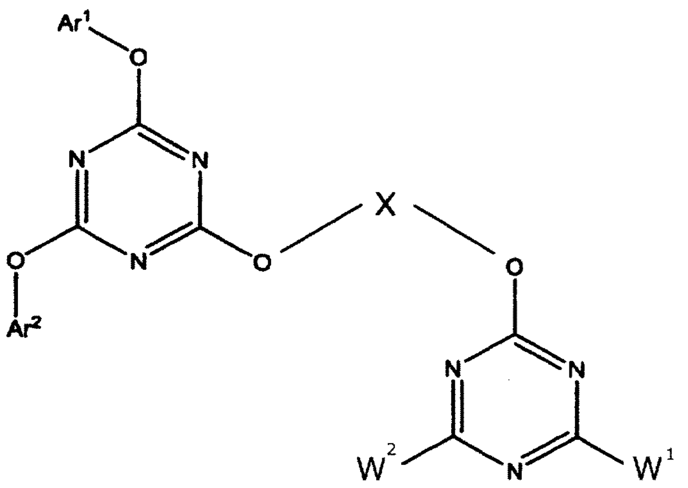

(2)

(where X represents a divalent group, $Ar^1$ and $Ar^2$ each independently represent an unsubstituted or substituent-containing phenyl group, and $W^1$ and $W^2$ each independently represent an unsubstituted or substituted phenyloxy group, or a monovalent group represented by general formula (1)), and more preferably a compound represented by general formula (3):

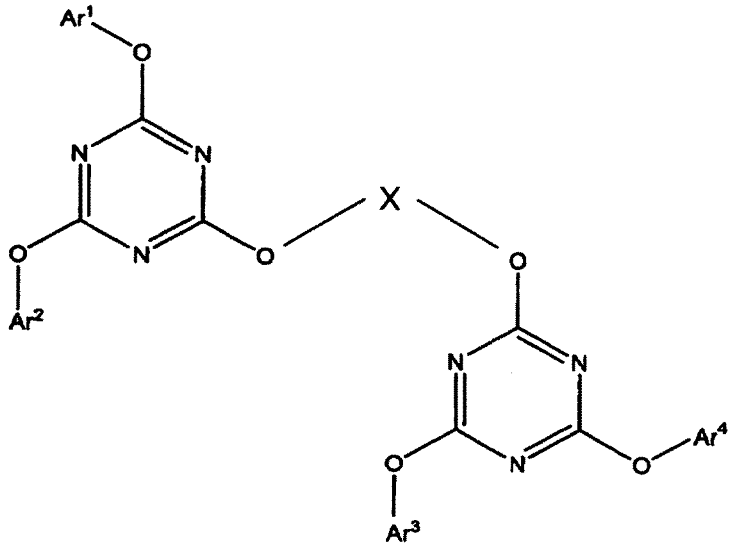

(3)

(where X represents a divalent group, and $Ar^1$, $Ar^2$, $Ar^3$, and $Ar^4$ each independently represent an unsubstituted or substituted phenyl group). Here, preferably, X is a group represented by general formula (5) or (6):

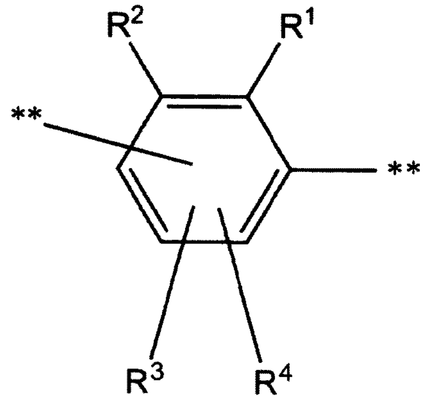

(5)

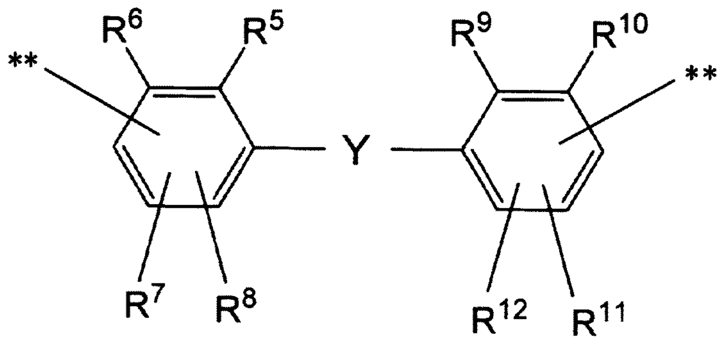

(6)

(where ** represents a site bonding to an oxygen atom, $R^1$ to $R^{12}$ each independently represent a hydrogen atom, an alkyl group having from 1 to 10 carbon atoms, an alkoxy group having from 1 to 10 carbon atoms, or a halogen atom, and Y represents a single bond, an oxygen atom, a sulfur atom, a sulfonyl group, or an unsubstituted or substituted alkanediyl group). More preferably, X is a group represented by general formula (6), and Y is a single bond.

The compound of the present invention may be a compound represented by general formula (4):

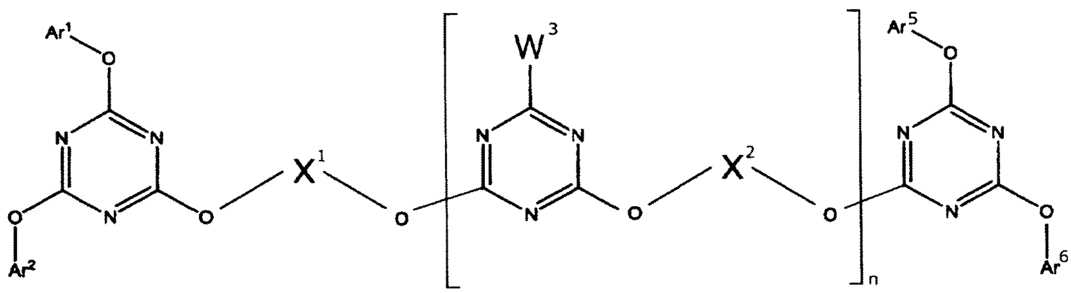

(4)

(where $X^1$ and $X^2$ each independently represent a divalent group, $Ar^1$, $Ar^2$, $Ar^5$, and $Ar^6$ each independently represent an unsubstituted or substituted phenyl group, $W^3$ represents an unsubstituted or substituted phenyloxy group or a monovalent group represented by general formula (1), and n represents an integer of 1 or more). Here, preferably, $X^1$ and $X^2$ are each independently a group represented by general formula (5) or (6):

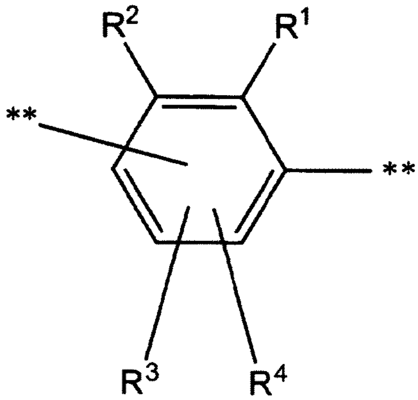

(5)

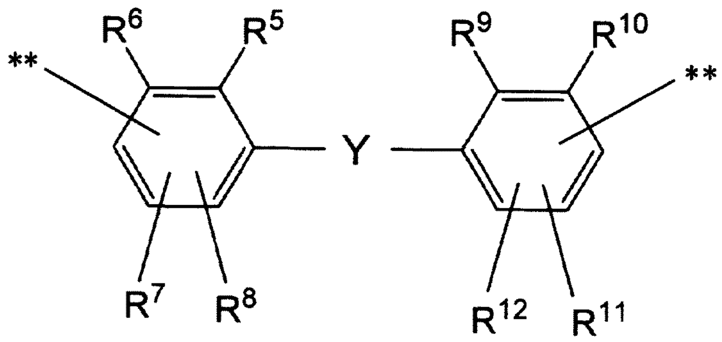

(6)

(where ** represents a site bonding to an oxygen atom, $R^1$ to $R^{12}$ each independently represent a hydrogen atom, an alkyl group having from 1 to 10 carbon atoms, an alkoxy group having from 1 to 10 carbon atoms, or a halogen atom, and Y represents a single bond, an oxygen atom, a sulfur atom, a sulfonyl group, or an unsubstituted or substituted alkanediyl group). More preferably, $X^1$ and $X^2$ are each independently a group represented by general formula (6), and Y is a single bond.

The additive for synthetic resins of the present invention is an additive for synthetic resins containing the compound of the present invention.

The additive for synthetic resins of the present invention is preferably a nucleating agent.

Furthermore, the additive composition for synthetic resins of the present invention is an additive composition for synthetic resins, containing the additive for synthetic resins of the present invention.

The additive composition for synthetic resins of the present invention is preferably an additive composition for synthetic resins containing another nucleating agent in addition to the additive agent for synthetic resins, and more preferably the additive composition for synthetic resins wherein the other nucleating agent contains a compound represented by general formula (7):

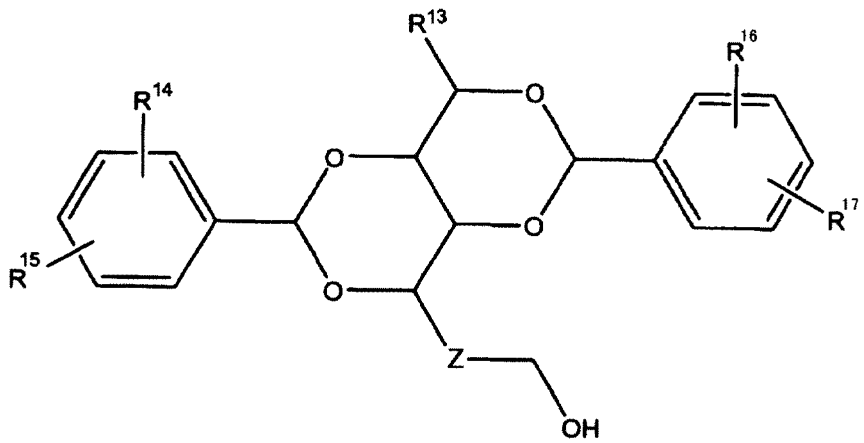

(7)

(where $R^{13}$ represents a hydrogen atom or an alkyl group having from 1 to 10 carbon atoms, $R^{14}$ to $R^{17}$ each independently represent a hydrogen atom, a halogen atom, a cyano group, an alkyl group having from 1 to 10 carbon atoms, or an alkoxy group having from 1 to 10 carbon atoms, or $R^{14}$ and $R^{15}$ or $R^{16}$ and $R^{17}$ are linked together to represent an alkylene group having from 3 to 6 carbon atoms or an alkylenedioxy group having from 1 to 4 carbon atoms, and Z represents a single bond, a -CH(OH)- group, or a -CH(OH)CH(OH)- group.).

The additive composition for synthetic resins of the present invention is preferably an additive composition for synthetic resins containing a lubricant, and is more preferably the additive composition for synthetic resins wherein the lubricant contains at least one selected from the group consisting of a fatty acid ester and a fatty acid amide.

Furthermore, the additive composition for synthetic resins of the present invention is preferably an additive composition for synthetic resins containing a colorant.

Still further, the resin composition of the present invention is a resin composition containing a synthetic resin and the additive for synthetic resins of the present invention.

The resin composition of the present invention may be a resin composition containing a synthetic resin and the additive composition for synthetic resins of the invention.

The resin composition of the present invention is preferably a resin composition wherein the synthetic resin contains a polyolefin resin, and is more preferably the resin composition wherein the polyolefin resin contains at least one selected from the group consisting of a polyethylene resin and a polypropylene resin. The resin composition of the present invention is preferably a resin composition wherein the synthetic resin contains an elastomer.
Furthermore, the molded article of the present invention is a molded article obtained by molding the resin composition of the present invention.
The molded article of the present invention is preferably the molded article wherein an a* value and a b* value of a transmitted color, when measured with a colorimeter, satisfy $-7 \leq a^* \leq 1$ and $-1 \leq b^* \leq 5$.

EFFECTS OF THE INVENTION

The present invention can provide a compound, an additive for synthetic resins, an additive composition for synthetic resins, a resin composition, and a molded article thereof, which can improve the properties of synthetic resins.

MODE FOR CARRYING OUT THE INVENTION

The following is a detailed description of embodiments of the present invention. First, the compound of the present embodiment will be described.

< Compound >

The compound of the present embodiment contains a monovalent group represented by general formula (1):

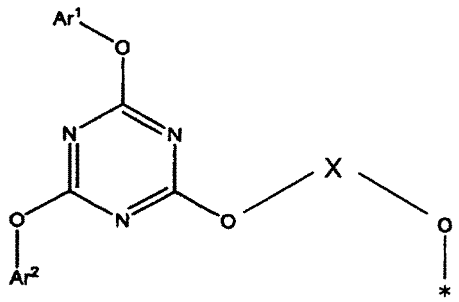

(1)

In general formula (1), X represents a divalent group, and $Ar^1$ and $Ar^2$ each independently represent an unsubstituted or substituted phenyl group. Note that * represents a site bonded to another atom.
The compound of the present embodiment can improve the properties of synthetic resins.
Examples of the compound containing a monovalent group represented by general formula (1) include a compound represented by general formula (2) below, an oligomer or polymer represented by general formula (4) below, and a dendrimer represented by general formula (8) below. Among them, a compound represented by general formula (2) is preferable. The compound containing a monovalent group represented by general formula (1) may be an oligomer or polymer represented by general formula (4).

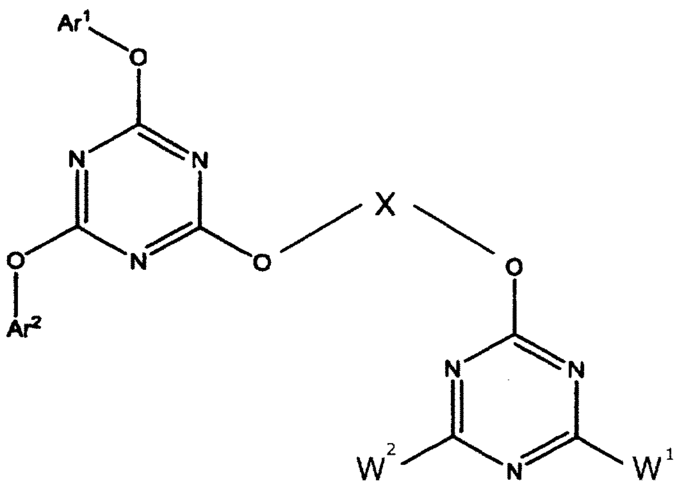

(2)

In general formula (2), X represents a divalent group, $Ar^1$ and $Ar^2$ each independently represent an unsubstituted or substituted phenyl group, and $W^1$ and $W^2$ each independently represent an unsubstituted or substituted phenyloxy group or a monovalent group represented by general formula (1).

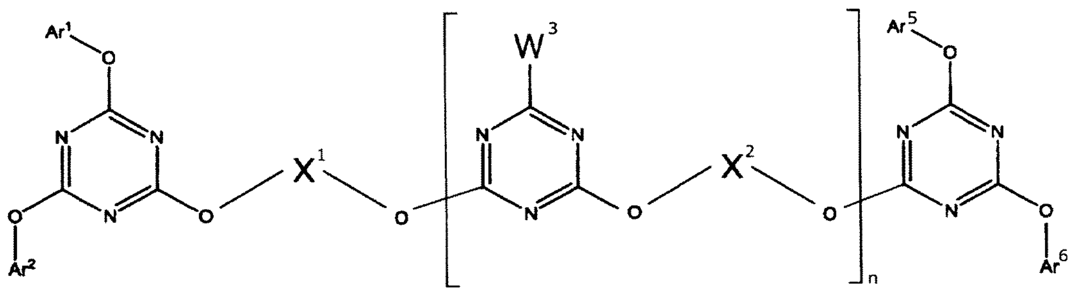

(4)

In general formula (4), $X^1$ and $X^2$ each independently represent a divalent group, $Ar^1$, $Ar^2$, $Ar^5$, and $Ar^6$ each independently represent an unsubstituted or substituted phenyl group, $W^3$ represents an unsubstituted or substituted phenyloxy group, or a monovalent group represented by general formula (1), and n is an integer of 1 or more.

In general formula (4), n may be, for example, 500 or less, and is preferably 100 or less, more preferably 50 or less, further preferably 10 or less, and still further preferably 5 or less. n is preferably 2 or more. Furthermore, n is particularly preferably 2. In general formula (4), $W^3$ is preferably an unsubstituted or substituted phenyloxy group.

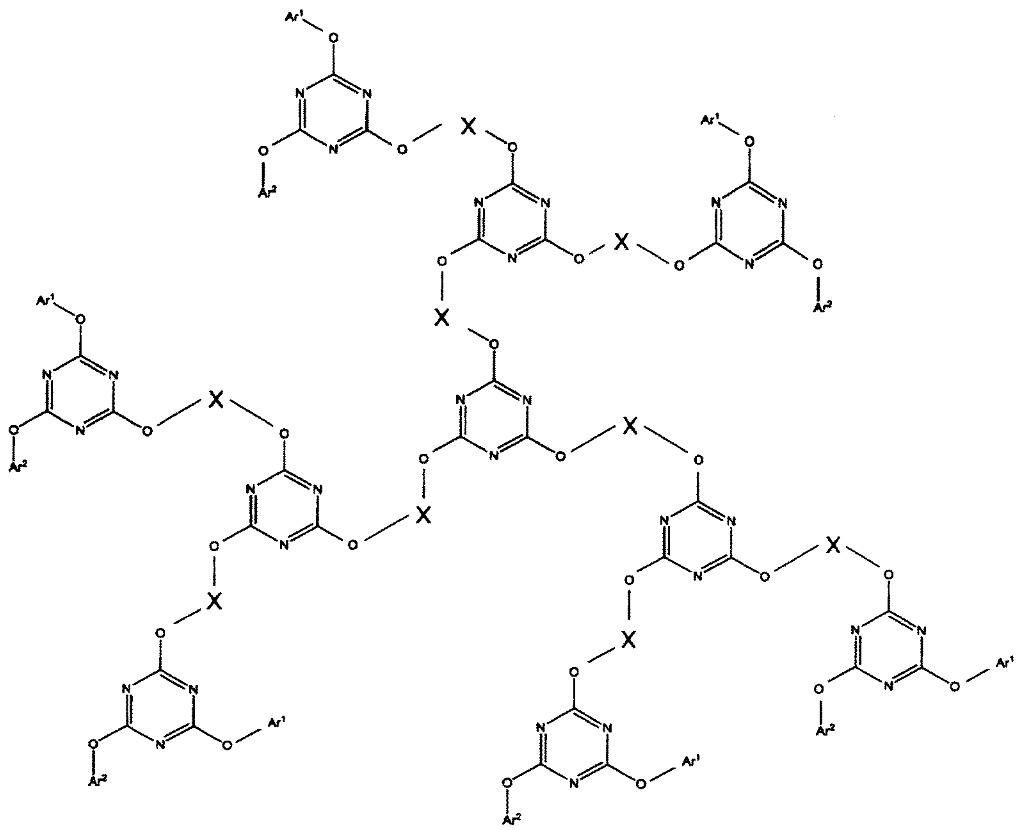

(8)

In general formula (8), X represents a divalent group, and $Ar^1$ and $Ar^2$ each independently represent an unsubstituted or substituted phenyl group.

Examples of the compound represented by general formula (2) include a compound represented by general formula (3) below, a compound represented by general formula (9) below, and a compound represented by general formula (10) below. Among them, a compound represented by general formula (3) is preferable.

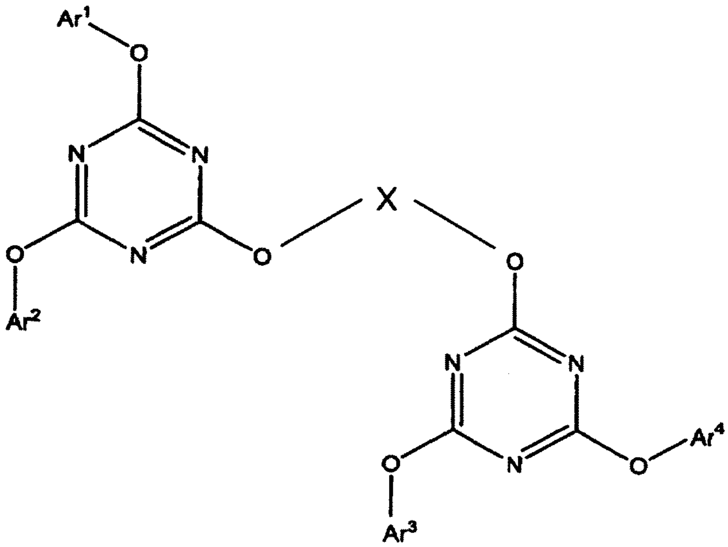

(3)

In general formula (3), X represents a divalent group, and $Ar^1$, $Ar^2$, $Ar^3$, and $Ar^4$ each independently represent an unsubstituted or substituted phenyl group.

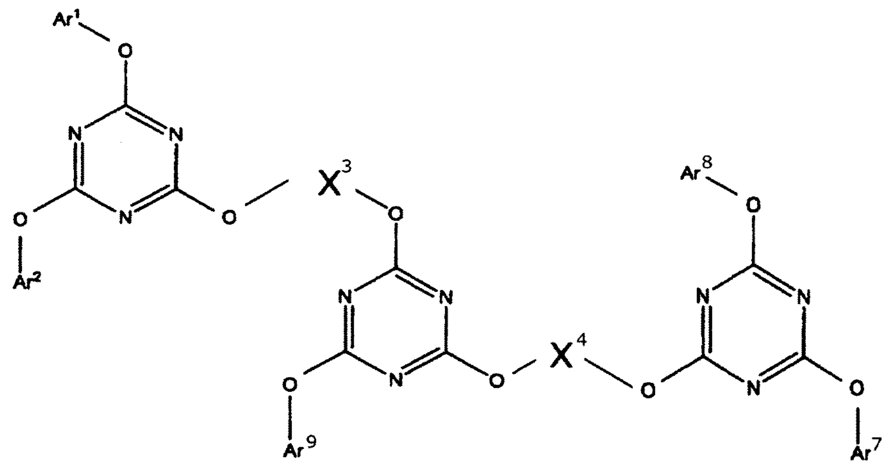

(9)

In general formula (9), $X^3$ and $X^4$ each independently represent a divalent group, and $Ar^1$, $Ar^2$, $Ar^7$, $Ar^8$, and $Ar^9$ each independently represent an unsubstituted or substituted phenyl group.

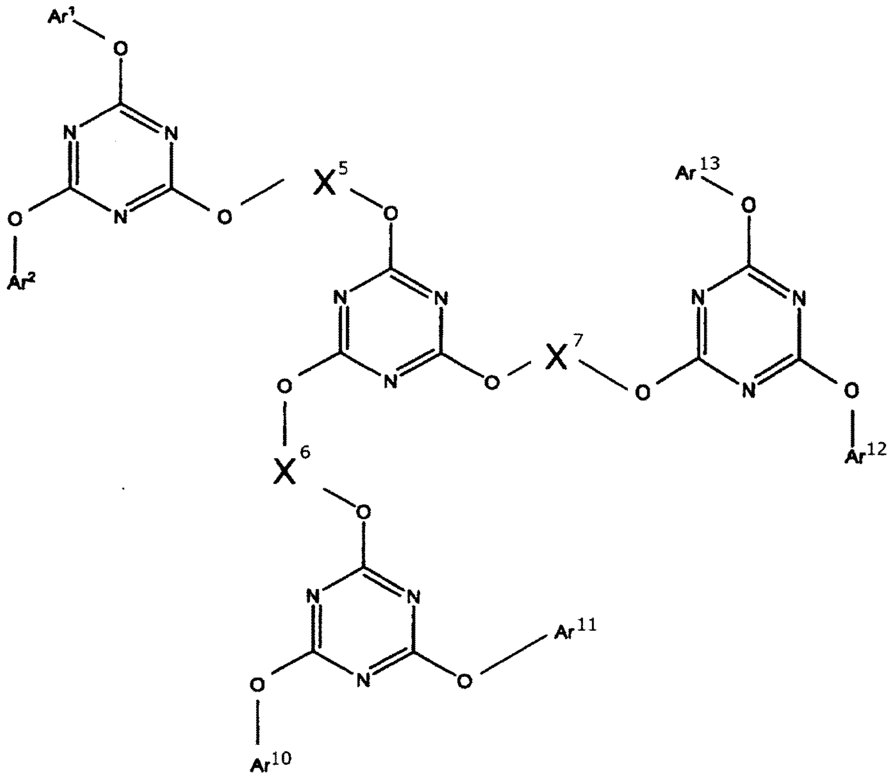

(10)

In general formula (10), $X^5$, $X^6$, and $X^7$ each independently represent a divalent group, and $Ar^1$, $Ar^2$, $Ar^{10}$, $Ar^{11}$, $Ar^{12}$, and $Ar^{13}$ each independently represent an unsubstituted or substituted phenyl group.

Examples of X and $X^1$ to $X^7$ include: an alkylene group such as a methylene group, an ethylene group, a propylene group, or a butylene group; an alkylidene group such as an ethylidene group, a propylidene group, or a butylidene group; an arylene group such as a phenylene group, a naphthylene group, an anthracylene group, a phenanthrylene group, a biphenylylene group, or a terphenylylene group; an alkylenearylene group such as a methylenephenylene group or a methylenebiphenylylene group; an alkylene arylenealkylene group such as a methylene phenylene methylene group or a methylene biphenylene methylene group; an arylenealkylene alkylene group such as a phenylenemethylenephenylene group or a biphenylenemethylenebiphenylenephenylenephenylenemethylene group; and an arylenealkylidenearylene group such as a phenyleneethylidenephenylene group or a phenylenemethylethylidenephenylene group. These groups may be unsubstituted or may have a substituent.

Examples of the substituent when X and $X^1$ to $X^7$ contain a substituent include an alkyl group having from 1 to 10 carbon atoms, an alkoxy group having from 1 to 10 carbon atoms, an alkyl carbonyl group having from 2 to 11 carbon

atoms, an aryl group having from 6 to 20 carbon atoms, an aryloxy group having from 6 to 20 carbon atoms, an aryl carbonyl group having from 7 to 21 carbon atoms, a heterocyclic group having from 2 to 20 carbon atoms, an amino group, an aminocarbonyl group, a halogen atom, a hydroxy group, a nitro group, a cyano group, a formyl group, a carboxy group, a sulfo group, a sulfonamide group. Here, the carboxy group and the sulfo group may form a salt.

Examples of the alkyl group having from 1 to 10 carbon atoms include: a linear alkyl group such as a methyl group, an ethyl group, an *n*-propyl group, an *n*-butyl group, an *n*-pentyl group, an *n*-hexyl group, an *n*-heptyl group, an *n*-octyl group, an *n*-nonyl group, or an *n*-decyl group; an alkyl group with a branch such as an isopropyl group, an isobutyl group, a *sec*-butyl group, a *tert*-butyl group, an isopentyl group, a neopentyl group, a *tert*-amyl group, a 2-heptyl group, a *tert*-heptyl group, a *tert*-octyl group, an isononyl group, or an isodecyl group; and a cyclic alkyl group such as a cyclopentyl group, a cyclohexyl group, a cyclooctyl group, or an adamantyl group.

Examples of the alkoxy group having from 1 to 10 carbon atoms include a group having a structure in which the alkyl group having from 1 to 10 carbon atoms is bonded to an oxygen atom.

Examples of the alkyl carbonyl group having from 2 to 11 carbon atoms include a group having a structure in which the alkyl group having from 1 to 10 carbon atoms is bonded to a carbonyl group.

Examples of the aryl group having from 6 to 20 carbon atoms include an unsubstituted aryl group such as a phenyl group, an *o*-biphenylyl group, an *m*-biphenyl group, a *p*-biphenyl group, $\alpha$-naphthyl group, a $\beta$-naphthyl group, a 1-anthryl group, a 2-anthryl group, a 9-anthryl group, a 1-phenanthryl group, a 2-phenanthryl group, a 3-phenanthryl group, a 4-phenanthryl group, or a 9-phenanthryl group; and a substituted aryl group such as a *p*-methylphenyl group, an *o*-methylphenyl group, a *p*-*tert*-butylphenyl group, a *p*-methoxyphenyl group, a *p*-chlorophenyl group, a *p*-nitrophenyl group, or a *p*-cyanophenyl group.

Examples of the aryloxy group having from 6 to 20 carbon atoms include a group having a structure in which the aryl group having from 6 to 20 carbon atoms is bonded to an oxygen atom.

Examples of the aryl carbonyl group having from 7 to 21 carbon atoms include a group having a structure in which the aryl group having from 6 to 20 carbon atoms is bonded to a carbonyl group.

Examples of the heterocyclic group having from 2 to 20 carbon atoms include a pyridyl group, a pyrimidyl group, a furyl group, a thienyl group, a tetrahydrofuryl group, a dioxolanyl group, a benzoxazol-2-yl group, a tetrahydropyranyl group, a pyrrolidyl group, an imidazolidyl group, a pyrazolidyl group, a thiazolidyl group, an isothiazolidyl group, an oxazolidyl group, an isoxazolidyl group, a piperidyl group, a piperazyl group, a morpholinyl group.

The amino group is a group having a structure of $-NA^1A^2$. Here, $A^1$ and $A^2$ each independently represent a hydrogen atom, an alkyl group having from 1 to 10 carbon atoms, or an aryl group having from 6 to 20 carbon atoms. $A^1$ and $A^2$ may be linked together to form a ring. Examples of an alkyl group having from 1 to 10 carbon atoms and an aryl group having from 6 to 20 carbon atoms include those exemplified as substituents in cases in which X has a substituent.

Examples of the aminocarbonyl group include a group having a structure in which the amino group is bonded to a carbonyl group.

Examples of the halogen atom include fluorine, chlorine, bromine, and iodine.

Examples of the substituents when $Ar^1$ to $Ar^{13}$ are substituted phenyl groups include those exemplified as substituents when X and $X^1$ to $X^7$ have substituents.

Examples of the substituents when $W^1$ to $W^3$ are substituted phenyloxy groups also include those exemplified as substituents when X and $X^1$ to $X^7$ are substituted.

In the compound of the present embodiment, X is preferably a group represented by general formula (5) or (6) below, and is more preferably a group represented by general formula (6).

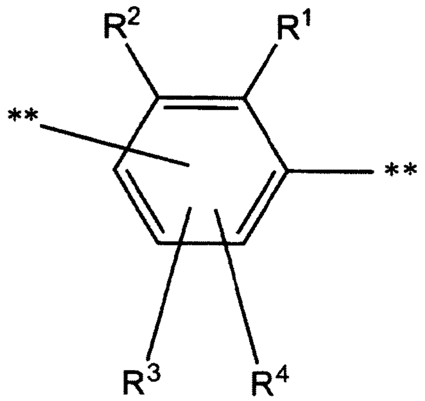

(5)

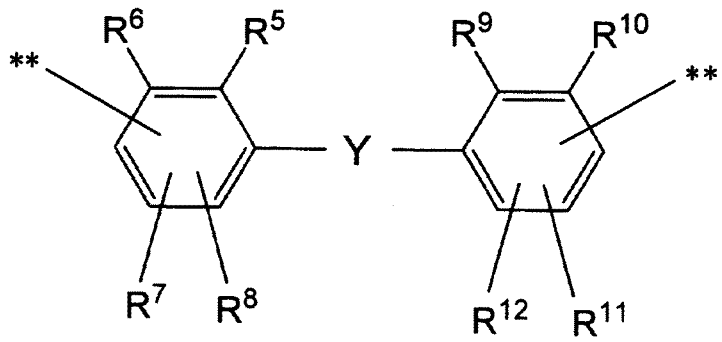

(6)

In general formulas (5) and (6), ** represents a site bonding to an oxygen atom, $R^1$ to $R^{12}$ each independently represent a hydrogen atom, an alkyl group having from 1 to 10 carbon atoms, an alkoxy group having from 1 to 10 carbon atoms, or a halogen atom, and Y represents a single bond, an oxygen atom, a sulfur atom, a sulfonyl group, or an unsubstituted or substituted alkanediyl group.

In the compound of the present embodiment, $X^1$ and $X^2$ are preferably groups represented by general formula (5) or (6), and are more preferably groups represented by general formula (6).

Furthermore, in the compound of the present embodiment, $X^3$ to $X^7$ are preferably groups represented by general formula (5) or (6), and are more preferably groups represented by general formula (6).

Examples of the alkyl group having from 1 to 10 carbon atoms, an alkoxy group having from 1 to 10 carbon atoms, and a halogen atom represented by $R^1$ to $R^{12}$ include those exemplified as substituents in cases in which X is substituted.

Examples of the alkanediyl group represented by Y include an alkylene group such as a methylene group, an ethylene group, a propylene group, or a butylene group; and an alkylidene group such as an ethylidene group, a propane-1-ylidene group, a propane-2-ylidene group, a butane-1-ylidene group, a butane-2-ylidene group, or a cyclohexylidene group. Here, the number of carbon atoms of the alkanediyl group may be, for example, from 1 to 10, and is preferably from 1 to 6. Examples of a substituent when an alkanediyl group is substituted include those exemplified as substituents in cases in which X is substituted.

In the compound of the present embodiment, when X and $X^1$ to $X^7$ are groups represented by general formula (5), X and $X^1$ to $X^7$ are preferably groups represented by general formula (5') below. When X and $X^1$ to $X^7$ are groups represented by general formula (6), X and $X^1$ to $X^7$ are preferably groups represented by general formula (6') below. Furthermore, $R^1$ to $R^{12}$ are preferably hydrogen atoms or alkyl groups having 1 to 10 carbon atoms, and are more preferably hydrogen atoms. Furthermore, Y is preferably a single bond. In the compound of the present embodiment, X and $X^1$ to $X^7$ may be groups represented by general formula (5), and Y may be a single bond.

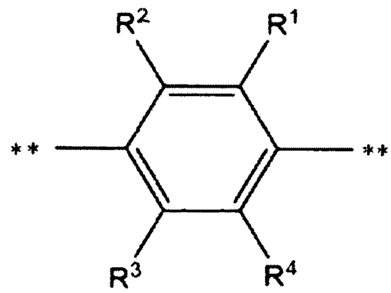

(5')

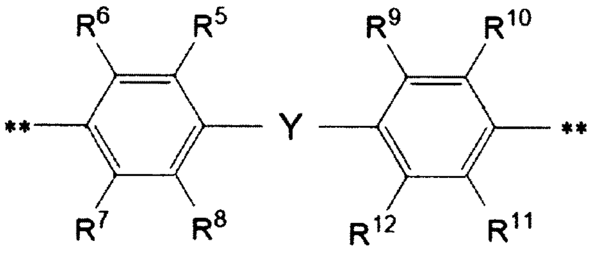

(6')

In the compounds of the present embodiment, Ar1 to Ar13 are preferably a 2-methylphenyl group, a 3-methylphenyl group, a 4-methylphenyl group, a 2-ethylphenyl group, a 3-ethylphenyl group, a 4-ethylphenyl group, a 2-*n*-propylphenyl group, a 3-*n*-propylphenyl group, a 4-*n*-propylphenyl group, a 2-*t*-butylphenyl group, a 3-*t*-butylphenyl group, a 4-*t*-butylphenyl group, a 2,3-dimethylphenyl group, a 3,4-dimethylphenyl group, a 2,3-dichlorophenyl group, a 3,4-dichlorophenyl group, a 2-cyclohexylphenyl group, a 3-cyclohexylphenyl group, a 4-cyclohexylphenyl group, a 2 biphenyl group, a 3-biphenyl group or a 4-biphenyl group, more preferably a 4-ethylphenyl group, a 4-*n*-propylphenyl group, a 4-*t*-butylphenyl group, a 3,4-dimethylphenyl group, a 3,4-dichlorophenyl group, a 4-cyclohexylphenyl group, or a 4-biphenylyl group, further preferably a 4-cyclohexylphenyl group or a 4-biphenylyl group, and particularly preferably a 4-cyclohexylphenyl group.

Specific examples of the compound containing a monovalent group represented by general formula (1) include the following compounds, but the compounds of the present embodiment are not limited to these specific examples.

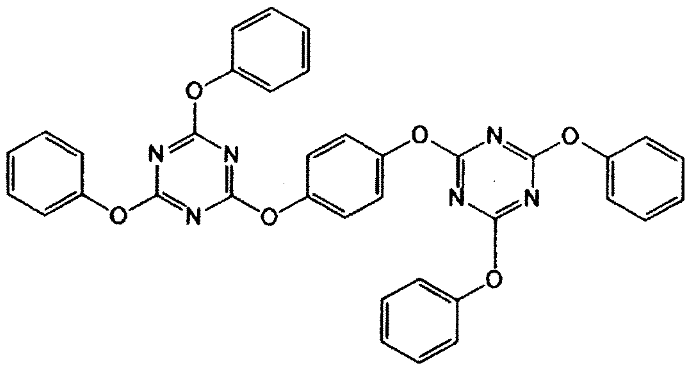

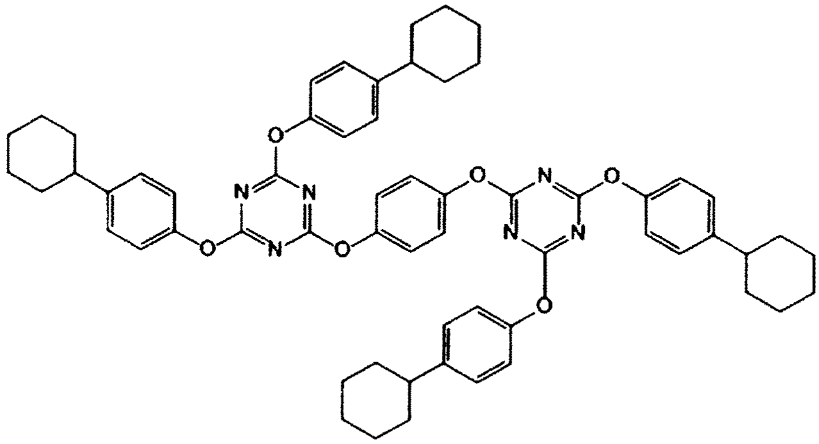

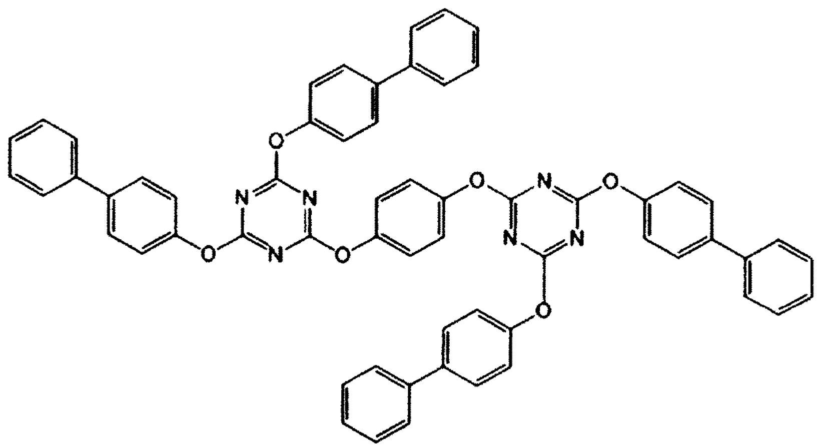

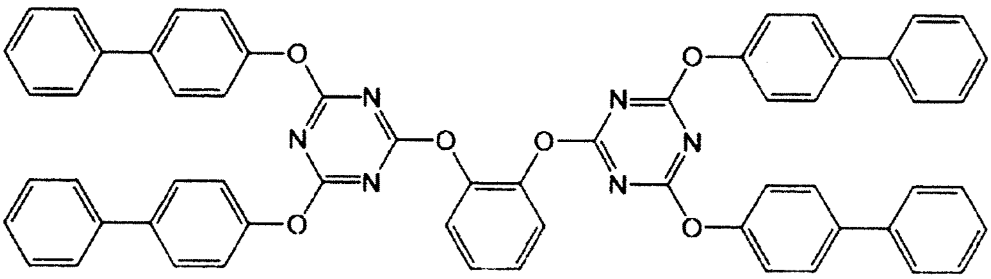

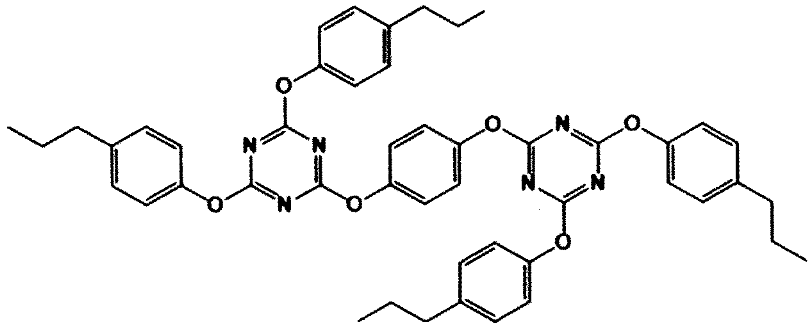

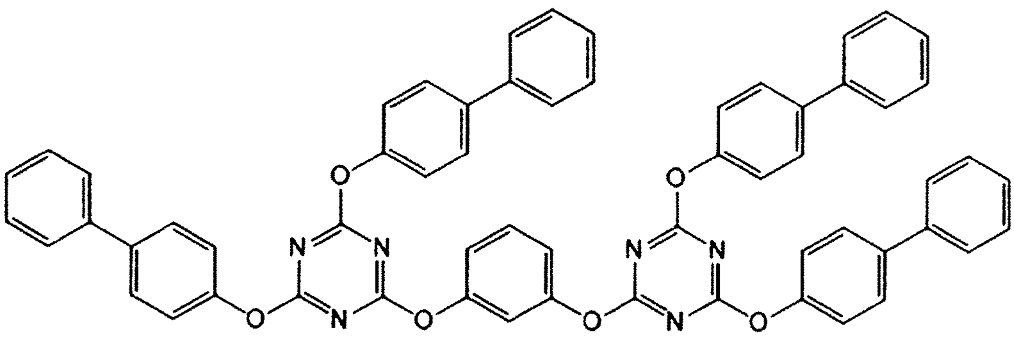

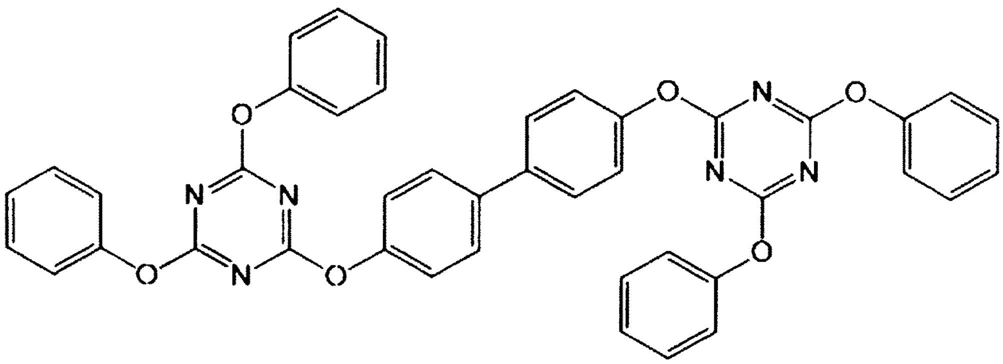

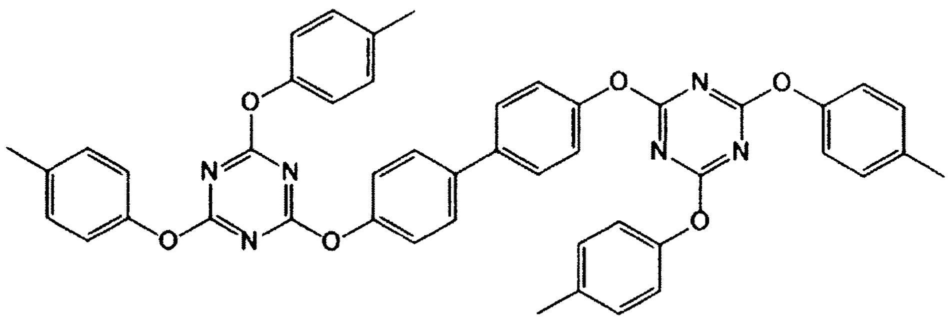

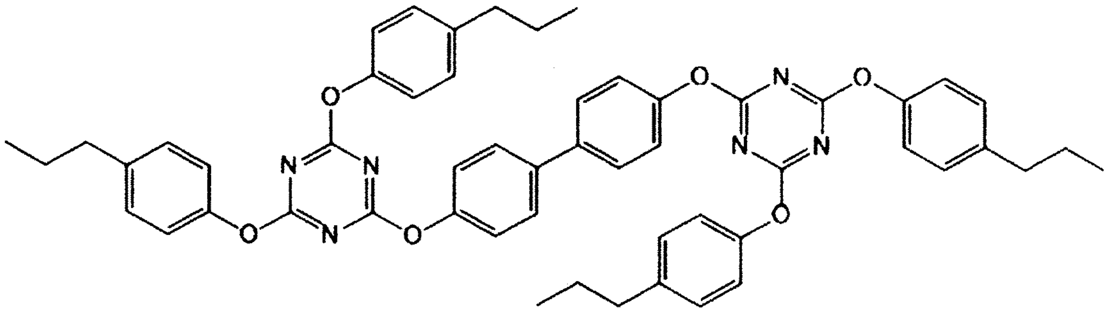

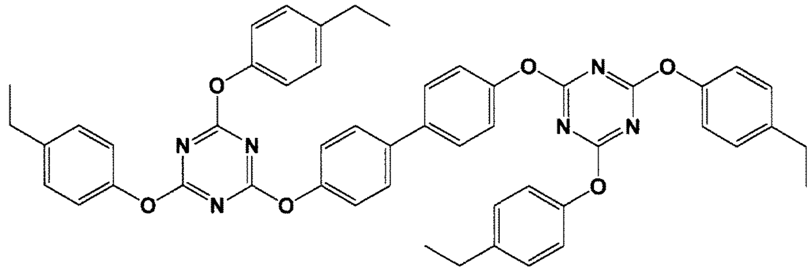

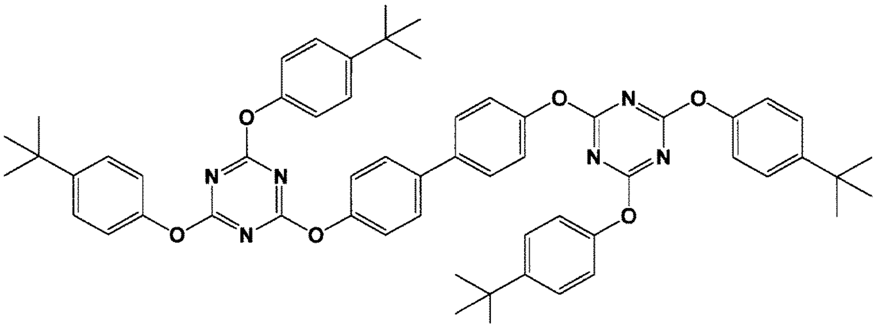

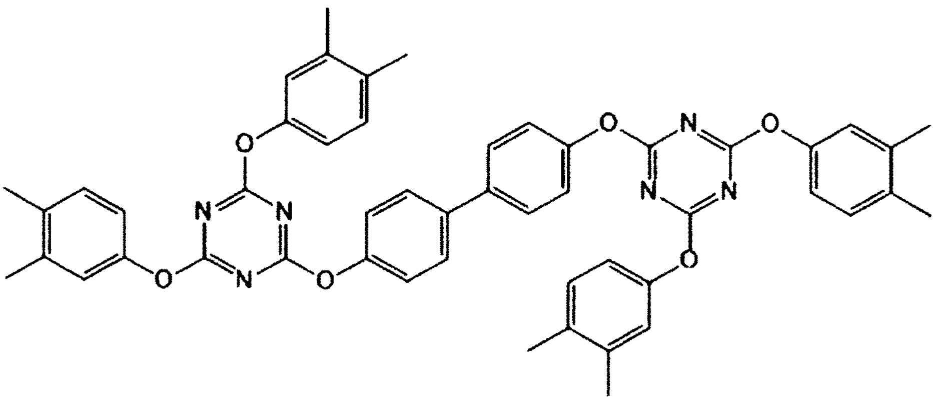

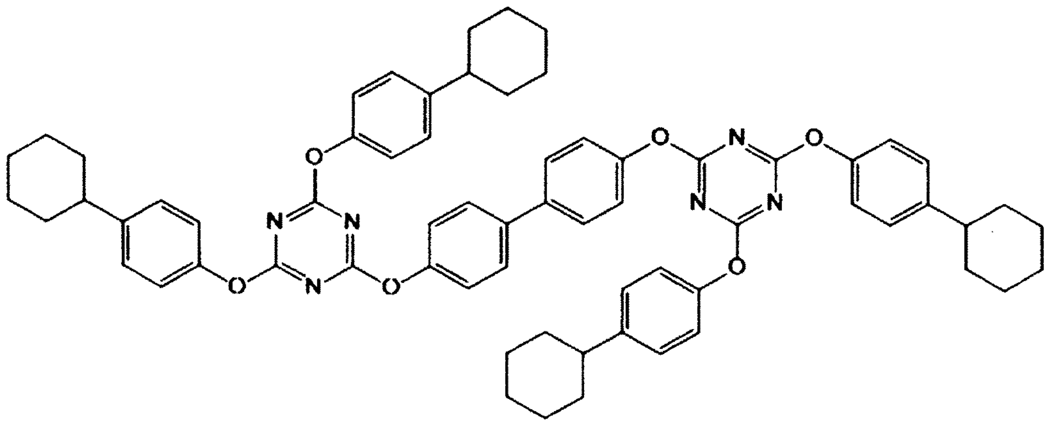

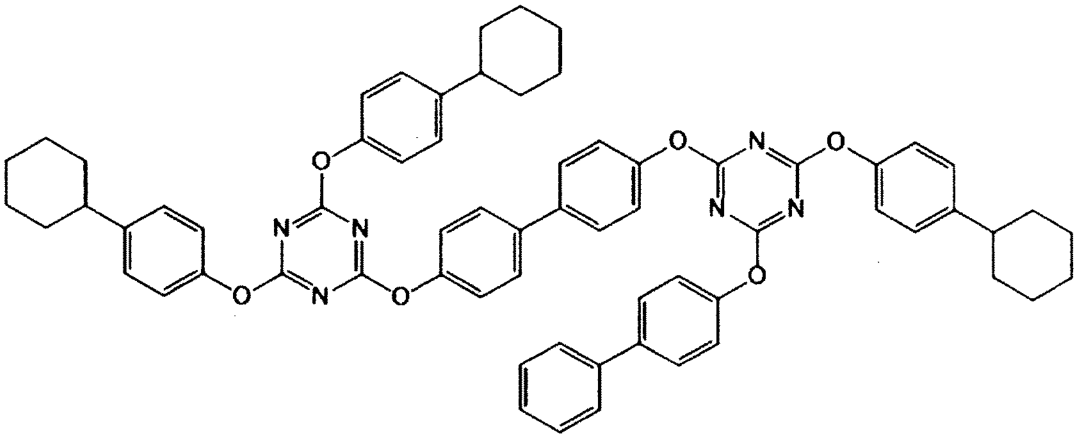

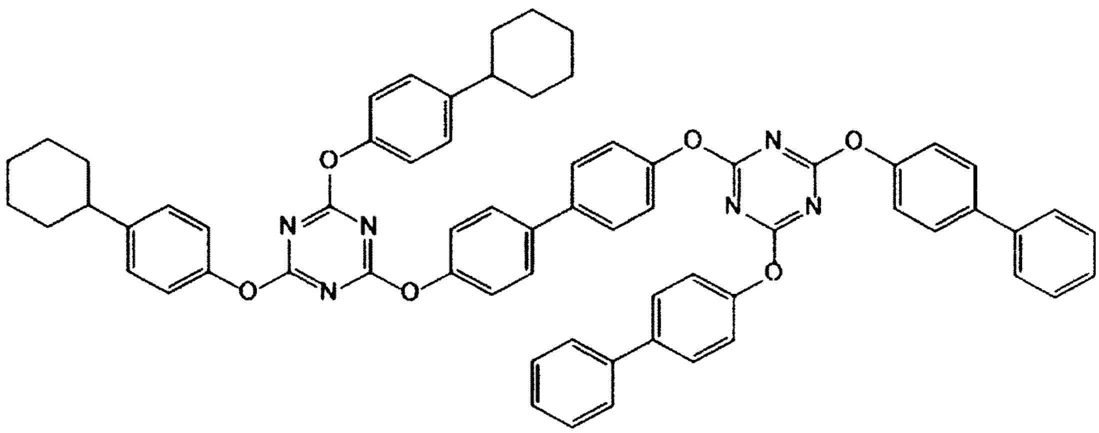

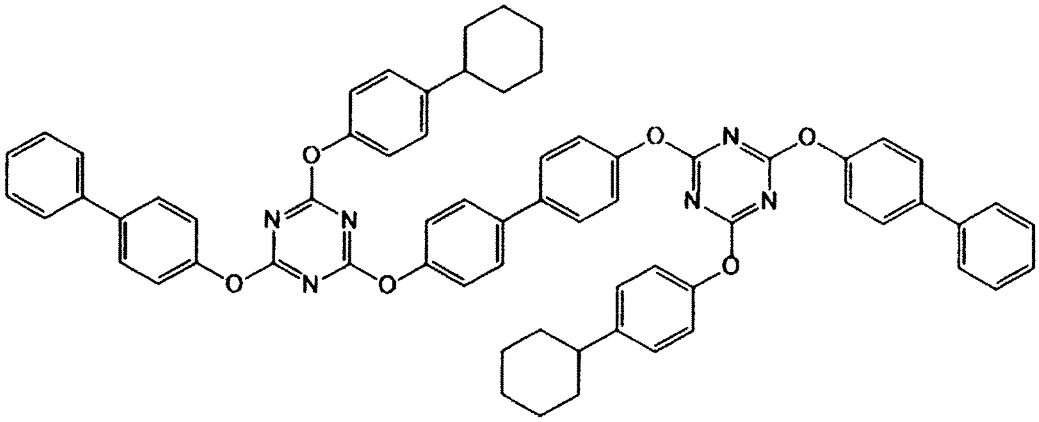

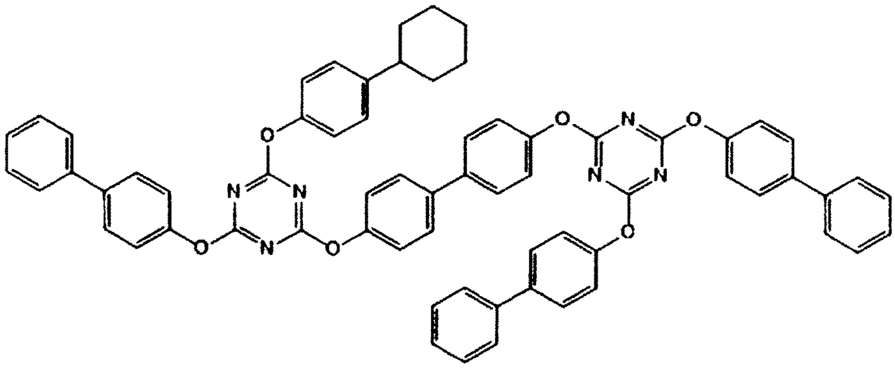

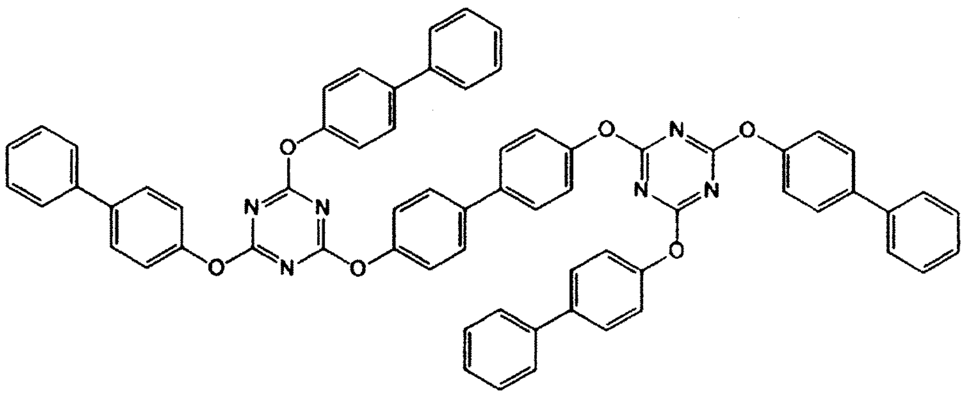

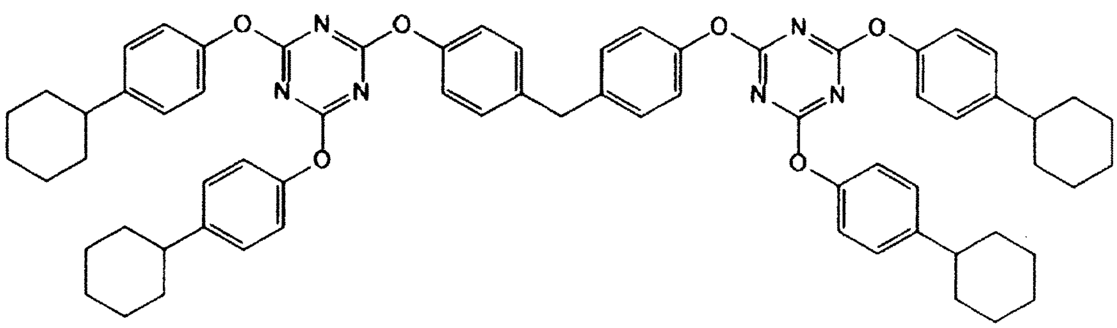

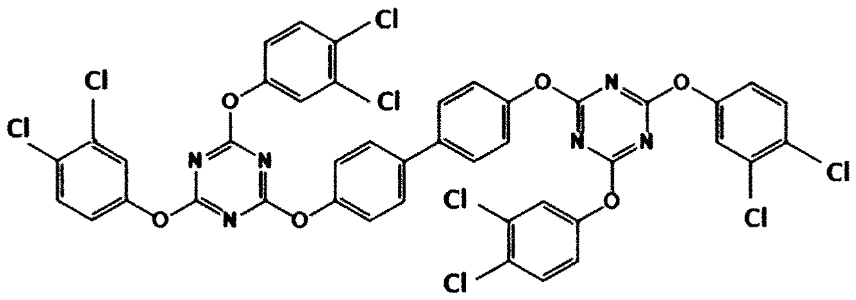

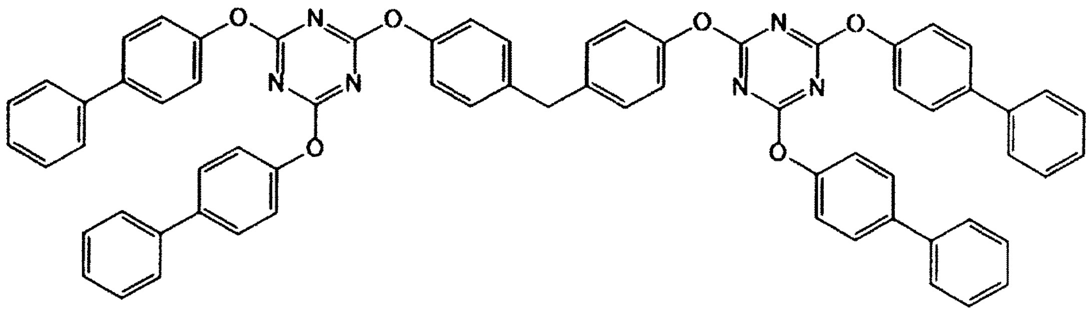

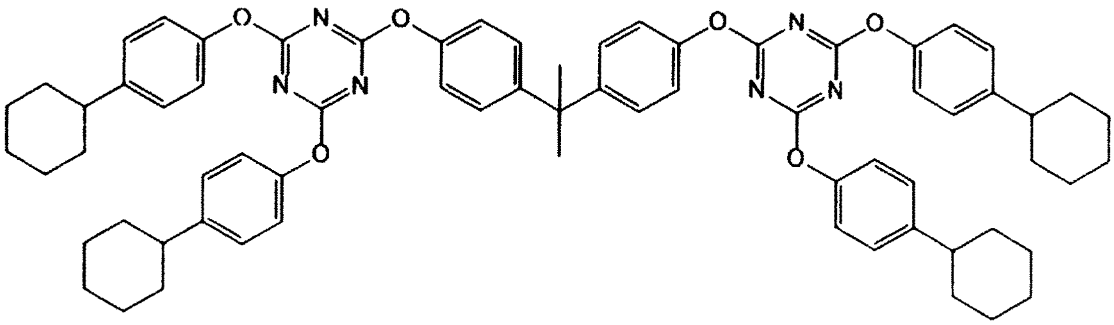

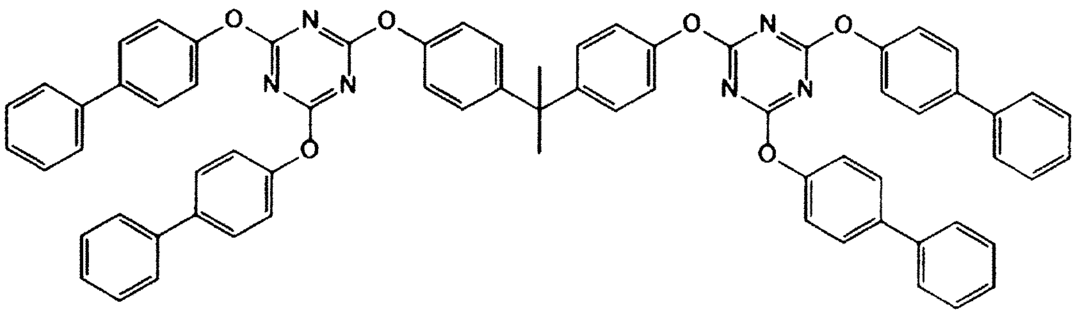

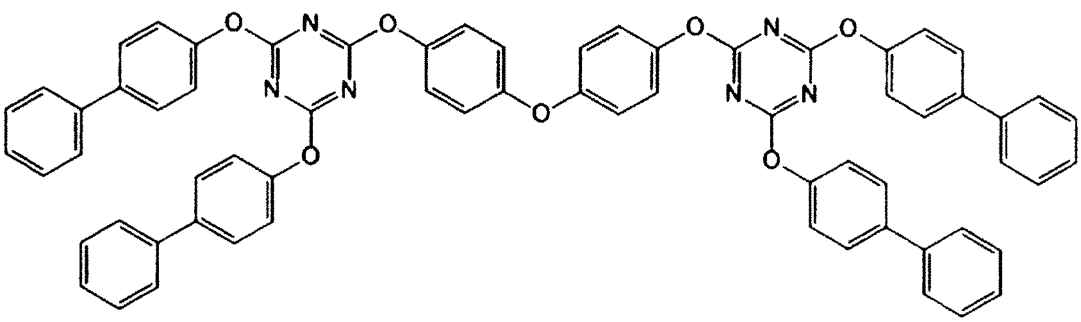

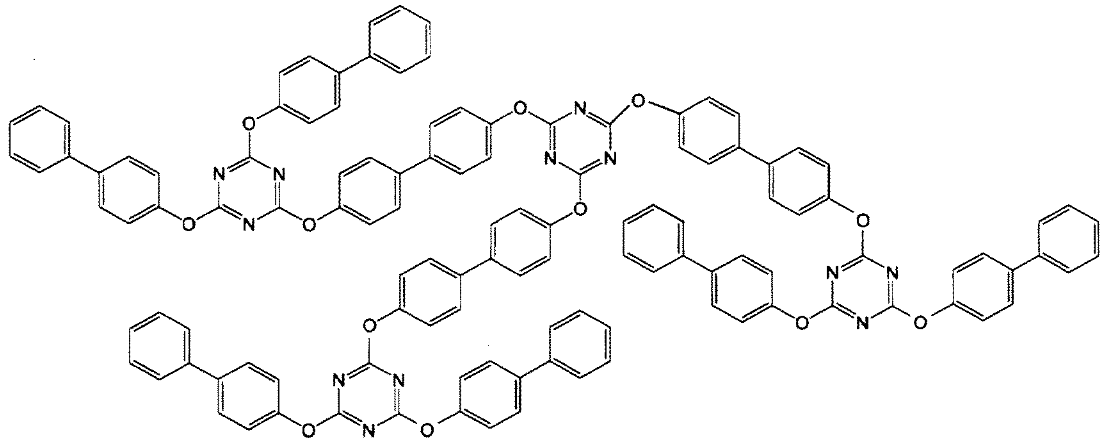

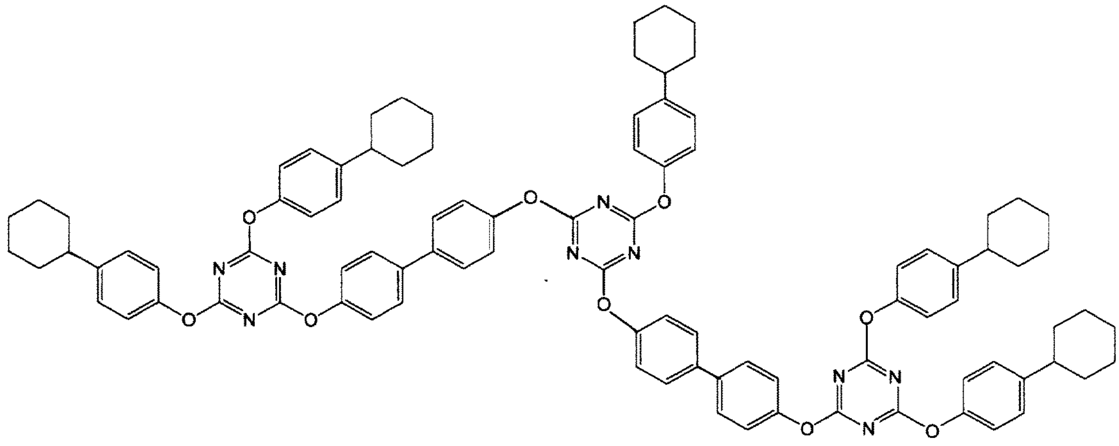

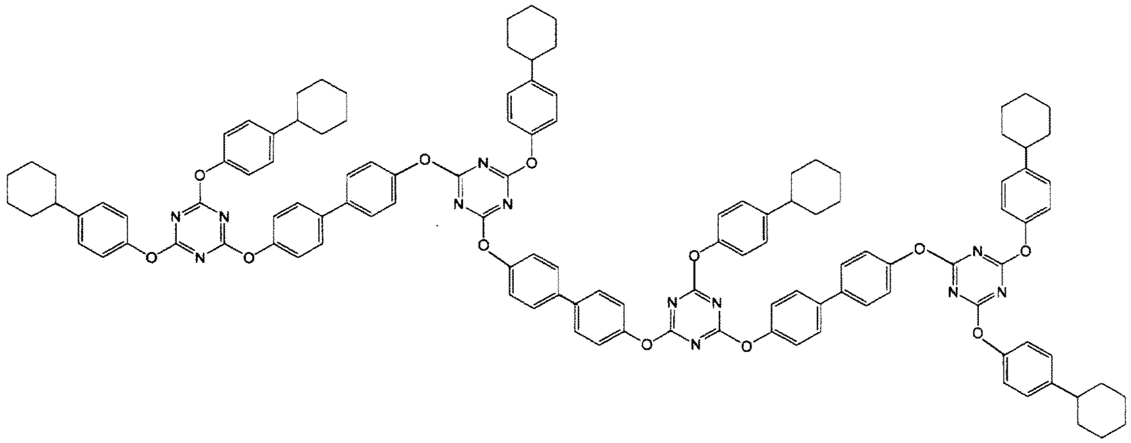

A compound containing a monovalent group represented by general formula (1) can be produced by a combination of known synthetic methods. For example, a compound represented by general formula (3) can be produced by a method of preparing a first intermediate by sequentially reacting one equivalent of each of $Ar^1$-OH, $Ar^2$-OH, and HO-X-OH for one equivalent of cyanuric acid chloride in the presence of a base such as triethylamine or sodium hydroxide, then preparing a second intermediate by sequentially reacting one equivalent each of $Ar^3$-OH and $Ar^4$-OH for one equivalent of cyanuric acid chloride in the presence of a base, and further reacting one equivalent of the first intermediate with one equivalent of the second intermediate in the presence of a base.

The compound of the present embodiment can be used as an additive for synthetic resins or oils, and among these applications, the compound can be particularly suitably used as an additive for synthetic resins.

Next, the additive for synthetic resins of the present form will be described.

< Additive for Synthetic Resins >

The additive for synthetic resins of the present embodiment is composed of a compound containing a monovalent group represented by general formula (1).

The additive for synthetic resins of the present embodiment can improve the properties of synthetic resins.

The additive for synthetic resins of the present embodiment can be used specifically as a nucleating agent, an antioxidant, an ultraviolet absorber, a thickening agent, a filler, an electrical conductor, an anti-wear agent, a light stabilizer, or a metal deactivating agent (copper damage inhibitor). The additive for synthetic resins of the present embodiment is particularly suitable for use as a nucleating agent because the additive is remarkably effective in promoting crystallization of a crystalline resin as a nucleating agent. Here, examples of the nucleating agent include a nucleating agent for polyolefin resins, such as a nucleating agent for polyethylene resins or a nucleating agent for polypropylene resins, a nucleating agent for polyamide resins, a nucleating agent for polyester resins, a nucleating agent for polyacetal, a nucleating agent for polylactic acid, and a nucleating agent for polyphenylene sulfide. The additive for synthetic resins of the present embodiment is preferably used as a nucleating agent for polyolefin resins, is more preferably used as a nucleating agent for polyethylene resins or polypropylene resins, and is further preferably used as a nucleating agent for polypropylene resins.

The additive for synthetic resins of the present embodiment can be, for example, in particle form. When the additive for synthetic resins of the present embodiment is in particle form, the powder property values of the additive for synthetic resins, such as average particle size, angle of repose, loose bulk density, packed bulk density, and degree of compressibility, may be such that the dispersibility of the additive for synthetic resins in a synthetic resin and the fluidity of the particles are at the desired degree. Specifically, the average particle size may be, for example, from 0.1 to 100 $\mu$m, the angle of repose may be, for example, from 20 to 70°, the loose bulk density may be, for example, from 0.1 to 0.8 g/cm3, the packed bulk density may be, for example, from 0.2 to 1 g/cm$^3$, and the compression ratio may be, for example, from 1 to 10. The average particle size is the average particle size calculated from the particle size distribution measured by the laser diffraction method according to JIS Z 8825, the angle of repose is the angle of repose measured by the cylindrical rotation method, the loose bulk density is the bulk density measured according to JIS K 5101-12-1, the packed bulk density is the bulk density measured in accordance with JIS K 5101-12-2, and the compression ratio is a value calculated by the following equation:

$$(\text{compression ratio}) = (\text{packed bulk density})/(\text{loose bulk density}).$$

The additive for synthetic resins of the present embodiment may be a one-pack composite additive that is further blended and granulated with a granulation aid such as a binder, a wax, a solvent, or silica. The additive for synthetic resins of the present embodiment may be a master batch that further contains a synthetic resin.

The synthetic resin contained in the master batch is not particularly limited, and may be a thermoplastic resin or a thermosetting resin. Examples of the thermoplastic resin include a crystalline resin such as a polyolefin resin, a polyamide resin, a polyester resin, a polyacetal resin, a polylactic acid, or a polyphenylene sulfide, an amorphous resin such as a polycarbonate resin, a styrene resin, an acrylic resin, a urethane resin, a halogen-containing resin, a petroleum resin, a coumarone resin, polyvinyl alcohol, polyvinyl acetate, and a polyphenylene oxide, and a thermoplastic elastomer. Examples of the thermosetting resin include a phenol resin, a urea resin, a melamine resin, an epoxy resin, an unsaturated polyester resin, and a synthetic rubber. One type of synthetic resin may be contained singly, or two or more types of synthetic resins may be contained in combination. The synthetic resin may be a copolymer or a polymer alloy.

The content of a synthetic resin in a master batch of the total master batch, for example, may be 99.9% by mass or less, preferably 90% by mass or less, more preferably 80% by mass or less, and still more preferably 60% by mass or less. The content of the synthetic resin in the master batch may be, for example, 10% by mass or more of the total master batch.

Next, the additive composition for synthetic resins of the present embodiment will be described.

< Additive Composition for Synthetic Resin >

The additive composition for synthetic resins of the present embodiment contains the above-described additive for synthetic resins.

According to the additive composition for synthetic resins of the present embodiment, the properties of synthetic resins can be improved.

The additive composition for synthetic resins of the present embodiment preferably contains another nucleating agent in addition to the additive agent for synthetic resins described above. Here, the other nucleating agent is an additive agent that promotes crystallization of a synthetic resin, and is composed of a compound other than a compound containing

a monovalent group represented by general formula (1).

Examples of the other nucleating agent include a compound represented by general formula (7) below; an aromatic phosphate metal salt such as sodium 2,2'-methylenebis(4,6-di-*tert*-butylphenyl)phosphate, lithium 2,2'-methylenebis(4,6-di-*tert*-butylphenyl)phosphate, dihydroxyaluminum 2,2'-methylenebis(4,6-di-*tert*-butylphenyl)phosphate, or hydroxyaluminum bis[2,2'-methylenebis(4,6-di-*tert*-butylphenyl)phosphate]; a carboxylic acid metal salt such as sodium benzoate, 4-*tert*-butylbenzoate aluminum salt, sodium adipate, disodium bicyclo[2.2.1]heptane-2,3-dicarboxylate, or calcium cyclohexane-1,2-dicarboxylate; an amide compound such as *N,N',N''-tris*[2-methylcyclohexyl]-1,2,3-propane tricarboxamide, *N,N',N''*-tricyclohexyl-1,3,5-benzene tricarboxamide, or *N,N'*-dicyclohexylnaphthalene dicarboxamide, 1,3,5-tris[(2,2-dimethylpropanoylamino)]benzene; and 2,4,6-tri(aryloxy)-1,3,5-triazine compounds described in WO2020/067144, other than compounds containing a monovalent group represented by general formula (1).

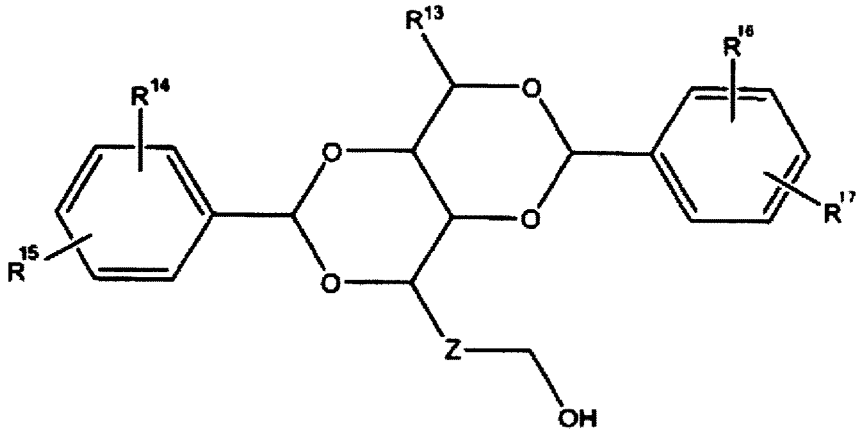

(7)

In general formula (7), $R^{13}$ represents a hydrogen atom or an alkyl group having from 1 to 10 carbon atoms. $R^{14}$ to $R^{17}$ each independently represent a hydrogen atom, a halogen atom, a cyano group, an alkyl group having from 1 to 10 carbon atoms or an alkoxy group having from 1 to 10 carbon atoms, or $R^{14}$ and $R^{15}$ or $R^{16}$ and $R^{17}$ are linked together to represent an alkylene group having from 3 to 6 carbon atoms or an alkylenedioxy group having from 1 to 4 carbon atoms. Z represents a single bond, a -CH(OH)- group, or a -CH(OH)CH(OH)-group.

Examples of the alkyl group having from 1 to 10 carbon atoms represented by $R^{13}$ to $R^{17}$, the alkoxy group having from 1 to 10 carbon atoms represented by $R^{14}$ to $R^{17}$, and the halogen atom represented by $R^{14}$ to $R^{17}$ include those exemplified as substituents in cases in which X is substituted in general formula (1). As the alkyl group having from 1 to 10 carbon atoms, an alkyl group having from 1 to 4 carbon atoms is preferable. As the alkoxy group having from 1 to 10 carbon atoms, an alkoxy group having from 1 to 4 carbon atoms is preferable.

Examples of the alkylene group having from 3 to 6 carbon atoms in general formula (7) include a propylene group, a butylene group, a pentylene group, and a hexylene group. Examples of the alkylenedioxy group having from 1 to 4 carbon atoms include a methylenedioxy group, an ethylenedioxy group, a propylenedioxy group, and a butylenedioxy group.

In the additive composition for synthetic resins of the present embodiment, as the compound represented by general formula (7), one in which $R^{13}$ to $R^{17}$ in general formula (7) are each independently a hydrogen atom or an alkyl group having from 1 to 4 carbon atoms and Z is a -CH(OH)- group is preferable.

Specific examples of the compound represented by general formula (7) include dibenzylidene sorbitol, bis(*p*-methylbenzylidene)sorbitol, bis(*p*-ethylbenzylidene)sorbitol, bis(3,4-dimethylbenzylidene)sorbitol, 1,2,3-trideoxy-4,6:5,7-*o*-bis(4-propylbenzylidene)nonitol. From the viewpoint of further improving the properties of a synthetic resin, among them, dibenzylidene sorbitol, bis(p-methylbenzylidene)sorbitol, bis(3,4-dimethylbenzylidene)sorbitol, or 1,2,3-trideoxy-4,6:5,7-o-bis(4-propylbenzylidene)nonitol is preferable, and bis(3,4-dimethylbenzylidene)sorbitol or 1,2,3-trideoxy-4,6:5,7-o-bis(4-propylbenzylidene)nonitol is more preferable.

A method of producing a compound represented by general formula (7) includes, for example, dehydration condensation of an alditol compound such as sorbitol with an aryl aldehyde in the presence of an acid catalyst.

Among the compounds represented by general formula (7), examples of those commercially available include: GEL ALL D (trade name), GEL ALL MD (trade name), GEL ALL DXR (trade name), GEL ALL E-200 (trade name), GEL ALL MD-LM30G (trade name), RiKAFAST P1 (trade name) manufactured by New Japan Chemical Co., Ltd.; and Millad 3988 (trade name), Millad 3988i (trade name), Millad NX-8000 (trade name), Millad NX-8000J (trade name) manufactured by Milliken & Company.

In the additive composition for synthetic resins of the present embodiment, the other nucleating agent preferably contains at least one selected from the group consisting of an aromatic phosphate metal salt, a carboxylic acid metal

salt, and a compound represented by general formula (7), and more preferably contains a compound represented by general formula (7).

When the additive composition for synthetic resins of the present embodiment contains another nucleating agent in addition to the above-described additive agent for synthetic resins, the ratio B/C of the content B (parts by mass) of the additive agent for synthetic resins to the content C (parts by mass) of the other nucleating agent in the additive composition for synthetic resins may be, for example, from 1/99 to 99/1. From the viewpoint of further improving the properties of a synthetic resin, the value of B/C is preferably 3/97 or higher, and is more preferably 5/95 or higher. From a similar viewpoint, the value of B/C is preferably 50/50 or less, and is more preferably 40/60 or less.

In the present embodiment, when the additive agent for synthetic resins contained in the additive composition for synthetic resins is a nucleating agent and the additive composition for synthetic resins contains another nucleating agent in addition to the additive agent for synthetic resins, the additive composition for synthetic resins can provide excellent transparency to a molded article made of a synthetic resin.

The additive composition for synthetic resins of the present embodiment preferably contains a lubricant.

Examples of the lubricant include a fatty acid ester, a fatty acid amide, a fatty acid, a higher alcohol, and a sugar alcohol. When the additive composition for synthetic resins of the present embodiment contains a lubricant, the lubricant preferably contains at least one selected from the group consisting of a fatty acid ester and a fatty acid amide.

Examples of the fatty acid ester include: a fatty acid alkyl ester such as methyl fatty acid or ethyl fatty acid; an alkylene glycol fatty acid monoester such as an ethylene glycol fatty acid monoester or a propylene glycol fatty acid monoester; an alkylene glycol fatty acid diester such as an ethylene glycol fatty acid diester or a propylene glycol fatty acid diester; a glycerol fatty acid ester such as a glycerol fatty acid monoester, a glycerol fatty acid diester, or a glycerol fatty acid triester; and a pentaerythritol fatty acid ester such as a pentaerythritol fatty acid monoester, a pentaerythritol fatty acid diester, a pentaerythritol fatty acid triester, or a pentaerythritol fatty acid tetraester.

The fatty acid residue constituting the fatty acid ester may be, for example, a fatty acid residue having from 7 to 29 carbon atoms. Here, the fatty acid residue refers to a group obtained from a fatty acid by excluding a carboxyl group. The number of carbon atoms of the fatty acid residue is preferably from 11 to 23, and more preferably from 13 to 21. Examples of the fatty acid residue include a group obtained by removing a carboxyl group from a fatty acid such as lauric acid, myristic acid, palmitic acid, stearic acid, oleic acid, linoleic acid, linolenic acid, arachidic acid, arachidonic acid, behenic acid, erucic acid, lignoceric acid, serotinic acid, montanic acid, melicic acid, 12-hydroxystearic acid, or ricinoleic acid. Among them, a group obtained by removing a carboxyl group from lauric acid, myristic acid, palmitic acid, or stearic acid is preferable, and a group obtained by removing a carboxyl group from stearic acid is particularly preferable.

In the additive composition for synthetic resins of the present embodiment, a fatty acid ester preferably includes a glycerol fatty acid monoester. Examples of the glycerol fatty acid monoester include a glycerol laurate monoester, a glycerol myristate monoester, a glycerol palmitate monoester, a glycerol stearate monoester, a glycerol oleate monoester, a glycerol linoleate monoester, a glycerol linolenate monoester, a glycerol arachidic acid monoester, a glycerol arachidonic acid monoester, a glycerol behenic acid monoester, a glycerol erucic acid monoester, a glycerol lignoceric acid monoester, a glycerol serotinic acid monoester, a glycerol monomontanoic acid monoester, a glycerol melissic acid monoester, a glycerol 12-hydroxystearic acid monoester, or a glycerol ricinoleic acid monoester. Among them, a glycerol laurate monoester, a glycerol myristate monoester, a glycerol palmitate monoester, or a glycerol stearate monoester is preferable, and a glycerol stearate monoester is particularly preferable.

Examples of the fatty acid amide include a fatty acid monoamide, an alkylene bis-fatty acid amide, an alkylol fatty acid amide, and an *N*-alkyl fatty acid amide. Among them, a fatty acid monoamide or an alkylene bis-fatty acid amide is preferable.

Examples of the fatty acid residue constituting the fatty acid amide include those exemplified as the above-described fatty acid residue constituting the fatty acid ester.

Specific examples of the fatty acid monoamide include a lauric acid amide, a myristic acid amide, a palmitic acid amide, a stearic acid amide, an oleic acid amide, a linoleic acid amide, a linolenic acid amide, an arachidic acid amide, an arachidonic acid amide, a behenic acid amide, an erucic acid amide, a lignoserynic acid amide, a serotinic acid amide, a montanic acid amide, a melicic acid amide, a 12-hydroxystearic acid amide, and a ricinoleic acid amide. Among them, a lauric acid amide, a myristic acid amide, a palmitic acid amide, a stearic acid amide, an oleic acid amide, or an erucic acid amide is preferable, a stearic acid amide, an oleic acid amide, or an erucic acid amide is more preferable, and an oleic acid amide or an erucic acid amide is particularly preferred.

Examples of the alkylene bis-fatty acid amides include a methylene bis-lauric acid amide, a methylene bis-myristate acid amide, a methylene bis-palmitate acid amide, a methylene bis-stearate acid amide, a methylene bis-oleic acid amide, a methylene bis-slinoleic acid amide, a methylene bis-linolenic acid amide, a methylene bis-arachidinic acid amide, a methylenebis-arachidonate acid amide, a methylenebis-behenate acid amide, a methylenebis-lignoselinic acid amide, a methylenebis-serotinic acid amide, a methylenebis-montanate acid amide, a methylenebis-mellicinic acid amide, a methylenebis-12-hydroxystearate amide, a methylenebis-ricinoleic acid amide, an ethylenebis-laurinic acid amide, an ethylenebis-myristic acid amide, an ethylenebis-palmitic acid amide, an ethylenebis-stearic acid amide, an ethylenebis-oleic acid amide, an ethylenebis-linoleic acid amide, an ethylenebis-linolenic acid amide, an ethylene bis-arachidinic acid amide, an ethylene bis-arachidonic acid amide, an ethylene bis-behenic acid amide, an ethylene bis-lignoserynic acid amide, an ethylene bis-serotinic acid amide, an ethylene bis-montanic acid amide, an ethylene bis-mericinic acid amide, an ethylene bis-12-hydroxystearic acid amide, an ethylene bis-ricinoleic acid amide. Among them, a methylene bis-laurinic acid amide, a methylene bis-myristic acid amide, a methylene bis-palmitic acid amide, a methylene bis-stearic acid amide, an ethylene bis-laurinic acid amide, an ethylene bis-myristic acid amide, an ethylene bis-palmitic acid amide, or an ethylene bis-stearic acid amide is preferable, a methylene bis-stearic acid amide or an ethylene bis-stearic acid amide is more preferable, and an ethylene bis-stearic acid amide is particularly preferable.

Examples of the alkylol fatty acid amide include a methylol fatty acid amide and an ethylol fatty acid amide.

Examples of the N-alkyl fatty acid amide include an *N*-stearyl stearic acid amide, an *N*-stearyl oleic acid amide, an *N*-oleoyl stearic acid amide, an *N*-stearyl erucic acid amide, and an N-oleoyl oleic acid amide.

Examples of the fatty acid include lauric acid, myristic acid, palmitic acid, stearic acid, oleic acid, linoleic acid, linolenic acid, arachidic acid, arachidonic acid, behenic acid, erucic acid, lignoseric acid, serotinic acid, montanic acid, melicic acid, 12-hydroxystearic acid, and ricinoleic acid.

Examples of the higher alcohol include one in which a hydroxyl group is bonded to a fatty acid residue exemplified as a fatty acid residue constituting the fatty acid ester.

Examples of the sugar alcohol include mannitol.

When the additive composition for synthetic resins of the present embodiment contains a lubricant, the content of the lubricant may be, for example, from 5 to 500 parts by mass per 100 parts by mass of a compound containing a monovalent group represented by general formula (1). From the viewpoint of further improving the properties of a synthetic resin, the content of the lubricant with respect to 100 parts by mass of a compound containing a monovalent group represented by general formula (1) is preferably 10 parts by mass or more, more preferably 20 parts by mass or more, further preferably 30 parts by mass or more, and still more preferably 50 parts by mass or more. From a similar viewpoint, the content of the lubricant with respect to 100 parts by mass of the compound containing a monovalent group represented by general formula (1) is preferably 300 parts by mass or less, more preferably 150 parts by mass or less, further preferably 100 parts by mass or less, and still more preferably 80 parts by mass or less.

In the present embodiment, when the additive agent for synthetic resins contained in the additive composition for synthetic resins is a nucleating agent and the additive composition for synthetic resins contains a lubricant, the additive composition for synthetic resins can provide excellent transparency to a molded article made of a synthetic resin.

The additive composition for synthetic resins of the present embodiment preferably contains a colorant.

Examples of the colorant include a pigment and a dye. From the viewpoint of durability of a colorant, a pigment is preferable among them.

Examples of the pigment include: Pigment Red 1, 2, 3, 9, 10, 17, 22, 23, 31, 38, 41, 48, 49, 88, 90, 97, 112, 119, 122, 123, 144, 149, 166, 168, 169, 170, 171, 177, 179, 180, 184, 185, 192, 200, 202, 209, 215, 216, 217, 220, 223, 224, 226, 227, 228, 240, 254; Pigment Orange 13, 31, 34, 36, 38, 43, 46, 48, 49, 51, 52, 55, 59, 60, 61, 62, 64, 65, 71; Pigment Yellow 1, 3, 12, 13, 14, 16, 17, 20, 24, 55, 60, 73, 81, 83, 86, 93, 95, 97, 98, 100, 109, 110, 113, 114, 117, 120, 125, 126, 127, 129, 137, 138, 139, 147, 148, 150, 151, 152, 153, 154, 166, 168, 175, 180, 185; Pigment Green 7, 10, 36; Pigment Blue 15, 15:1, 15:2, 15:3, 15:4, 15:5, 15:6, 22, 24, 29, 56, 60, 61, 62, 64; and Pigment Violet 1, 15, 19, 23, 27, 29, 30, 32, 37, 40, 50.

Examples of the dye include an azo dye, an anthraquinone dye, an indigoid dye, a triarylmethane dye, a xanthene dye, an alizarin dye, an acridine dye, a stilbene dye, a thiazole dye, a naphthol dye, a quinoline dye, a nitro dye, an indamine dye, an oxazine dye, a phthalocyanine dye, and a cyanine dye.

When the additive composition for synthetic resins of the present embodiment contains a colorant, the content of the colorant with respect to 100 parts by mass of a compound containing a monovalent group represented by general formula (1) may be, for example, from 0.01 to 100 parts by mass. The content of the colorant with respect to 100 parts by mass of the compound containing a monovalent group represented by general formula (1) is preferably from 0.05 to 50 parts by mass, and more preferably from 0.1 to 30 parts by mass.

In the additive composition for synthetic resins of the present embodiment, the colorant preferably includes a blue colorant.

Examples of the blue colorant include: a blue pigment such as Pigment Blue 15, 15:1, 15:2, 15:3, 15:4, 15:5, 15:6, 22, 24, 29, 56, 60, 61, 62, or 64; a purple pigment such as Pigment Violet 1, 15, 19, 23, 27, 29, 30, 32, 37, 40, or 50; a green pigment such as Pigment Green 7, 10, or 36; a blue dye such as Acid blue 1, 3, 5, 7, 9, 11, C.I.13, 15, 17, 22, 24, 26, 34, 38, 48, 74, 75, 83, 84, 86, 88, 90, 90:1, 91, 93, 93:1, 99, 100, 103, 104, 108, 109, 110, 119, 123, 147, or 213; a purple dye such as Acid Violet 15, 17, 24, 43, or 49; and a green dye such as Acid Green 3, 9, or 16. Among them, a blue colorant, a purple colorant, or a green colorant is preferable, and a blue colorant or a purple colorant is more preferable.

In the present embodiment, when the additive agent for synthetic resins is a nucleating agent and the additive composition for synthetic resins contains a colorant, the additive composition for synthetic resins is capable of imparting

an excellent appearance to a molded article made of a synthetic resin.

The additive composition for synthetic resins of the present embodiment may further contain one or more additives such as a phenolic antioxidant, a phosphorus antioxidant, a sulfur antioxidant, another antioxidant, a hindered amine compound, an ultraviolet absorber, a fatty acid metal salt, a flame retardant, a flame retardant aid, a filler, a hydrotalcite, an antistatic agent, or a fluorescent brightening agent, which consist of compounds other than compounds containing a monovalent group represented by general formula (1) (hereinafter referred to as "other additives"). The additive composition for synthetic resins may be a one-pack composite additive composition in which a granulation aid such as a binder, a wax, a solvent, and silica is further blended and granulated, or may be a composite master batch containing more of the synthetic resin. Here, for example, the content of the synthetic resin in the composite master batch with respect to the total composite master batch may be 99.9% by mass or less, and is preferably 90% by mass or less, more preferably 80% by mass or less, and still more preferably 60% by mass or less. The content of the synthetic resin in the composite master batch may be, for example, 10% by mass or more of the total composite master batch.

Examples of the phenolic antioxidants include a 3-(3,5-dialkyl-4-hydroxyphenyl)propionic acid derivative such as 2,6-di-*tert*-butyl-4-ethylphenol, 2-*tert*-butyl-4,6-dimethylphenol, styrenated phenol, 2,2'-methylenebis(4-ethyl-6-*tert*-butylphenol), 2,2'-thiobis-(6-*tert*-butyl-4-methylphenol), 2,2'-thiodiethylenebis[3-(3,5-di-*tert*-butyl-4-hydroxyphenyl)propionate], 2-methyl-4,6-bis(octylsulfanylmethyl)phenol, 2,2'-isobutylidenebis(4,6-dimethylphenol), isooctyl-3-(3,5-di-*tert*-butyl-4-hydroxyphenyl)propionate, *N,N'*-hexane-1,6-diylbis[3-(3,5-di-*tert*-butyl-4-hydroxyphenyl)propionamide], 2,2'-oxamide-bis[ethyl-3-(3,5-di-*tert*-butyl-4-hydroxyphenyl)propionate], 2-ethylhexyl-3-(3',5'-di-*tert*-butyl-4'-hydroxyphenyl)propionate, 2,2'-ethylenebis(4,6-di-*tert*-butylphenol), an ester of 3,5-di-*tert*-butyl-4-hydroxybenzenepropanoic acid and C13-15 alkyl, 2,5-di-*tert*-amylhydroquinone, hindered phenol polymer (ADEKA POLYMER ADDITIVES, manufactured by EUROPE SAS, trade name "AO.OH.98"), 2,2'-methylenebis[6-(1-methylcyclohexyl)-p-cresol], 2-*tert*-butyl-6-(3-*tert*-butyl-2-hydroxy 5-methylbenzyl)-4-methylphenyl acrylate, 2-[1-(2-hydroxy-3,5-di-*tert*-pentylphenyl)ethyl]-4,6-di-*tert*-pentylphenyl acrylate, 6-[3-(3-*tert*-butyl-4-hydroxy-5-methyl)propoxy]-2,4,8,10-tetra-*tert*-butylbenz[d,f][1,3,2]-di oxaphosphobin, hexamethylenebis[3-(3,5-di-*tert*-butyl-4-hydroxyphenyl)propionate], bis[monoethyl(3,5-di-*tert*-butyl-4-hydroxybenzyl)phosphonate] calcium salt, a reaction product of 5,7-bis(1,1-dimethylethyl)-3-hydroxy-2(3*H*)-benzofuranone and *o*-xylene, 2,6-di-*tert*-butyl-4-(4,6-bis(octylthio)-1,3,5-triazin-2-ylamino)phenol, DL-a-tocopherol (vitamin E), 2,6-bis(α-methylbenzyl)-4-methylphenol, bis[3,3-bis-(4'-hydroxy-3'-*tert*-butyl-phenyl)butanoic acid] glycol ester, 2,6-di-*tert*-butyl-*p*-cresol, 2,6-diphenyl-4-octadecyloxyphenol, stearyl *(3,5-di-tert*-butyl-4-hydroxyphenyl)propionate, distearyl (3,5-di-*tert*-butyl-4-hydroxybenzyl)phosphonate, tridecyl-3,5-*tert*-butyl-4-hydroxybenzylthioacetate, thiodiethylene bis[(3,5-di-*tert*-butyl-4-hydroxyphenyl)propionate], 4,4'-thiobis(6-*tert*-butyl-m-cresol), 2-octylthio-4,6-di(3,5-di-*tert*-butyl-4-hydroxyphenoxy)-*s*-triazine, 2,2'-methylenebis(4-methyl-6-*tert*-butylphenol), bis[3,3-bis(4-hydroxy-3-*tert*-butylphenyl)butyric acid]glycol ester, 4,4'-butylidenebis(2,6-di-*tert*butylphenol), 4,4'-butylidenebis(6-*tert*-butyl-3-methylphenol), 2,2'-ethylidenebis(4,6-di-*tert*butylphenol), 1,1,3-tris(2-methyl-4-hydroxy-5-*tert*-butylphenyl)butane, bis[2-*tert*-butyl-4-methyl-6-(2-hydroxy-3-*tert*-butyl-5-methylbenzyl)phenyl]terephthalate, 1,3,5-tris(2,6-dimethyl-3-hydroxy-4-*tert*-butylbenzyl)isocyanurate, 1,3,5-tris(3,5-di-*tert*-butyl-4-hydroxybenzyl)isocyanurate, 1,3,5-tris(3,5-di-*tert*-butyl-4-hydroxybenzyl)-2,4,6-trimethylbenzene, 1,3,5-tris[(3,5-di-*tert*-butyl-4-hydroxyphenyl)propionyloxyethyl]isocyanurate, tetrakis[methylene-3-(3',5'-*tert*-butyl-4'-hydroxyphenyl)propionate]methane, 2-*tert*-butyl-4-methyl-6-(2-acryloyloxy-3-*tert*-butyl-5-methylbenzyl)phenol, 3,9-bis[2-(3-*tert*-butyl-4-hydroxy-5-methylhydrocinnamoyloxy)-1,1-dimethylethyl]-2,4,8,10-*t*etraoxaspiro[5.5] undecane, triethylene glycol bis[β-(3-*tert*-butyl-4-hydroxy-5-methylphenyl)propionate], stearyl-3-(3,5-di-*tert*-butyl-4-hydroxyphenyl)propionamide, palmityl-3-(3,5-di-*tert*-butyl-4-hydroxyphenyl)propionamide, myristyl-3-(3,5-di-*tert*-butyl-4-hydroxyphenyl)propionamide, or lauryl-3-(3,5-di-*tert*-butyl-4-hydroxyphenyl)propionic acid amide.

Examples of the phosphorus antioxidants include triphenyl phosphite, diisooctyl phosphite, heptakis (dipropylene glycol) triphosphite, triisodecyl phosphite, diphenyl isooctyl phosphite, diisooctylphenyl phosphite, diphenyltridecyl phosphite, triisooctyl phosphite, trilauryl phosphite, diphenyl phosphite, tris(dipropylene glycol) phosphite, dioleyl hydrogen phosphite, trilauryl trithiophosphite, bis(tridecyl)phosphite, tris(isodecyl)phosphite, tris(tridecyl)phosphite, diphenyldecyl phosphite, dinonylphenyl bis(nonylphenyl)phosphite, poly(dipropylene glycol) phenyl phosphite, tetraphenyl dipropylene glycol diphosphite, trisnonylphenyl phosphite, tris(2,4-di-*tert*-butylphenyl)phosphite, tris(2,4-di-*tert*-butyl-5-methylphenyl)phosphite, tris[2-*tert*-butyl-4-(3-*tert*-butyl-4-hydroxy-5-methylphenylthio)-5-methylphenyl]phosphite, tri(decyl)phosphite, octyldiphenyl phosphite, di(decyl) monophenyl phosphite, a mixture of distearylpentaerythritol and calcium stearate, alkyl (C10) bisphenol A phosphite, tetraphenyl-tetra(tridecyl)pentaerythritol tetraphosphite, bis(2,4-di-*tert*-butyl-6-methylphenyl)ethyl phosphite, tetra(tridecyl)isopropylidenediphenol diphosphite, tetra(tridecyl)-4,4'-*n*-butylidenebis(2-*tert*-butyl-5-methylphenol) diphosphite, hexa(tridecyl)-1,1,3-tris(2-methyl-4-hydroxy-5-*tert*-butylphenyl)butane triphosphite, tetrakis(2,4-di-*tert*-butylphenyl)biphenylenediphosphonite, 9,10-dihydro-9-oxa-10-phosphaphenanthrene-10-oxide, (1-methyl-1-propenyl-3-ylidene)tris(1,1-dimethylethyl)-5-methyl-4,1-phenylene)hexatridecylphosphite, 2,2'-methylenebis(4,6-di-*tert*-butylphenyl)-2-ethylhexylphosphite, 2,2'-methylenebis(4,6-di-*tert*-butylphenyl)-octadecylphosphite, 2,2'-ethylidenebis(4,6-di-*tert*-butylphenyl)fluorophosphite, 4,4'-butylidenebis(3-methyl-6-*tert*-butylphenylditridecyl)phosphite, tris(2-[(2,4,8,10-tetrakis-*tert*-butyldibenzo[d,f][1,3,2]dioxaphosphepin-6-yl)oxy]ethyl)amine, 3,9-bis(4-nonylphenoxy)-2,4,8,10-tetraoxa-3,9-diphosphespiro[5,5]undecane, 2,4,6-tri-*tert*-butylphenyl-2-butyl-2-ethyl-1,3-propanediol phosphite, poly 4,4'-isopropylidenediphenol C12-15 alcohol phosphite, bis(diisodecyl)pentaerythritol diphosphite, bis(tridecyl)pentaerythritol diphosphite, bis(octadecyl)pentaerythritol diphosphite, bis(nonylphenyl)pentaerythritol diphosphite, bis(2,4-di-*tert*-butylphenyl)pentaerythritol diphosphite, bis(2,4,6-tri-*tert*-butylphenyl)pentaerythritol diphosphite, bis(2,6-di-*tert*-butyl-4-methylphenyl)pentaerythritol diphosphite, and bis(2,4-dicumylphenyl)pentaerythritol diphosphite.

Examples of the sulfur antioxidant include tetrakis[methylene-3-(laurylthio)propionate]methane, bis(methyl-4-[3-*n*-alkyl(C12/C14)thiopropionyloxy]5-*tert*-butylphenyl)sulfide, ditridecyl-3,3'-thiodipropionate, dilauryl-3,3'-thiodipropionate, dimyristyl-3,3'-thiodipropionate, distearyl-3,3'-thiodipropionate, lauryl/stearyl thiodipropionate, 4,4'-thiobis(6-*tert*-butyl-*m*-cresol), 2,2'-thiobis(6-*tert*-butyl-*p*-cresol), and distearyl-disulfide.

Examples of the other antioxidant include a benzofuran compound such as *N*-benzyl-$\alpha$-phenyl nitrone, *N*-ethyl-$\alpha$-methyl nitrone, *N*-octyl-$\alpha$-heptyl nitrone, *N*-lauryl-$\alpha$-undecyl nitrone, *N*-tetradecyl-$\alpha$-tridecyl nitrone, *N*-hexadecyl-$\alpha$-pentadecyl nitrone, *N*-octyl-$\alpha$-heptadecyl nitrone, *N*-hexadecyl-$\alpha$-heptadecyl nitrone, *N*-octadecyl-$\alpha$-pentadecyl nitrone, *N*-heptadecyl-$\alpha$-heptadecyl nitrone, nitrone compounds such as *N*-octadecyl-$\alpha$-heptadecyl nitrone, 3-arylbenzofuran-2(3*H*)-one, 3-(alkoxyphenyl)benzofuran-2-one, 3-(acyloxyphenyl)benzofuran-2(3*H*)-one, 5,7-di-*tert*-butyl-3-(3,4-dimethylphenyl)-benzofuran-2(3*H*)-one, 5,7-di-*tert*-butyl-3-(4-hydroxyphenyl)-benzofuran-2(3H)-one, 5,7-di-*tert*-butyl-3-{4-(2-hydroxyethoxy)phenyl }-benzofuran-2(3H)-one, 6-(2-(4-(5,7-di-*tert*-2-oxo-2,3-dihydrobenzofuran-3-yl)phenoxy)ethoxy)-6-oxohexyl-6-((6-hydroxyhexa noyl)oxy)hexanoate, or 5-di-*tert*-butyl-3-(4-((15-hydroxy-3,6,9,13-tetraoxapentadecyl)oxy)phenyl)benzofuran-2(3*H*)one.

Examples of hindered amine compounds include 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl) sebacate, tetrakis(2,2,6,6-tetramethyl-4-piperidyl)-1,2,3,4-butanetetracarboxylate, tetrakis(1,2,2,6,6-pentamethyl-4-piperidyl)-1,2,3,4-butanetetracarboxylate, bis(2,2,6,6-tetramethyl-4-piperidyl) di(tridecyl)-1,2,3,4-butane tetracarboxylate, bis(1,2,2,6,6-pentamethyl-4-piperidyl)-di(tridecyl)-1,2,3,4-butanetetracarboxylate, bis(1,2,2,4,4-pentamethyl-4-piperidyl)-2-butyl-2-(3,5-di-*tert*-butyl-4-hydroxybenzyl)malonate, 1-(2-hydroxyethyl)-2,2,6,6-tetramethyl-4-piperidinol/diethyl succinate polycondensate, 1,6-bis(2,2,6,6-tetramethyl-4-piperidylamino)hexane/2,4-dichloro-6-morpholino-s-triazine polycondensate, 1,6-bis(2,2,6,6-tetramethyl-4-piperidylamino)hexane/2,4-dichloro-6-*tert*-octylamino-s-triazine polycondensate, 1,5,8,12-tetrakis[2,4-bis(*N*-butyl-*N*-(2,2,6,6-tetramethyl-4-piperidyl)amino)-*s*-triazin-6-yl]-1,5,8,12-tetraazadodecane, 1,5,8,12-tetrakis[2,4-bis(*N*-butyl-*N*-(1,2,2,6,6-pentamethyl-4-piperidyl)amino)-s-triazin-6-yl]-1,5,8-12-tetraazadodecane, 1,6,11-tris[2,4-bis(*N*-butyl-*N*-(2,2,6,6-tetramethyl-4-piperidyl)amino-*s*-triazin-6-ylamino]undecane, 1,6,11-tris[2,4-bis(*N*-butyl-*N*-(1,2,2,6,6-pentamethyl-4-piperidyl)amino-*s*-triazin-6-ylamino]undecane, 3,9-bis[1,1-dimethyl-2-{tris(2,2,6,6-tetramethyl-4-piperidyloxycarbonyl)butylcarbonyloxy}ethyl]-2,4,8,10-tetra oxaspiro[5.5]undecane, 3,9-bis[1,1-dimethyl-2-{tris(1,2,2,6,6-pentamethyl-4-piperidyloxycarbonyl)butylcarbonyloxy}ethyl]-2,4,8,10-tetraoxaspiro[5.5]undecane, bis(1-undecyloxy-2,2,6,6-tetramethylpiperidin-4-yl)carbonate, 2,2,6,6-tetramethyl-4-piperidyl hexadecanoate, 2,2,6,6-tetramethyl-4-piperidyl octadecanoate.

Examples of the ultraviolet absorber include: a 2-hydroxybenzophenone such as 2,4-dihydroxybenzophenone or 5,5'-methylenebis(2-hydroxy-4-methoxybenzophenone); a 2-(2-hydroxyphenyl)benzotriazole such as 2-(2-hydroxy-5-methylphenyl)benzotriazole, 2-(2-hydroxy-5-*tert*-octylphenyl)benzotriazole, 2-(2-hydroxy-3,5-di-*tert*-butylphenyl)-5-chlorobenzotriazole, 2-(2-hydroxy-3-*tert*-butyl-5-methylphenyl)-5-chlorobenzotriazole, 2-(2-hydroxy-3,5-dicumylphenyl)benzotriazole, 2,2'-methylenebis(4-*tert*-octyl-6-benzotriazolylphenol), polyethylene glycol ester of 2-(2-hydroxy-3-*tert*-butyl-5-carboxyphenyl)benzotriazole, 2-[2-hydroxy-3-(2-acryloyloxyethyl)-5-methylphenyl]benzotriazole, 2-[2-hydroxy-3-(2-methacryloyloxyethyl)-5-*tert*-butylphenyl]benzotriazole, 2-[2-hydroxy-3-(2-methacryloyloxyethyl)-5-*tert*-octylphenyl]benzotriazole, 2-[2-hydroxy-3-(2-methacryloyloxyethyl)-5-*tert*-butylphenyl]-5-chlorobenzotriazole, 2-[2-hydroxy-5-(2-methacryloyloxyethyl)phenyl]benzotriazole, 2-[2-hydroxy-3-*tert*-butyl-5-(2-methacryloyloxyethyl)phenyl]benzotriazole, 2-[2-hydroxy-3-*tert*-amyl-5-(2-methacryloyloxyethyl)phenyl]benzotriazole, 2-[2-hydroxy-3-*tert*-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, or 2-[2-hydroxy-4-(3-methacryloyloxypropyl)phenyl]benzotriazole; a benzoate such as phenyl salicylate, resorcinol monobenzoate, 2,4-di-*tert*-butylphenyl-3,5-di-*tert*-butyl-4-hydroxybenzoate, octyl (3,5-di-*tert*-butyl-4-hydroxy)benzoate, dodecyl (3,5-di-*tert*-butyl-4-hydroxy)benzoate, tetradecyl (3,5-di-*tert*-butyl-4-hydroxy)benzoate, hexadecyl (3,5-di-*tert*-butyl-4-hydroxy)benzoate, octadecyl (3,5-di-*tert*-butyl-4-hydroxy)benzoate, or behenyl(3,5-di-*tert*-butyl-4-hydroxy)benzoate; a substituted oxanilide such as 2-ethyl-2'-ethoxyoxanylide or 2-ethoxy-4'-dodecyl oxanilide; a cyanoacrylate such as ethyl-$\alpha$-cyano-$\beta,\beta$-diphenyl acrylate or methyl-2-cyano-3-methyl-3-(*p*-methoxyphenyl)acrylate; a triazine such as 2-(4,6-diphenyl-1,3,5-triazin-2-yl)-5-hexyloxyphenol, 2-(2-hydroxy-4-octoxyphenyl)-4,6-bis(2,4-dimethylphenyl)-1,3,5-triazine, trioctyl-2,2',2"-((1,3,5-triazine-2,4,6-triyl)tris(3-hydroxybenzene-4-,1-diyl)tripropionate), 2-(4,6-diphenyl-1,3,5-triazin-2-yl)-5-[2-(2-ethylhexanoyloxy)ethoxy]phenol, 2,4,6-tris(2-hydroxy-4-hexyloxy-3-methylphenyl)-1,3,5-triazine, or 1,12-bis[2-[4-(4,6-diphenyl-1,3,5-triazin-2-yl)-3-hydroxyphenoxy]ethyl]dodecanedioate; a variety of metal salts, or metal chelates, particularly a nickel or a chromium salt, or a chelate.

Examples of the fatty acid metal salt include a metal salt of a fatty acid having from 12 to 30 carbon atoms

containing a linear or branched fatty acid residue. Examples of a metal ion constituting the fatty acid metal salt include a sodium ion, a potassium ion, a lithium ion, a dihydroxyaluminum ion, a calcium ion, a zinc ion, a barium ion, a magnesium ion, and a hydroxyaluminum ion., among them, a sodium ion, a potassium ion, a lithium ion, or a calcium ion is preferable. Examples of a fatty acid constituting the fatty acid metal salt include lauric acid, myristic acid, palmitic acid, stearic acid, oleic acid, linoleic acid, linolenic acid, arachidic acid, arachidonic acid, behenic acid, lignoceric acid, serotinic acid, montanic acid, and melicic acid, and among them, a myristic acid or a stearic acid is preferable. In the fatty acid constituting the fatty acid metal salt, one or more of hydrogen atoms of a fatty acid residue may be substituted with a hydroxyl group. Examples of such a fatty acid include 12-hydroxystearic acid and 12-hydroxyoleic acid.

Examples of the flame retardant include: a phosphonate esters such as triphenyl phosphate, tricresyl phosphate, trixylenyl phosphate, cresyl diphenyl phosphate, cresyl-2,6-dixylenyl phosphate, resorcinol bis(diphenyl phosphate), (1-methylethylidene)-4,1-phenylenetetraphenyl diphosphate, 1,3-phenylenetetrakis(2,6-dimethylphenyl)phosphate, an aromatic phosphate of trade name "ADEKA Stub FP-500", "ADK STAB FP-600", or "ADK STAB FP-800" manufactured by ADEKA Co., Ltd., divinyl phenylphosphonate, diallyl phenylphosphonate, or phenylphosphonic acid (1-butenyl); a phosphinate ester such as phenyl diphenylphosphinate, methyl diphenylphosphinate, a 9,10-dihydro-9-oxa-10-phosphaphenanthrene-10-oxide derivative; a phosphazene compound such as bis(2-allylphenoxy)phosphazene or dicresylphosphazene; a phosphorus-containing flame retardant such as melamine phosphate, melamine pyrophosphate, melamine polyphosphate, melam polyphosphate, ammonium polyphosphate, piperazine phosphate, piperazine pyrophosphate, piperazine polyphosphate, a phosphorus-containing vinylbenzyl compound, or red phosphorus; a metal hydroxide such as magnesium hydroxide or aluminum hydroxide; and a brominated flame retardant such as brominated bisphenol A type epoxy resin, brominated phenolic novolak type epoxy resin, hexabromobenzene, pentabromotoluene, ethylenebis(pentabromophenyl), ethylenebistetrabromophthalimide, 1,2-dibromo-4-(1,2-dibromoethyl)cyclohexane, tetrabromocyclooctane, hexabromocyclododecane, bis(tribromophenoxy)ethane, brominated polyphenylene ether, brominated polystyrene and 2,4,6-tris(tribromophenoxy)-1,3,5-triazine, tribromophenylmaleimide, tribromophenyl acrylate, tribromophenyl methacrylate, tetrabromobisphenol A-type dimethacrylate, pentabromobenzyl acrylate, or brominated styrene. These flame retardants are preferably used in combination with an anti-drip agent such as a fluoropolymer, or a flame retardant aid such as a polyhydric alcohol or hydrotalcite. Examples of the filler include talc, mica, calcium carbonate, calcium oxide, calcium hydroxide, magnesium carbonate, magnesium hydroxide, magnesium oxide, magnesium sulfate, aluminum hydroxide, barium sulfate, glass powder, glass fiber, clay, dolomite, silica, alumina, potassium titanate whisker, wollastenite, and fibrous magnesium oxysulfate, and the particle size (the fiber size or fiber length and aspect ratio in the fibrous form) can be selected as appropriate. Among these fillers, talc is particularly preferable because talc is effective in imparting rigidity and is readily available. A filler can be surface-treated if necessary.

The hydrotalcite may be a complex salt compound containing magnesium, aluminum, a hydroxyl group, a carbonate group, and any crystalline water, which may be a natural product or a synthetic product. The crystal structure, the particle shape, and the particle size of a hydrotalcite are not particularly limited. Furthermore, the hydrotalcite may be one in which at least a part of magnesium or aluminum is substituted with another metal such as an alkali metal or zinc, or one in which at least a part of a hydroxyl group or a carbonate group is substituted with another anionic group. Still further, the hydrotalcite may be one in which the crystalline water is dehydrated, or one in which the surface is coated with a higher fatty acid such as stearic acid, a higher fatty acid metal salt such as alkali metal salt of oleic acid, an organic sulfonic acid metal salt such as alkali metal salt of dodecylbenzenesulfonic acid, a higher fatty acid amide, a higher fatty acid ester or wax or the like.

Examples of the antistatic agent include a low molecular weight type antistatic agent using a nonionic, anionic, cationic, amphoteric surfactant, or the like, and a high molecular weight type antistatic agent using a high molecular weight compound. Examples of the nonionic surfactant include a polyethylene glycol type nonionic surfactant such as a higher alcohol ethylene oxide adduct, a fatty acid ethylene oxide adduct, a higher alkylamine ethylene oxide adduct, or a polyolefin glycol ethylene oxide adduct; and a polyhydric alcohol type nonionic surfactant such as polyethylene oxide, a fatty acid ester of glycerin, a fatty acid ester of pentaerythrit, a fatty acid ester of sorbitol or sorbitan, an alkyl ether of a polyhydric alcohol, or an aliphatic amide of an alkanolamine. Examples of the anionic surfactant include a carboxylic acid salt such as an alkali metal salt of a higher fatty acid; a sulfate such as a higher alcohol sulfate or a higher alkyl ether sulfate, a sulfonate such as an alkylbenzene sulfonate, an alkyl sulfonate, or a paraffin sulfonate; and a phosphate ester salt such as a higher alcohol phosphate ester salt. Examples of the cationic surfactant include a quaternary ammonium salt such as an alkyl trimethyl ammonium salt. Examples of the amphoteric surfactant include an amino acid-type amphoteric surfactant such a higher alkyl aminopropionate salt, and a betaine-type amphoteric surfactant such as a higher alkyl dimethyl betaine or a higher alkyl dihydroxyethyl betaine. Among them, an anionic surfactant is preferable, and in particular, a sulfonate such as alkylbenzene sulfonate, alkyl sulfonate, and paraffin sulfonate is preferable.

Examples of the high molecular weight type antistatic agent include a block polymer with an ionomer or polyethylene glycol as the hydrophilic moiety. Examples of the ionomer include the ionomer described in JP 2010-132927A. Examples of the polymer with polyethylene glycol as the hydrophilic moiety include the polyether ester amide described

in JP H07-10989A, the polymer composed of the polyolefin and the polyethylene glycol described in US6552131B1, the polymer composed of the polyester and the polyethylene glycol described in JP 2016-023254A.

The fluorescent brightening agent is a compound that absorbs ultraviolet rays of sunlight or artificial light, converts the ultraviolet rays to visible rays of violet to blue light, and radiates the visible rays, thereby enhancing the whiteness and bluish color of a molded article. Examples of the fluorescent brightening agent include a benzoxazole compound C.I. Fluorescent Brightener 184; a coumarin compound C.I. Fluorescent Brightener 52; a diaminostilbenedisulfonate compound C.I. Fluorescent Brightener 24, 85, 71.

The additive composition for synthetic resins of the present embodiment may be, for example, in particle form. When the additive composition for synthetic resins of the present embodiment is in particle form, the powder property values of the additive composition for synthetic resins, such as average particle size, angle of repose, loose bulk density, packed bulk density, and degree of compressibility, may be such that the dispersibility of the additive composition for synthetic resins in a synthetic resin, the fluidity of particles, and the like are at desired degrees. Specifically, the average particle size may be, for example, from 0.1 to 100 $\mu$m, the angle of repose may be, for example, from 20 to 70°, the loose bulk density may be, for example, from 0.1 to 0.8 g/cm$^3$, the packed bulk density may be, for example, from 0.2 to 1 g/cm$^3$, and the compression ratio may be, for example, from 1 to 10. Here, the average particle size is the average particle size calculated from the particle size distribution measured by the laser diffraction method in accordance with JIS Z 8825, the angle of repose is the angle of repose measured by the cylindrical rotation method, the loose bulk density is the bulk density measured in accordance with JIS K 5101-12-1, the packed bulk density is the bulk density measured in accordance with JIS K 5101-12-2, and the compression ratio is the value calculated by the following equation:

$$\text{(compression ratio)} = \text{(packed bulk density)}/\text{(loose bulk density)}.$$

Next, the resin composition of the present embodiment will be described.

< Resin Composition >

The resin composition of the present embodiment contains a synthetic resin and the additive for synthetic resins.

The resin composition of the present embodiment will have excellent properties.

Examples of the synthetic resin include those contained in the master batch described above. In the resin composition of the present embodiment, the synthetic resin is preferably a thermoplastic resin, and is further preferably a crystalline resin. In the resin composition of the present embodiment, the synthetic resin preferably contains a polyolefin resin. Examples of the polyolefin resin include: a polyethylene resin such as a low-density polyethylene, a linear low-density polyethylene, a high-density polyethylene, a cross-linked polyethylene, or an ultrahigh molecular weight polyethylene; a polypropylene resin such as homopolypropylene, random copolymer polypropylene, block copolymer polypropylene, impact copolymer polypropylene, high impact copolymer polypropylene, or maleic anhydride-modified polypropylene; an $\alpha$-olefin polymer such as polybutene-1, a cyclo-olefin polymer, poly-3-methyl-1-butene, poly-3-methyl-1-pentene, or poly-4-methyl-1-pentene; and an $\alpha$-olefin copolymer such as ethylene-methyl methacrylate copolymer, or ethylene-vinyl acetate copolymer. From the viewpoint of making a resin composition have more excellent heat resistance, in the resin composition of the present embodiment, the polyolefin resin preferably contains at least one selected from the group consisting of a polyethylene resin and a polypropylene resin, and particularly preferably contains a polypropylene resin. From the viewpoint of providing more excellent transparency of a molded article made of a resin composition, a random copolymer polypropylene is particularly preferable as a polypropylene resin. The molecular weight, the degree of polymerization, the density, the softening point, the percentage of insoluble content in a solvent, the degree of stereoregularity, the presence or absence of a catalyst residue, the type and blending ratio of monomers used as raw materials, the type of catalyst used for polymerization (for example, a Ziegler catalyst, or a metallocene catalyst), and the like of a polyolefin resin are not particularly limited, and may be selected as appropriate.

In the resin composition of the present embodiment, the synthetic resin may also include an elastomer. In this case, a molded article made of such a resin composition will have excellent impact resistance. Here, examples of the elastomer include: a synthetic rubber such as isoprene rubber, butadiene rubber, acrylonitrile-butadiene copolymerized rubber, styrenebutadiene copolymerized rubber, fluororubber, or silicone rubber; and a thermoplastic elastomer such as polyolefin thermoplastic elastomer, polystyrene thermoplastic elastomer, polyvinyl chloride thermoplastic elastomer, polyurethane thermoplastic elastomer, polyester thermoplastic elastomer, or polyamide thermoplastic elastomer. Among them, a thermoplastic elastomer is preferable from the viewpoint of making a resin composition more processable and reducing the weight of a molded article made of such a resin composition. Among thermoplastic elastomers, a polyolefin thermoplastic elastomer is particularly preferable. In the resin composition of the present embodiment, when a synthetic resin contains an elastomer, the content of the elastomer with respect to the total synthetic resin may be, for example, 50% by mass or less, and is preferably 30% by mass or less, and further preferably 25% by mass or less. The content

of an elastomer may be, for example, 5% by mass or more of the total synthetic resin.

In the resin composition of the present embodiment, the content of an additive for synthetic resins may be, for example, from 0.001 to 10 parts by mass with respect to 100 parts by mass of a synthetic resin. From the viewpoint of improving the properties of a resin composition, the content of the additive for synthetic resins with respect to 100 parts by mass of synthetic resin is preferably 0.005 parts by mass or more, more preferably 0.01 parts by mass or more, and further preferably 0.02 parts by mass or more, and still further preferably 0.05 parts by mass or more. From the viewpoint of sufficiently suppressing blooming and migration of an additive component, the content of an additive for synthetic resins with respect to 100 parts by mass of a synthetic resin is preferably 5 parts by mass or less, more preferably 3 parts by mass or less, further preferably 1 part by mass or less, and still further preferably 0.5 parts by mass or less. Furthermore, from the viewpoint of achieving more excellent transparency of a molded article made of a resin composition, the content of the additive for synthetic resins is also preferably 0.2 parts by mass or less per 100 parts by mass of a synthetic resin.

The resin composition of the present embodiment may further contain, if necessary, other additives exemplified as additives contained in the additive composition for synthetic resins described above.

The resin composition of the present embodiment may also contain a synthetic resin and the additive composition for synthetic resins.

The method of producing the resin composition of the present embodiment is not particularly limited, and examples thereof include a method including a preparation process for preparing the additive for synthetic resins and another additive as necessary, and a blending process for blending each component prepared in the preparation process into a synthetic resin. Here, the preparation process may be a process for preparing the additive composition for synthetic resins. The method of compounding each component in the blending process is not particularly limited, and examples thereof include a method in which each component prepared in the preparation process is added to a synthetic resin and then mixed using a mixing device such as an FM mixer, a mill roll, a Banbury mixer, a super mixer, or the like. Furthermore, the method of producing the resin composition of the present embodiment may further include, in addition to the preparation process and the blending process, a melt kneading process in which a mixture obtained in the blending process is melt kneaded using a melt kneading device such as a single-screw extruder or a twin-screw extruder. Here, the temperature of melt kneading in the melt kneading process may be, for example, from 180 to 280°C. The method of producing the resin composition of the present embodiment may further include a granulation process for granulating a kneaded material obtained in the melt kneading process. Here, the method of granulation is not particularly limited and includes, for example, the use of a pelletizer or other granulation devices. The shape of a resin composition obtained by granulation is not particularly limited, and for example, the shape may be in the form of a pellet. Furthermore, the method of producing the resin composition of the present embodiment may be a method in which at least one of the additives for synthetic resins and another additive, if necessary, is added before or during polymerization of a synthetic resin monomer or oligomer, and the remaining components are added to the obtained polymer.

Next, the molded article of the present embodiment will be described.

< Molded Article >

The molded article of the present embodiment is obtained by molding the resin composition.

The molded article of the present embodiment will have excellent properties.

In the molded article of the present embodiment, the a* value and the b* value of the transmitted color preferably satisfy $-7 \leq a^* \leq 1$ and $-1 \leq b^* \leq 5$. In this case, a molded article having a more excellent appearance can be obtained compared to cases in which the a* value or the b* value is outside the above-described range. From the viewpoint of further improving the appearance of a molded article, the a* value is more preferably -6 or higher, further preferably -5 or higher, and still further preferably -3 or higher. The a* value is more preferably 0.5 or lower, further preferably 0.1 or lower, and still further preferably 0 or lower. From a similar viewpoint, the b* value is more preferably 0 or higher, further preferably 1 or higher, still further preferably 1.5 or higher, and particularly preferably 2.5 or higher. The b* value is more preferably 4.5 or less, and further preferably 4 or less. In the present embodiment, the a* value and the b* value of a transmitted color of a molded article are measured using a colorimeter.

Examples of the molded article include an injection molded article, a fiber, a flat yarn, a biaxially oriented film, a uniaxially oriented film, a non-oriented film, a sheet, a thermoformed molded article, an extrusion blow molded article, an injection blow molded article, an injection stretch blow molded article, a profile extrusion molded article, and a rotational molded article. Preferable specific examples of the molded article include a bottle, ajar, a cup, a bucket, a box, a can, and a tank.

The method of producing a molded article is not particularly limited and examples thereof include injection molding, extrusion molding, blow molding, rotational molding, vacuum molding, inflation molding, calendaring, slush molding, dip molding, and thermoforming molding.

Examples

The present invention will now be described more specifically by way of Examples, but the present invention is not limited in any way by the following Examples.

< Synthesis of Compound Containing Monovalent Group Represented by General Formula (1) >

(Synthesis Example 1)

In a 500-mL three-necked flask equipped with a 200-mL dropping funnel, 1.8 g of cyanuric acid chloride was prepared and dissolved with 100 mL of acetone. The obtained solution was cooled in an ice bath, and while stirring, a solution of 3.35 g of 4-phenylphenol and 0.8 g of sodium hydroxide dissolved in 100 mL distilled water was added dropwise. The dropping rate was adjusted in such a manner that the temperature of a reaction solution during the dropping was 10°C or lower. After the dropping was completed, the ice bath was removed and the reaction solution was stirred for another 1 hour at room temperature. After the stirring was completed, 100 mL of distilled water was added to the reaction solution to precipitate a precipitate. The precipitated precipitate was filtered off, washed with distilled water, and dried under reduced pressure to obtain a white solid. In another 500-mL three-necked flask, 0.91 g of 4,4'-biphenol and 1.04 g of sodium carbonate were prepared, and 60 mL distilled water and 90 mL acetone were added to dissolve. After all of the white solid was added to the obtained solution, the mixture was stirred at room temperature for 3 hours. After the stirring was completed, 120 mL of distilled water was added to the reaction solution to precipitate a precipitate. The precipitate was filtered off, washed with distilled water, and dried under reduced pressure to obtain a solid. The solid obtained was purified by silica gel column chromatography (expansion solvent: toluene/hexane 9:1) to obtain 4.0 g of white solid.

The resulting white solid was subjected to $^1$H-NMR ($CDCl_3$, 400 MHz) and MALDI-TOF-MS analysis. The results of the analyses are shown below.

($^1$H-NMR)
δ(v.s.TMS): 7.57-7.47(m,12H), 7.44-7.34(m,10H), 7.25-7.17(m,10H), 7.16-7.08(m,12H)
(MALDI-TOF-MS)
m/z = 1016 (Dithranol was used as a matrix and sodium trifluoroacetate as an ionization aid)

From the analysis, the obtained white solid was confirmed to be 4",4'''-bis[4',6'-bis(4-biphenyloxy)-1',3',5'-triazine-2'-oxy]biphenyl having the following structure:

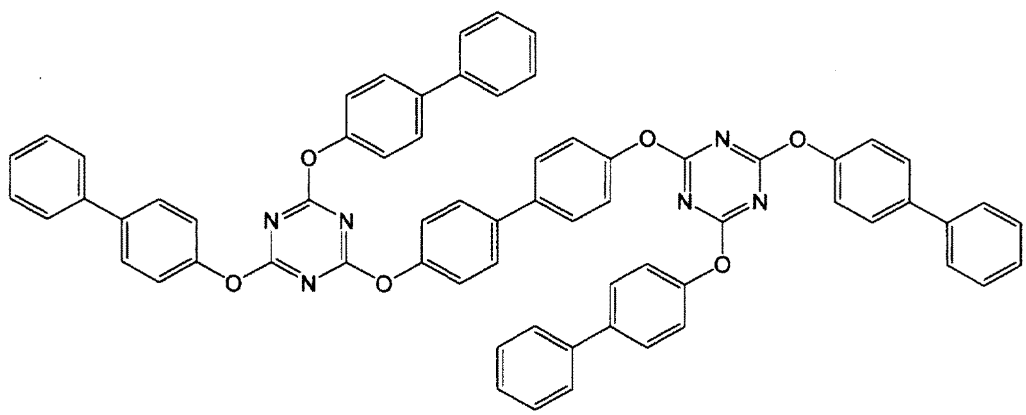

The white solid was then designated as Compound 1.

(Synthesis Example 2)

4.1 g of white solid was obtained in the same manner as in Synthesis Example 1, except that 3.47 g of 4-cyclohexylphenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.50-7.47(m,4H), 7.26-7.14(m,12H), 7.05-7.01(m,8H), 2.46-2.44(m,4H), 1.82-1.72(m,20H), 1.49-1.31(m,16H), 1.26-1.20(m,4H)
(MALDI-TOF-MS)
m/z = 1041 (Dithranol was used as a matrix and sodium trifluoroacetate as an ionization aid)

From the analysis, the obtained white solid was confirmed to be 4",4‴-bis[4',6'-bis(4-cyclohexylphenoxy)-1',3',5'-triazine-2'-oxy]biphenyl having the following structure:

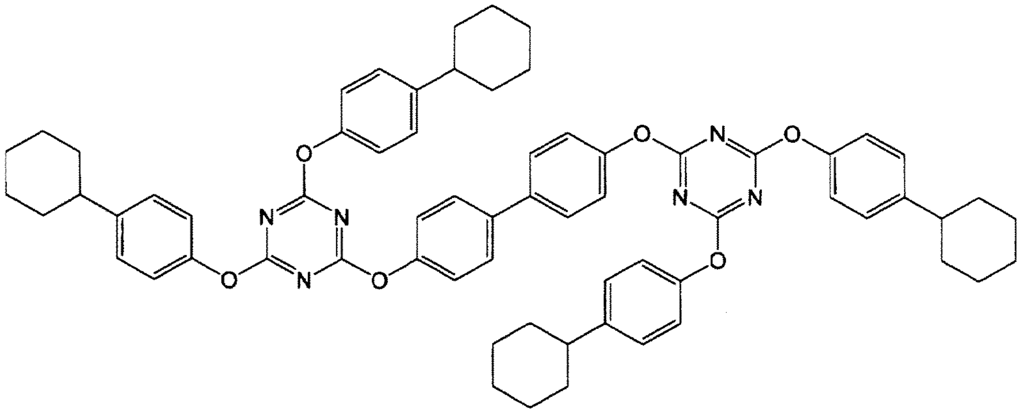

The white solid was then designated as Compound 2.

(Synthesis Example 3)

0.9 g of white solid was obtained in the same manner as in Synthesis Example 1, except that 1.74 g of 4-cyclohexylphenol and 1.68 g of 4-phenylphenol were used in combination in place of 4-phenylphenol.
The resulting white solid was subjected to $^1$H-NMR ($CDCl_3$, 400 MHz) analysis. The results of the analyses are shown below.

($^1$H-NMR)
δ(v.s.TMS): 7.55-7.51(m,8H), 7.47-7.39(m,8H), 7.38-7.33(m,2H), 7.21-7.15(m,12H), 7.06-7.04(m,4H), 2.45-2.42(m,2H), 1.82-1.71(m,10H), 1.36-1.31(m,10H)
(MALDI-TOF-MS)
m/z = 1028 (Dithranol was used as a matrix and sodium trifluoroacetate as an ionization aid)

From the analysis, the obtained white solid was confirmed to be 4",4‴-bis[4'-(4-biphenyloxy)-6'-(4-cyclohexylphenoxy)-1',3',5'-triazine-2'-oxy]biphenyl having the following structure:

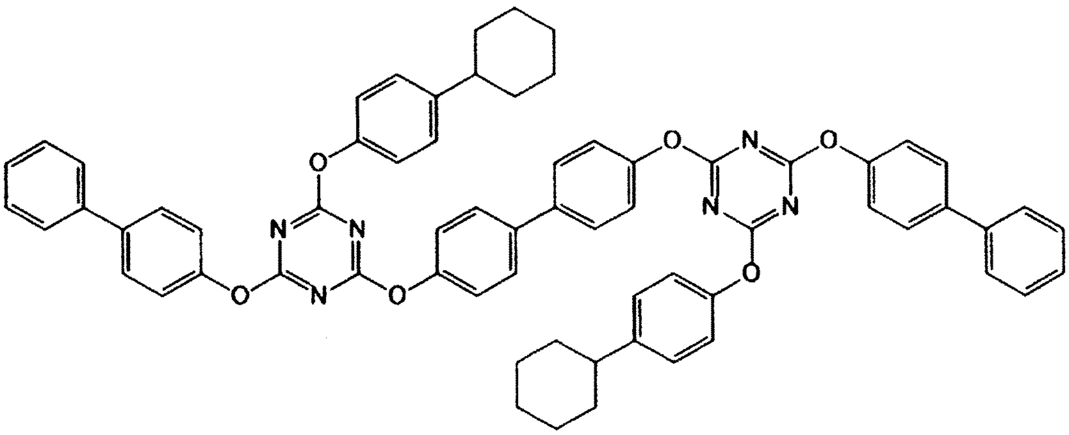

The white solid was then designated as Compound 3.

(Synthesis Example 4)

3.2 g of white solid was obtained in the same manner as in Synthesis Example 1, except that 2.40 g of 4-ethyl phenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.51-7.49(m,4H), 7.22-7.20(m,4H), 7.18-7.16(m,8H), 7.07-7.06(m,8H), 2.61(dd,J=15.2Hz,7.6Hz,8H), 1.22(t,J=7.6Hz,12H)
(MALDI-TOF-MS)
m/z = 825 (Dithranol was used as a matrix and sodium trifluoroacetate as an ionization aid)

From the analysis, the obtained white solid was confirmed to be 4",4‴-bis[4',6'-bis(4-ethylphenoxy)-1',3',5'-triazine-2'-oxy]biphenyl having the following structure:

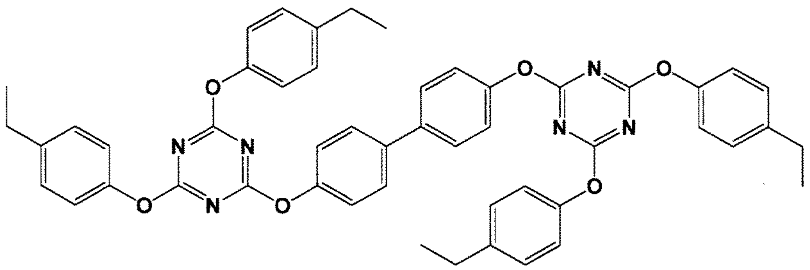

The white solid was then designated as Compound 4.

(Synthesis Example 5)

3.5 g of white solid was obtained in the same manner as in Synthesis Example 1, except that 2.68g of 4-*n*-propyl phenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.51-7.48(m,4H), 7.22-7.20(m,4H), 7.15-7.13(m,8H), 7.06-7.05(m,8H), 2.55(t,J=7.6Hz,8H), 1.61(dt,J=7.6Hz,7.2Hz,8H), 0.92(t,J=7.2Hz,12H)
(MALDI-TOF-MS)
m/z = 881 (Dithranol was used as a matrix and sodium trifluoroacetate as an ionization aid)

From the analysis, the obtained white solid was confirmed to be 4",4‴-bis[4',6'-bis(4-*n*-propylphenoxy)-1',3',5'-triazine-2'-oxy]biphenyl having the following structure:

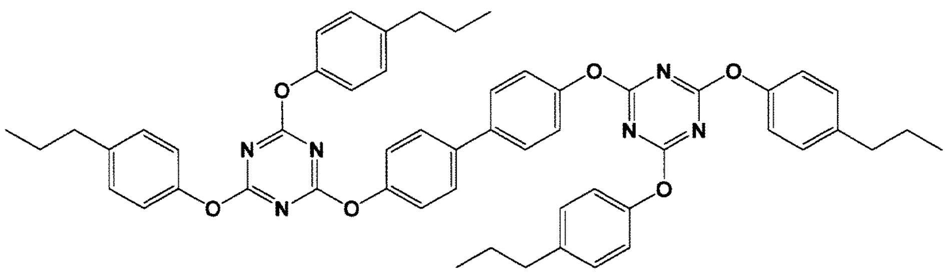

The white solid was then designated as Compound 5.

(Synthesis Example 6)

4.68 g of white solid was obtained in the same manner as in Synthesis Example 2, except that 0.59 g of hydroquinone was used in place of 4,4'-biphenol.

The resulting white solid was subjected to $^1$H-NMR ($CDCl_3$, 400 MHz) analysis. The results of the analyses are shown below.

($^1$H-NMR)
δ(v.s.TMS): 7.17-7.14(m,8H), 7.08(s,4H), 7.04-7.00(m,8H), 2.50-2.45(m,4H), 1.85-1.82(m,16H), 1.76-1.73(m,4H), 1.42-1.31(m,16H), 1.28-1.16(m,4H)
(MALDI-TOF-MS)
m/z = 965 (Dithranol was used as a matrix and sodium trifluoroacetate as an ionization aid)

From the analysis, the obtained white solid was confirmed to be 1",4"-bis[4',6'-bis(4-cyclohexylphenoxy)-1',3',5'-triazine-2'-oxy]benzene having the following structure:

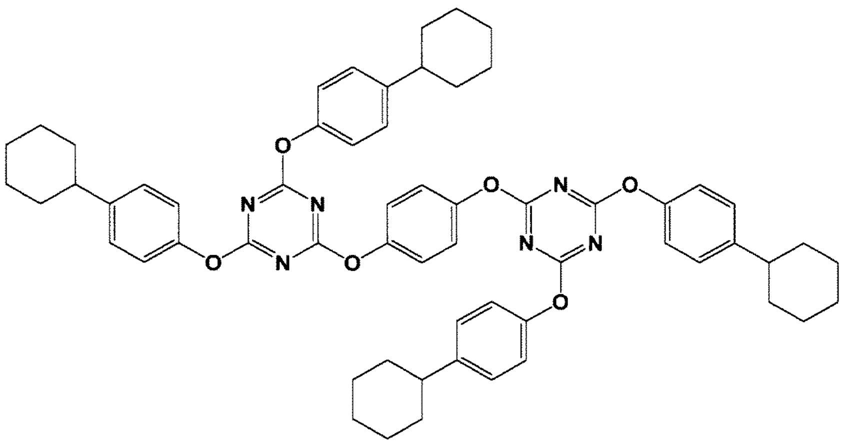

The white solid was then designated as Compound 6.

(Synthesis Example 7)

4.6 g of white solid was obtained in the same manner as in Synthesis Example 1, except that 2.94 g of 4-t-butyl phenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.52-7.50(m,4H), 7.37-7.34(m,8H), 7.22-7.20(m,4H), 7.09-7.07(m,8H), 1.29(s,36H)
(MALDI-TOF-MS)
m/z = 937 (Dithranol was used as a matrix and sodium trifluoroacetate as an ionization aid)

From the analysis, the obtained white solid was confirmed to be 4",4‴-bis[4',6'-bis(4-*t*-butylphenoxy)-1',3',5'-triazine-2'-oxy]biphenyl having the following structure:

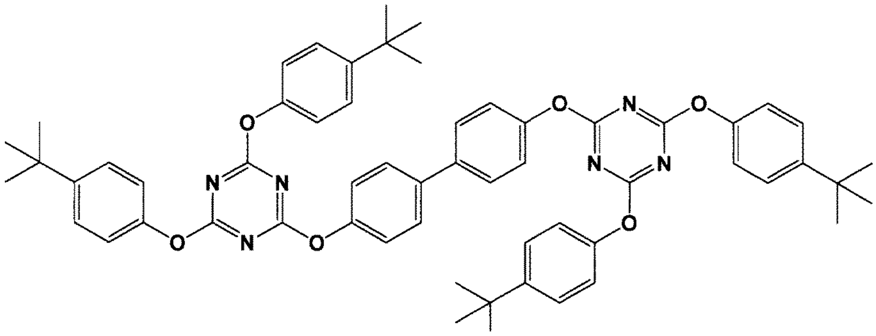

The white solid was then designated as Compound 7.

(Synthesis Example 8)

4.69 g of white solid was obtained in the same manner as in Synthesis Example 1, except that 0.61 g of hydroquinone was used in place of 4,4'-biphenol.

The resulting white solid was subjected to $^1$H-NMR ($CDCl_3$, 400 MHz) analysis. The results of the analyses are shown below.

($^1$H-NMR)
δ(v.s.TMS): 7.56-7.49(m,10H), 7.42-7.38(m,6H), 7.36-7.32(m,4H), 7.22-7.18(m,4H), 7.15-7.08(m,16H),
(MALDI-TOF-MS)
m/z = 941 (Dithranol was used as a matrix and sodium trifluoroacetate as an ionization aid)

From the analysis, the obtained white solid was confirmed to be 1",4"-bis[4',6'-bis(4-biphenyloxy)-1',3',5'-triazine-2'-oxy]benzene having the following structure:

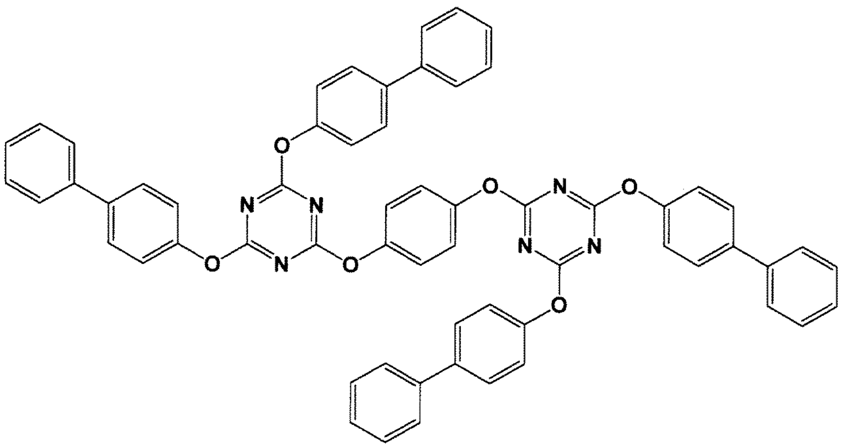

The white solid was then designated as Compound 8.

(Synthesis Example 9)

5.12 g of white solid was obtained in the same manner as in Synthesis Example 2, except that 1.08 g of bis(4-hydroxyphenyl)methane was used in place of 4,4'-biphenol.

The resulting white solid was subjected to $^1$H-NMR ($CDCl_3$, 400 MHz) analysis. The results of the analyses are shown below.

($^1$H-NMR)
δ(v.s.TMS): 7.16-7.13(m,12H), 7.08-7.06(m,4H), 7.04-7.00(m,8H), 3.91(s,2H), 2.55-2.42(m,4H), 1.90-1.80(m,16H), 1.79-1.71(m,4H), 1.42-1.33(m,16H), 1.30-1.19(m,4H)
(MALDI-TOF-MS)

m/z = 1056 (Dithranol was used as a matrix and sodium trifluoroacetate as an ionization aid)

From the analysis, the obtained white solid was confirmed to be {4"-[4',6'-bis(4-cyclohexylphenoxy)-1',3',5'-triazine-2'-oxy]phenyl}methane having the following structure:

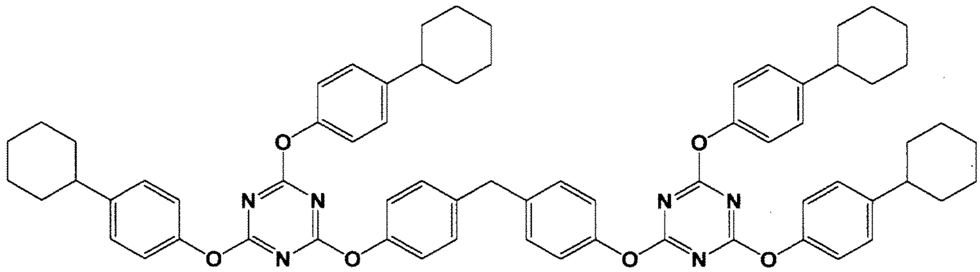

The white solid was then designated as Compound 9.

(Synthesis Example 10)

3.2 g of white solid was obtained in the same manner as in Synthesis Example 1, except that 2.40 g of 3,4-dimethylphenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.53-7.49(m,4H), 7.24-7.20(m,4H), 7.12-7.08(m,4H), 6.93-6.91(m,4H), 6.90-6.85(m,4H), 2.22(s,24H)
(MALDI-TOF-MS)
m/z = 825 (Dithranol was used as a matrix and sodium trifluoroacetate as an ionization aid)

From the analysis, the obtained white solid was confirmed to be 4",4'''-bis[4',6'-bis(3,4-dimethylphenoxy)-1',3',5'-triazine-2'-oxy]biphenyl having the following structure:

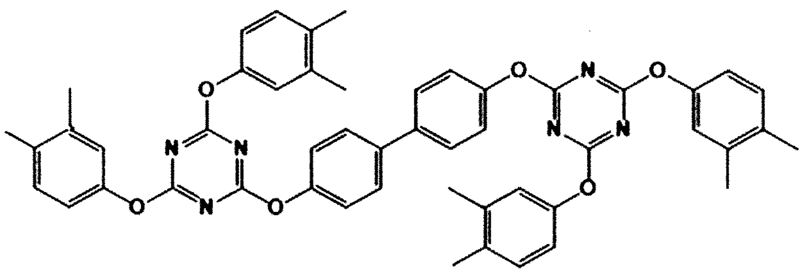

The white solid was then designated as Compound 10.

(Synthesis Example 11)

3.63 g of white solid was obtained in the same manner as in Synthesis Example 1, except that 3.20 g of 3,4-dichlorophenol was used in place of 4-phenylphenol.

The resulting white solid was subjected to $^1$H-NMR ($CDCl_3$, 400 MHz) analysis. The results of the analyses are shown below.

($^1$H-NMR)
δ(v.s.TMS): 7.56-7.54(m,4H), 7.46-7.42(m,4H), 7.30-7.29(m,4H), 7.22-7.18(m,4H), 7.04-7.00(m,4H)
(MALDI-TOF-MS)
m/z = 984 (Dithranol was used as a matrix and sodium trifluoroacetate as an ionization aid)

From the analysis, the obtained white solid was confirmed to be 4",4'''-bis[4',6'-bis(3,4-dichlorophenoxy)-1',3',5'-triazine-2'-oxy]biphenyl having the following structure:

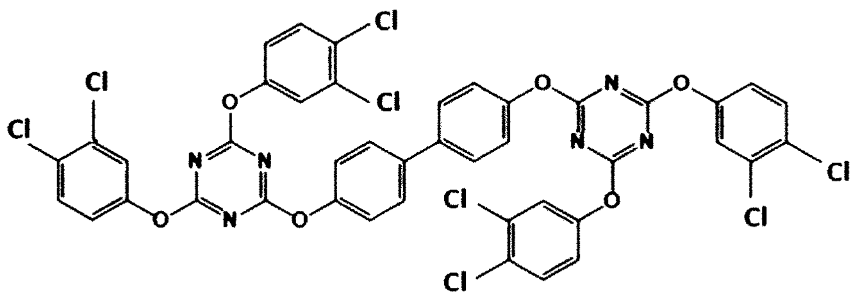

The white solid was then designated as Compound 11.

(Synthesis Example 12)

3.8 g of white solid was obtained in the same manner as in Synthesis Example 5, except that 0.61 g of hydroquinone was used in place of 4,4'-biphenol.
The resulting white solid was subjected to $^1$H-NMR ($CDCl_3$, 400 MHz) analysis. The results of the analyses are shown below.

($^1$H-NMR)
δ(v.s.TMS): 7.18-7.12(m,8H), 7.11-7.09(m,4H), 7.08-6.98(m,8H), 2.56(t,J=7.9Hz,8H), 1.62(dt,J=7.9Hz,7.4Hz,8H), 0.93(t,J=7.4Hz,12H)
(MALDI-TOF-MS)
m/z = 805 (Dithranol was used as a matrix and sodium trifluoroacetate as an ionization aid)

From the analysis, the obtained white solid was confirmed to be 1",4"-bis[4',6'-bis(4-n-propylphenoxy)-1',3',5'-triazine-2'-oxy]benzene having the following structure:

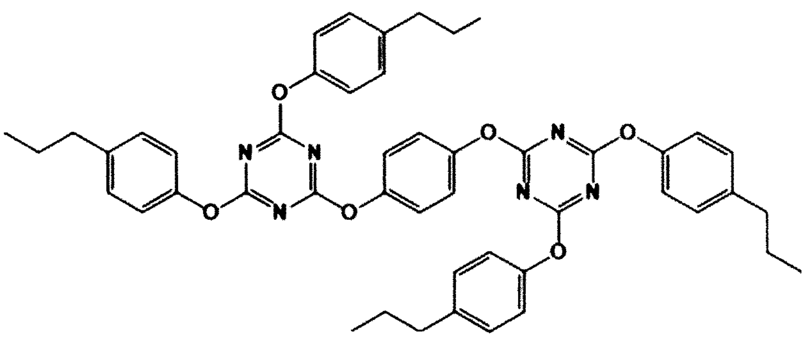

The white solid was then designated as Compound 12.

(Synthesis Example 13)

In a 50-mL three-necked flask equipped with a 20-mL dropping funnel, 0.18 g of cyanuric acid chloride was prepared and dissolved with 10 mL of acetone. The obtained solution was cooled in an ice bath, and while stirring, a solution of 0.34 g of 4-phenylphenol and 0.08 g of sodium hydroxide dissolved in 10 mL distilled water was added dropwise. The dropping rate was adjusted in such a manner that the temperature of a reaction solution during the dropping was 10°C or lower. After the dropping was completed, the ice bath was removed and the reaction solution was stirred for another 1 hour at room temperature. After the stirring was completed, 10 mL of distilled water was added to the reaction solution to precipitate a precipitate. The precipitated precipitate was filtered off, washed with distilled water, and dried under reduced pressure to obtain a white solid. In another 50-mL three-necked flask, 0.10 g of 4,4'-biphenol and 0.10 g of sodium carbonate were prepared, and 6 mL distilled water and 9 mL acetone were added to dissolve. After all of the white solid was added to the obtained solution, the mixture was stirred at room temperature for 3 hours. After the stirring was completed, 12 mL of distilled water was added to the reaction solution to precipitate a precipitate. The precipitated precipitate was filtered off, washed with distilled water, and dried under reduced pressure to obtain a solid. In another 50-mL three-necked flask, 0.06 g of cyanuric acid chloride and 0.04 g of sodium carbonate were

prepared, and 10 mL of acetone was added to dissolve them. After adding all of the solid to the obtained solution, the mixture was stirred at room temperature for 3 hours. After the stirring was completed, 10 mL of distilled water was added to the reaction solution to precipitate a precipitate. The precipitated precipitate was filtered off, washed with distilled water, and dried under reduced pressure to obtain a solid. The obtained solid was purified by recycling preparative high-performance liquid chromatography (HPLC) to obtain 0.42 g of white solid. The HPLC conditions were as follows:

HPLC system: LC-9230II manufactured by Japan Analytical Industry Co., Ltd.
Column: DOCOSIL SP100 manufactured by Senshu Scientific co., ltd.
Detector: UV detector, detection wavelength 220 nm
Eluent: acetonitrile
Flow rate: 2mL/min
Column temperature: 23°C

The resulting white solid was subjected to $^1$H-NMR (DMSO-d6, 400 MHz) analysis. The results of the analyses are shown below.

($^1$H-NMR)
δ(v.s.TMS): 7.72-7.67(m,12H), 7.64-7.59(m,18H), 7.58-7.53(m,6H), 7.47-7.40(m,12H), 7.39-7.23(m,30H)
(MALDI-TOF-MS)
m/z = 1879 (Dithranol was used as a matrix and sodium trifluoroacetate as an ionization aid)

From the analysis, the obtained white solid was confirmed to be 2"",4"",6""-tris[4"-{4',6'-bis(4-biphenyloxy)-1',3',5'-triazine-2'-oxy}-4'''-biphenyloxy]-1"",3"",5""-triazine having the following structure:

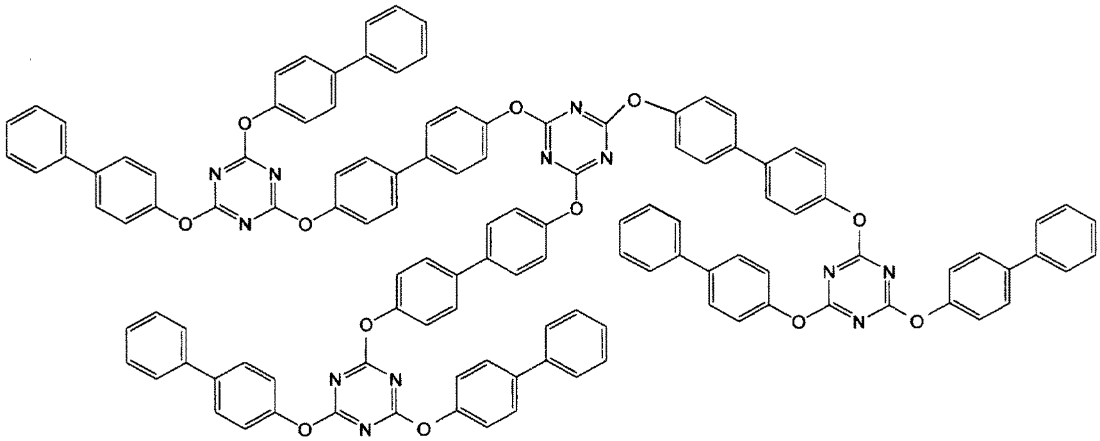

The white solid was then designated as Compound 13.

(Synthesis Example 14)

In a 50-mL three-necked flask equipped with a 20-mL dropping funnel, 0.18 g of cyanuric acid chloride was prepared and dissolved with 10 mL of acetone. The obtained solution was cooled in an ice bath, and while stirring, a solution of 0.29 g of 4-cyclohexylphenol and 0.07 g of sodium hydroxide dissolved in 10 mL distilled water was added dropwise. The dropping rate was adjusted in such a manner that the temperature of a reaction solution during the dropping was 10°C or lower. After the dropping was completed, the ice bath was removed and the reaction solution was stirred for another 1 hour at room temperature. After the stirring was completed, 10 mL of distilled water was added to the reaction solution to precipitate a precipitate. The precipitated precipitate was filtered off, washed with distilled water, and dried under reduced pressure to obtain a white solid. In another 50-mL three-necked flask, 0.12 g of 4,4'-biphenol and 0.14 g of sodium carbonate were prepared, and 6 mL distilled water and 9 mL acetone were added to dissolve. After all of the white solid was added to the obtained solution, the mixture was stirred at room temperature for 3 hours. After the stirring was completed, 12 mL of distilled water was added to the reaction solution to precipitate a precipitate. The precipitated precipitate was filtered off, washed with distilled water, and dried under reduced pressure to obtain a solid. The obtained solid was purified by recycling preparative high-performance liquid chromatography (HPLC) to obtain 0.34 g of white solid. The HPLC conditions were the same as in Synthesis Example 13.

The resulting white solid was subjected to $^1$H-NMR ($CDCl_3$, 400 MHz) analysis. The results of the analyses are shown below.

($^1$H-NMR)
δ(v.s.TMS): 7.52-7.38(m,8H), 7.26-7.00(m,28H), 2.46-2.44(m,5H), 1.90-1.70(m,24H), 1.51-1.45(m,5H), 1.44-1.33(m,16H), 1.26-1.20(m,5H)
(MALDI-TOF-MS)
m/z = 1478 (Dithranol was used as a matrix and sodium trifluoroacetate as an ionization aid)

From the analysis, the obtained white solid was confirmed to be 2""-(4-cyclohexylphenoxy)-4"",6""-bis[4"-{4’,6’-bis(4-cyclohexylphenoxy)-1’,3’,5’-triazine -2’-oxy}-4‴-biphenyloxy]-1"",3"",5""-triazine having the following structure:

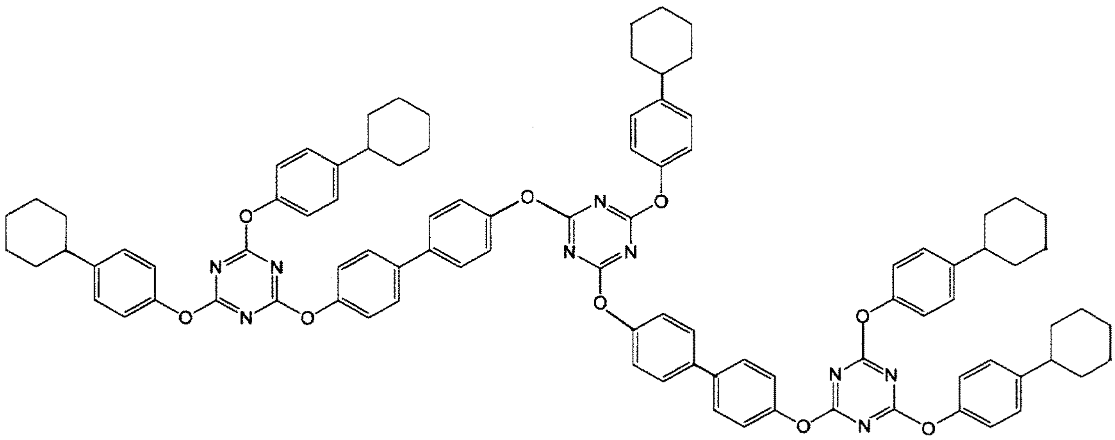

The white solid was then designated as Compound 14.

(Synthesis Example 15)

0.24 g of white solid was obtained in the same manner as in Synthesis Example 14, except that the amount of 4-cyclohexylphenol was changed to 0.26 g, the amount of sodium hydroxide was changed to 0.06 g, the amount of 4,4’-biphenol was changed to 0.14 g, and the amount of sodium carbonate was changed to 0.16 g.

The resulting white solid was subjected to $^1$H-NMR ($CDCl_3$, 400 MHz) analysis. The results of the analyses are shown below.

($^1$H-NMR)
δ(v.s.TMS): 7.55-7.36(m,12H), 7.29-6.98(m,36H), 2.48-2.42(m,6H), 1.93-1.67(m,30H), 1.53-1.42(m,6H), 1.41-1.31(m,18H), 1.26-1.19(m,6H)
(MALDI-TOF-MS)
m/z = 1915 (Dithranol was used as a matrix and sodium trifluoroacetate as an ionization aid)

From the analysis, the obtained white solid was confirmed to be 4″″,4″″″-bis[4″″-(4-cyclohexyphenoxy)-6″″-[4"-{4’,6’-bis(4-cyclohexyphenoxy)-1’,3’,5’-triazine-2’-oxy}-4‴-biphenyloxy]-1″″,3″″,5″″-triazine-2″″-oxy]biphenyl having the following structure:

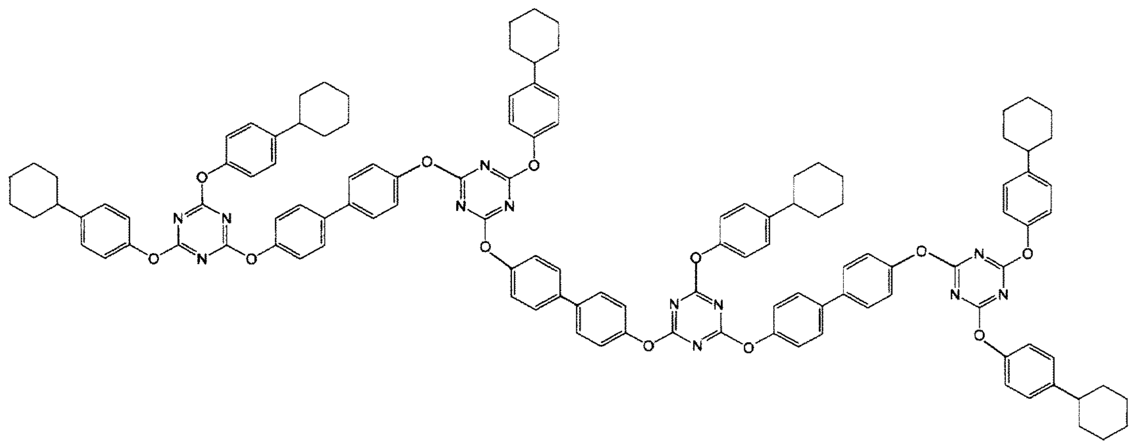

The white solid was then designated as Compound 15.

< Preparation of Resin Composition >

(Examples 1 to 5, 54 to 56)

To 100 parts by mass of homopolypropylene (MFR = 8 g/10 min at 230°C and 2.16 kg load), 0.05 parts by mass of tetrakis[methylene-3-(3',5'-tert-butyl-4'-hydroxyphenyl)propionate]methane, 0.1 parts by mass of tris(2,4-di-tert-butylphenyl)phosphite, 0.05 parts by mass of calcium stearate, and a compound containing a monovalent group represented by general formula (1) synthesized as described above were blended in the amounts listed in Tables 1 and 11 and mixed for 1 minute at 1,000 rpm using FM mixer (manufactured by Mitsui Mining Co., Ltd., FM200). The obtained mixture was fed into a twin-screw extruder (TEX-28V manufactured by The Japan Steel Works, Ltd.), melt kneaded at a melting temperature of 230°C and a screw speed of 150 rpm, and then granulated to obtain a pellet. The obtained pellet was dried at 60°C for 8 hours, and was used as resin compositions of Examples 1 to 5 and 54 to 56. In Table 1 and Table 11, the unit for the blending amount of each component is part by mass.

(Comparative Example 1)

A pellet was prepared in the same manner as in Example 1 except that Compound 1 was not blended, and the obtained pellet was dried at 60°C for 8 hours, and used as the resin composition of Comparative Example 1.

(Examples 6 to 29, 57 to 244)

To 100 parts by mass of random copolymer polypropylene (MFR = 12 g/10 min at 230°C and 2.16 kg load, trade name "Prime Polypro R720" manufactured by Prime Polymer Co., Ltd.), 0.05 parts by mass of tetrakis[methylene-3-(3',5'-*tert*-butyl-4'-hydroxyphenyl)propionate]methane, 0.1 parts by mass of tris(2,4-di-*tert*-butylphenyl)phosphite, 0.05 parts by mass of calcium stearate, a compound containing a monovalent group represented by general formula (1) synthesized as described above, a nucleating agent (other than a compound containing a monovalent group represented by general formula (1)), a lubricant, and a colorant were blended in the amounts listed in Tables 2 to 6 and 12 to 41 and mixed for 1 minute at 1,000 rpm using FM mixer (manufactured by Mitsui Mining Co., Ltd., FM200). The obtained mixture was fed into a twin-screw extruder (TEX-28V manufactured by The Japan Steel Works, Ltd.), melt kneaded at a melting temperature of 230°C and a screw speed of 150 rpm, and then granulated to obtain a pellet. The obtained pellet was dried at 60°C for 8 hours, and was used as resin compositions of Examples 6 to 29 and 57 to 244. In Tables 2 to 6, 12 to 41, the unit for the blending amount of each component is part by mass.

(Comparative Example 2)

A pellet was prepared in the same manner as in Example 6 except that Compound 1 was not blended, and the obtained pellet was dried at 60°C for 8 hours, and used as the resin composition of Comparative Example 2.
Other nucleating agents and lubricants and colorants used in Examples 57-244 are as follows.

[Other Nucleating Agents]

Nucleating agent 1: Bis(3,4-dimethylbenzylidene )sorbitol
Nucleating agent 2: 1,2,3-Trideoxy-4,6:5,7-*o*-bis(4-propylbenzylidene)nonitol
Nucleating agent 3: A mixture of lithium 2,2'-methylenebis(4,6-di-*tert*-butylphenyl)phosphate and stearic acid lithium (mass ratio 4:1)
Nucleating agent 4: A mixture of calcium cyclohexane-1,2-dicarboxylate and zinc stearate (mass ratio 2:1)

[Lubricant]

Lubricant 1: Glycerol stearate monoester
Lubricant 2: Oleic acid amide
Lubricant 3: Erucic acid amide

[Colorant]

Colorant 1: Pigment blue 27
Colorant 2: Acid blue 74
Colorant 3: Pigment violet 15
Colorant 4: Acid blue 9
Colorant 5: Pigment green 7
Colorant 6: Manufactured by Holland Color Company, trade name: Holcobatch 932216

(Examples 30 to 35)

To 100 parts by mass of low density polyethylene (MFR = 2.4 g/10min at 190°C and 2.16 kg load, manufactured by ENEOS NUC Corporation, trade name "NUC-8160"), 0.05 parts by mass of tetrakis[methylene-3-(3',5'-*tert*-butyl-4'-hydroxyphenyl)propionate]methane, 0.1 parts by mass of tris(2,4-di-*tert*-butylphenyl)phosphite, 0.05 parts by mass of calcium stearate, and a compound containing a monovalent group represented by general formula (1) synthesized as described above were blended in the amounts listed in Table 7 and mixed uniformly. The obtained mixture was fed into a twin-screw extruder (Labo Plastomill Micro manufactured by Toyo Seiki Seisaku-sho, Ltd.), melt kneaded at a melting temperature of 210°C and a screw speed of 100 rpm, and then granulated to obtain a pellet. The obtained pellet was dried at 60°C for 8 hours, and was used as resin compositions of Examples 30 to 35. In Table 7, the unit for the blending amount of each component is part by mass.

(Comparative Example 3)

A pellet was prepared in the same manner as in Example 30 except that Compound 1 was not blended, and the obtained pellet was dried at 60°C for 8 hours, and used as the resin composition of Comparative Example 3.

(Examples 36 to 41)

To 100 parts by mass of linear low density polyethylene ($C_4$LLDPE, MFR = 5.0 g/10min at 190°C and 2.16 kg load, manufactured by Japan Polyethylene Corporation, trade name "NOVATEC UR952G"), 0.05 parts by mass of tetrakis[methylene-3-(3',5'-*tert*-butyl-4'-hydroxyphenyl)propionate]methane, 0.1 parts by mass of tris(2,4-di-*tert*-butylphenyl)phosphite, 0.05 parts by mass of calcium stearate, and a compound containing a monovalent group represented by general formula (1) synthesized as described above were blended in the amounts listed in Table 8 and mixed uniformly. The obtained mixture was fed into a twin-screw extruder (Labo Plastomill Micro manufactured by Toyo Seiki Seisaku-sho, Ltd.), melt kneaded at a melting temperature of 210°C and a screw speed of 100 rpm, and then granulated to obtain a pellet. The obtained pellet was dried at 60°C for 8 hours, and was used as resin compositions of Examples 36 to 41. In Table 8, the unit for the blending amount of each component is part by mass.

(Comparative Example 4)

A pellet was prepared in the same manner as in Example 36 except that Compound 1 was not blended, and the obtained pellet was dried at 60°C for 8 hours, and used as the resin composition of Comparative Example 4.

(Examples 42 to 47)

To 100 parts by mass of linear low density polyethylene ($C_6$LLDPE, MFR = 2.9 g/10min at 190°C and 2.16 kg load, manufactured by Japan Polyethylene Corporation, trade name "NOVATEC C6 SF8402G"), 0.05 parts by mass of tetrakis[methylene-3-(3',5'-*tert*-butyl-4'-hydroxyphenyl)propionate]methane, 0.1 parts by mass of tris(2,4-di-*tert*-butylphenyl)phosphite, 0.05 parts by mass of calcium stearate, and a compound containing a monovalent group represented by general formula (1) synthesized as described above were blended in the amounts listed in Table 9 and mixed uniformly. The obtained mixture was fed into a twin-screw extruder (Labo Plastomill Micro manufactured by Toyo Seiki Seisaku-sho, Ltd.), melt kneaded at a melting temperature of 210°C and a screw speed of 100 rpm, and then granulated to obtain a pellet. The obtained pellet was dried at 60°C for 8 hours, and was used as resin compositions of Examples 42 to 47. In Table 9, the unit for the blending amount of each component is part by mass.

(Comparative Example 5)

A pellet was prepared in the same manner as in Example 42 except that Compound 1 was not blended, and the obtained pellet was dried at 60°C for 8 hours, and used as the resin composition of Comparative Example 5.

(Examples 48 to 53)

To 100 parts by mass of polyolefin thermoplastic elastomer (MFR = 40 g/10 min at 230°C, 10 kg load, manufactured by Mitsui Chemicals, Inc., trade name "MILASTOMER 6030NS"), 0.05 parts by mass of tetrakis[methylene-3-(3',5'-*tert*-butyl-4'-hydroxyphenyl)propionate]methane, 0.1 parts by mass of tris(2,4-di-*tert*-butylphenyl)phosphite, 0.05 parts by mass of calcium stearate, and a compound containing a monovalent group represented by general formula (1) synthesized as described above were blended in the amounts listed in Table 10 and mixed using an FM mixer (manufactured by Mitsui Mining Co., Ltd., FM200) at 1000 rpm for 1 minute. The obtained mixture was fed into a twin-screw extruder (TEX-28V manufactured by The Japan Steel Works, Ltd.), melt kneaded at a melting temperature of 200°C and a screw speed of 150 rpm, and then granulated to obtain a pellet. The obtained pellet was dried at 60°C for 8 hours, and was used as resin compositions of Examples 48 to 53. In Table 10, the unit for the blending amount of each component is part by mass.

(Comparative Example 6)

A pellet was prepared in the same manner as in Example 48 except that Compound 1 was not blended, and the obtained pellet was dried at 60°C for 8 hours, and used as the resin composition of Comparative Example 6.

< Property Evaluation >

(Crystallinity)

The crystallization temperatures of the resin compositions of Examples 1 to 56 and Comparative Examples 1 to 6 were measured by differential thermal analysis, and used as indices of the crystallinity of the resin compositions. Specifically, a pellet of a resin composition was introduced into differential scanning calorimetry (manufactured by Perkin Elmer, Diamond), the temperature was increased from room temperature to 230°C at a rate of 50°C/min under a nitrogen atmosphere, held for 20 minutes, and then cooled to 50°C at - 10°C/min, and the peak top temperature of the exothermic peak during the cooling process was defined as the crystallization temperature (°C). The results are shown together in Tables 1 to 11.

(Transparency)

The Haze values of molded articles composed of the resin compositions of Examples 1 to 29, 57 to 129, and Comparative Examples 1 to 2 were measured, and used as indices of the transparency of the molded articles. Specifically, pellets of the resin compositions of Examples 1 to 29, 57 to 129, and Comparative Examples 1 to 2 were injection molded using an injection molding machine (manufactured by Toshiba Machine Co., Ltd., EC100-2A) at a resin temperature of

230°C and a mold temperature of 50°C to prepare square sheet test pieces of 60 mm × 60 mm × 2 mm. The test pieces were allowed to stand still for 7 days in a constant temperature and humidity machine at 23°C and 60% RH, then the test pieces were removed from the machine, and the Haze values of the test pieces were measured with a Haze meter (manufactured by Toyo Seiki Mfg. Co., Ltd., Haze Guard 2). The results are shown together in Tables 1 to 6 and 12 to 25.

(Appearance)

Pellets of the resin compositions of Examples 130 to 244 were injection molded using an injection molding machine (manufactured by Toshiba Machine Co., Ltd., EC60NII) at a resin temperature of 230°C and a mold temperature of 40°C to prepare square sheet test pieces of 60 mm × 60 mm × 2 mm. The test pieces were placed in a constant temperature and humidity machine at 23°C and 60% RH for 7 days, then removed from the machine, and measured for a* and b* using a colorimeter (manufactured by X-rite, Inc. Color-Eye 7000A). Specifically, a test piece was placed on a white calibration plate, subjected to reflected color measurement using the SCI method (including normal reflected light) with a light source of D65 and a viewing angle of 10°, and L*, a*, b*, and YI were calculated from the obtained tristimulus values X, Y, and Z. The obtained L*, a*, b*, and YI values are shown together in Tables 26 to 41. Furthermore, the appearance of the test pieces was evaluated by visually observing the light from a fluorescent lamp (manufactured by Panasonic Corporation, Hf fluorescent lamp, model number FHF32EX-N-H) through the test pieces. Specifically, the color tone of the fluorescent lamp light observed through a test piece was compared with the color tone of the fluorescent lamp light observed directly without passing through the test piece, and when no difference between the two could be perceived, it was determined to be "Acceptable", and when a difference between the two could be perceived, it was determined to be "Unacceptable". As a result of this evaluation, the appearance of the test pieces prepared from the resin compositions of Examples 130 to 244 were all determined "Acceptable".

[Table 1]

| | Example 1 | Example 2 | Example 3 | Example 4 | Example 5 | Comparative Example 1 |
|---|---|---|---|---|---|---|
| Compound 1 | 0.03 | 0.05 | 0.08 | - | - | - |
| Compound 2 | - | - | - | 0.03 | 0.05 | - |
| Crystallization temperature (°C) | 130.9 | 131.2 | 130.7 | 127.7 | 129.8 | 113.3 |
| Haze (%) | 34.5 | 39.5 | 58.1 | 49.3 | 36.0 | 83.2 |

[Table 2]

| | Example 6 | Example 7 | Example 8 | Example 9 | Example 10 | Example 11 |
|---|---|---|---|---|---|---|
| Compound 1 | 0.02 | 0.03 | - | - | - | - |
| Compound 2 | - | - | 0.03 | 0.05 | 0.1 | 0.15 |
| Compound 3 | - | - | - | - | - | - |
| Crystallization temperature (°C) | 121.7 | 122.1 | 119.4 | 121.8 | 122.1 | 122.3 |
| Haze (%) | 18.5 | 19.2 | 27.3 | 12.2 | 9.1 | 9.5 |

[Table 3]

| | Example 12 | Example 13 | Example 14 | Example 15 | Comparative Example 2 |
|---|---|---|---|---|---|
| Compound 1 | - | - | - | - | - |
| Compound 2 | 0.2 | - | - | - | - |
| Compound 3 | - | 0.05 | 0.1 | 0.15 | - |
| Crystallization temperature (°C) | 120.8 | 111.1 | 114.7 | 117.3 | 104.2 |
| Haze (%) | 10.1 | 13.9 | 20.6 | 46.9 | 75.9 |

[Table 4]

| | Example 16 | Example 17 | Example 18 | Example 19 | Example 20 | Example 21 | Comparative Example 2 |
|---|---|---|---|---|---|---|---|
| Compound 4 | 0.03 | 0.05 | - | - | - | - | - |
| Compound 5 | - | - | 0.03 | 0.05 | - | - | - |
| Compound 6 | - | - | - | - | 0.03 | 0.05 | - |
| Crystallization temperature (°C) | 118.9 | 121.1 | 115.6 | 117.9 | 119.5 | 123.7 | 104.2 |
| Haze (%) | 47.0 | 38.6 | 69.5 | 52.9 | 60.4 | 26.3 | 75.9 |

[Table 5]

| | Example 22 | Example 23 | Example 24 | Example 25 | Example 26 |
|---|---|---|---|---|---|
| Compound 10 | 0.05 | - | - | - | - |
| Compound 11 | - | 0.05 | - | - | - |
| Compound 12 | - | - | 0.05 | - | - |
| Compound 13 | - | - | - | 0.03 | - |
| Compound 14 | - | - | - | - | 0.05 |
| Compound 15 | - | - | - | - | - |
| Crystallization temperature (°C) | 109.9 | 112.2 | 113.0 | 120.4 | 123.9 |
| Haze (%) | 62.0 | 57.9 | 50.8 | 27.6 | 61.9 |

[Table 6]

| | Example 27 | Example 28 | Example 29 | Comparative Example 2 |
|---|---|---|---|---|
| Compound 10 | - | - | - | - |
| Compound 11 | - | - | - | - |
| Compound 12 | - | - | - | - |
| Compound 13 | - | - | - | - |
| Compound 14 | - | 0.05 | - | - |
| Compound 15 | 0.1 | - | 0.1 | - |
| Crystallization temperature (°C) | 124.1 | 123.8 | 124.0 | 104.2 |
| Haze (%) | 23.9 | 63.3 | 25.1 | 75.9 |

[Table 7]

| | Example 30 | Example 31 | Example 32 | Example 33 | Example 34 | Example 35 | Comparative Example 3 |
|---|---|---|---|---|---|---|---|
| Compound 1 | 0.1 | - | - | - | - | - | - |
| Compound 2 | - | 0.1 | - | - | - | - | - |
| Compound 3 | - | - | 0.1 | - | - | - | - |
| Compound 4 | - | - | - | 0.1 | - | - | - |

(continued)

| | Example 30 | Example 31 | Example 32 | Example 33 | Example 34 | Example 35 | Comparative Example 3 |
|---|---|---|---|---|---|---|---|
| Compound 5 | - | - | - | - | 0.1 | - | - |
| Compound 6 | - | - | - | - | - | 0.1 | - |
| Crystallization temperature (°C) | 98.6 | 95.5 | 97.4 | 98.2 | 97.9 | 98.3 | 97.1 |

[Table 8]

| | Example 36 | Example 37 | Example 38 | Example 39 | Example 40 | Example 41 | Comparative Example 4 |
|---|---|---|---|---|---|---|---|
| Compound 1 | 0.1 | - | - | - | - | - | - |
| Compound 2 | - | 0.1 | - | - | - | - | - |
| Compound 3 | - | - | 0.1 | - | - | - | - |
| Compound 4 | - | - | - | 0.1 | - | - | - |
| Compound 5 | - | - | - | - | 0.1 | - | - |
| Compound 6 | - | - | - | - | - | 0.1 | - |
| Crystallization temperature (°C) | 113.5 | 113.4 | 112.3 | 113.0 | 112.7 | 113.2 | 111.4 |

[Table 9]

| | Example 42 | Example 43 | Example 44 | Example 45 | Example 46 | Example 47 | Comparative Example 5 |
|---|---|---|---|---|---|---|---|
| Compound 1 | 0.1 | - | - | - | - | - | - |
| Compound 2 | - | 0.1 | - | - | - | - | - |
| Compound 3 | - | - | 0.1 | - | - | - | - |
| Compound 4 | - | - | - | 0.1 | - | - | - |
| Compound 5 | - | - | - | - | 0.1 | - | - |
| Compound 6 | - | - | - | - | - | 0.1 | - |
| Crystallization temperature (°C) | 113.0 | 112.7 | 111.1 | 112.4 | 111.9 | 112.6 | 110.2 |

[Table 10]

| | Example 48 | Example 49 | Example 50 | Example 51 | Example 52 | Example 53 | Comparative Example 6 |
|---|---|---|---|---|---|---|---|
| Compound 1 | 0.1 | - | - | - | - | - | - |
| Compound 2 | - | 0.1 | - | - | - | - | - |
| Compound 3 | - | - | 0.1 | - | - | - | - |
| Compound 4 | - | - | - | 0.1 | - | - | - |
| Compound 5 | - | - | - | - | 0.1 | - | - |
| Compound 6 | - | - | - | - | - | 0.1 | - |
| Crystallization temperature (°C) | 123.2 | 123.0 | 119.1 | 122.5 | 121.3 | 123.0 | 110.0 |

[Table 11]

| | Example 54 | Example 55 | Example 56 | Comparative Example 1 |
|---|---|---|---|---|
| Compound 7 | 0.2 | - | - | - |
| Compound 8 | - | 0.1 | - | - |
| Compound 9 | - | - | 0.2 | - |
| Crystallization temperature (°C) | 123.7 | 130.4 | 116.0 | 113.3 |

[Table 12]

| | Example 57 | Example 58 | Example 59 | Example 60 | Example 61 | Example 62 |
|---|---|---|---|---|---|---|
| Compound 1 | 0.02 | 0.03 | - | - | - | - |
| Compound 2 | - | - | 0.03 | 0.1 | - | - |
| Compound 3 | - | - | - | - | 0.05 | 0.1 |
| Compound 4 | - | - | - | - | - | - |
| Compound 5 | - | - | - | - | - | - |
| Compound 6 | - | - | - | - | - | - |
| Nucleating agent 1 | 0.2 | 0.2 | 0.2 | 0.2 | 0.2 | 0.2 |
| Haze (%) | 17.1 | 17.4 | 24.1 | 8.5 | 12.4 | 18.6 |

[Table 13]

| | Example 63 | Example 64 | Example 65 | Example 66 | Example 67 | Example 68 |
|---|---|---|---|---|---|---|
| Compound 1 | - | - | - | - | - | - |
| Compound 2 | - | - | - | - | - | - |
| Compound 3 | - | - | - | - | - | - |

(continued)

| | Example 63 | Example 64 | Example 65 | Example 66 | Example 67 | Example 68 |
|---|---|---|---|---|---|---|
| Compound 4 | 0.03 | 0.05 | - | - | - | - |
| Compound 5 | - | - | 0.03 | 0.05 | - | - |
| Compound 6 | - | - | - | - | 0.03 | 0.05 |
| Nucleating agent 1 | 0.2 | 0.2 | 0.2 | 0.2 | 0.2 | 0.2 |
| Haze (%) | 24.1 | 23.6 | 24.4 | 23.9 | 24.3 | 22.8 |

[Table 14]

| | Example 69 | Example 70 | Example 71 | Example 72 | Example 73 | Example 74 |
|---|---|---|---|---|---|---|
| Compound 1 | 0.02 | 0.03 | - | - | - | - |
| Compound 2 | - | - | 0.03 | 0.1 | - | - |
| Compound 3 | - | - | - | - | 0.05 | 0.1 |
| Compound 4 | - | - | - | - | - | - |
| Compound 5 | - | - | - | - | - | - |
| Compound 6 | - | - | - | - | - | - |
| Nucleating agent 2 | 0.2 | 0.2 | 0.2 | 0.2 | 0.2 | 0.2 |
| Haze (%) | 18.2 | 18.5 | 26.9 | 8.6 | 12.6 | 19.0 |

[Table 15]

| | Example 75 | Example 76 | Example 77 | Example 78 | Example 79 | Example 80 |
|---|---|---|---|---|---|---|
| Compound 1 | - | - | - | - | - | - |
| Compound 2 | - | - | - | - | - | - |
| Compound 3 | - | - | - | - | - | - |
| Compound 4 | 0.03 | 0.05 | - | - | - | - |
| Compound 5 | - | - | 0.03 | 0.05 | - | - |
| Compound 6 | - | - | - | - | 0.03 | 0.05 |
| Nucleating agent 2 | 0.2 | 0.2 | 0.2 | 0.2 | 0.2 | 0.2 |
| Haze (%) | 29.3 | 28.8 | 29.6 | 29.1 | 29.5 | 25.3 |

[Table 16]

| | Example 81 | Example 82 | Example 83 | Example 84 | Example 85 | Example 86 |
|---|---|---|---|---|---|---|
| Compound 1 | 0.02 | 0.03 | - | - | - | - |
| Compound 2 | - | - | 0.03 | 0.1 | - | - |
| Compound 3 | - | - | - | - | 0.05 | 0.1 |
| Compound 4 | - | - | - | - | - | - |
| Compound 5 | - | - | - | - | - | - |
| Compound 6 | - | - | - | - | - | - |
| Nucleating agent 2 | 0.4 | 0.4 | 0.4 | 0.4 | 0.4 | 0.4 |

(continued)

| | Example 81 | Example 82 | Example 83 | Example 84 | Example 85 | Example 86 |
|---|---|---|---|---|---|---|
| Haze (%) | 12.8 | 13.8 | 16.2 | 7.9 | 10.8 | 14.1 |

[Table 17]

| | Example 87 | Example 88 | Example 89 | Example 90 | Example 91 | Example 92 |
|---|---|---|---|---|---|---|
| Compound 1 | - | - | - | - | - | - |
| Compound 2 | - | - | - | - | - | - |
| Compound 3 | - | - | - | - | - | - |
| Compound 4 | 0.03 | 0.05 | - | - | - | - |
| Compound 5 | - | - | 0.03 | 0.05 | - | - |
| Compound 6 | - | - | - | - | 0.03 | 0.05 |
| Nucleating agent 2 | 0.4 | 0.4 | 0.4 | 0.4 | 0.4 | 0.4 |
| Haze (%) | 16.5 | 16.2 | 16.3 | 16.3 | 16.2 | 15.9 |

[Table 18]

| | Example 93 | Example 94 | Example 95 | Example 96 | Example 97 |
|---|---|---|---|---|---|
| Compound 1 | 0.03 | - | - | - | - |
| Compound 2 | - | 0.03 | - | - | - |
| Compound 3 | - | - | 0.05 | - | - |
| Compound 4 | - | - | - | 0.03 | - |
| Compound 6 | - | - | - | - | 0.05 |
| Nucleating agent 3 | 0.003 | 0.003 | 0.005 | 0.003 | 0.005 |
| Haze (%) | 18.9 | 25.4 | 13.4 | 45.9 | 24.8 |

[Table 19]

| | Example 98 | Example 99 | Example 100 | Example 101 | Example 102 |
|---|---|---|---|---|---|
| Compound 1 | 0.03 | - | - | - | - |
| Compound 2 | - | 0.03 | - | - | - |
| Compound 3 | - | - | 0.05 | - | - |
| Compound 4 | - | - | - | 0.03 | - |
| Compound 6 | - | - | - | - | 0.05 |
| Nucleating agent 4 | 0.003 | 0.003 | 0.005 | 0.003 | 0.005 |
| Haze (%) | 19.0 | 26.0 | 13.5 | 46.3 | 25.1 |

[Table 20]

| | Example 103 | Example 104 | Example 105 | Example 106 | Example 6 |
|---|---|---|---|---|---|
| Compound 1 | 0.02 | 0.02 | 0.02 | 0.02 | 0.02 |

(continued)

| | Example 103 | Example 104 | Example 105 | Example 106 | Example 6 |
|---|---|---|---|---|---|
| Lubricant 1 | 0.02 | 0.04 | - | - | - |
| Lubricant 2 | - | - | 0.017 | - | - |
| Lubricant 3 | - | - | - | 0.02 | - |
| Haze (%) | 18.1 | 18.3 | 18.0 | 18.1 | 18.5 |

[Table 21]

| | Example 107 | Example 108 | Example 109 | Example 110 | Example 111 |
|---|---|---|---|---|---|
| Compound 2 | 0.03 | 0.03 | 0.03 | 0.03 | 0.03 |
| Lubricant 1 | 0.03 | 0.06 | - | - | - |
| Lubricant 2 | - | - | 0.005 | 0.01 | 0.02 |
| Lubricant 3 | - | - | - | - | - |
| Haze (%) | 24.8 | 25.0 | 27.0 | 26.1 | 21.0 |

[Table 22]

| | Example 112 | Example 113 | Example 114 | Example 8 |
|---|---|---|---|---|
| Compound 2 | 0.03 | 0.03 | 0.03 | 0.03 |
| Lubricant 1 | - | - | - | - |
| Lubricant 2 | 0.024 | 0.09 | - | - |
| Lubricant 3 | - | - | 0.03 | - |
| Haze (%) | 22.3 | 26.5 | 26.5 | 27.3 |

[Table 23]

| | Example 115 | Example 116 | Example 117 | Example 118 | Example 13 |
|---|---|---|---|---|---|
| Compound 3 | 0.05 | 0.05 | 0.05 | 0.05 | 0.05 |
| Lubricant 1 | 0.05 | 0.1 | - | - | - |
| Lubricant 2 | - | - | 0.033 | - | - |
| Lubricant 3 | - | - | - | 0.05 | - |
| Haze (%) | 13.3 | 13.4 | 13.0 | 13.1 | 13.9 |

[Table 24]

| | Example 119 | Example 120 | Example 121 | Example 122 | Example 16 |
|---|---|---|---|---|---|
| Compound 4 | 0.03 | 0.03 | 0.03 | 0.03 | 0.03 |
| Lubricant 1 | 0.03 | 0.06 | - | - | - |
| Lubricant 2 | - | - | 0.02 | - | - |
| Lubricant 3 | - | - | - | 0.03 | - |
| Haze (%) | 44.9 | 46.2 | 44.3 | 45.2 | 47.0 |

[Table 25]

| | Example 123 | Example 124 | Example 125 | Example 126 | Example 127 | Example 128 | Example 129 | Example 20 |
|---|---|---|---|---|---|---|---|---|
| Compound 6 | 0.03 | 0.03 | 0.03 | 0.03 | 0.03 | 0.03 | 0.03 | 0.03 |
| Lubricant 1 | 0.03 | 0.06 | - | - | - | - | - | - |
| Lubricant 2 | - | - | 0.01 | 0.02 | 0.03 | 0.04 | - | - |
| Lubricant 3 | - | - | - | - | - | - | 0.03 | - |
| Haze (%) | 57.4 | 58.9 | 57.9 | 57.5 | 58.3 | 59.0 | 58.0 | 60.4 |

[Table 26]

| | Example 130 | Example 131 | Example 132 | Example 133 | Example 134 | Example 135 | Example 136 | Example 137 |
|---|---|---|---|---|---|---|---|---|
| Compound 1 | 0.02 | 0.02 | 0.02 | 0.02 | 0.02 | 0.02 | 0.02 | 0.02 |
| Colorant 1 | 0.002 | 0.005 | 0.01 | 0.002 | 0.002 | 0.002 | - | - |
| Colorant 2 | - | - | - | - | - | - | 0.002 | 0.005 |
| Colorant 3 | - | - | - | 0.0002 | 0.001 | 0.0025 | - | - |
| Colorant 4 | - | - | - | - | - | - | - | - |
| Colorant 5 | - | - | - | - | - | - | - | - |
| Colorant 6 | - | - | - | - | - | - | - | - |
| L* | 92.70 | 92.04 | 91.31 | 92.37 | 91.89 | 90.82 | 92.45 | 91.80 |
| a* | 0.05 | -0.14 | -0.45 | -0.41 | -2.06 | -5.37 | -0.01 | -0.26 |
| b* | 3.67 | 2.83 | 1.64 | 3.58 | 3.48 | 3.37 | 3.65 | 3.20 |
| YI | 7.17 | 5.41 | 2.95 | 6.64 | 5.18 | 2.41 | 7.08 | 6.10 |

[Table 27]

| | Example 138 | Example 139 | Example 140 | Example 141 | Example 142 | Example 143 |
|---|---|---|---|---|---|---|
| Compound 1 | 0.02 | 0.02 | 0.02 | 0.02 | 0.02 | 0.02 |
| Colorant 1 | - | - | - | - | - | - |
| Colorant 2 | - | - | - | - | - | - |
| Colorant 3 | 0.0002 | 0.001 | 0.0025 | 0.0002 | 0.001 | 0.001 |
| Colorant 4 | - | - | - | 0.005 | 0.005 | - |
| Colorant 5 | - | - | - | - | - | 0.02 |
| Colorant 6 | - | - | - | - | - | - |
| L* | 92.75 | 92.10 | 91.32 | 90.09 | 89.48 | 91.04 |
| a* | -0.28 | -2.01 | -4.96 | -2.99 | -4.85 | -1.61 |

(continued)

| | Example 138 | Example 139 | Example 140 | Example 141 | Example 142 | Example 143 |
|---|---|---|---|---|---|---|
| b* | 4.01 | 3.96 | 3.91 | 1.48 | 1.31 | 2.71 |
| YI | 7.51 | 6.15 | 3.78 | 0.56 | -1.28 | 4.03 |

[Table 28]

| | Example 144 | Example 145 | Example 146 | Example 147 | Example 148 | Example 149 |
|---|---|---|---|---|---|---|
| Compound 1 | 0.02 | 0.02 | 0.02 | 0.02 | 0.02 | 0.02 |
| Colorant 1 | - | - | - | - | - | - |
| Colorant 2 | - | - | - | - | - | - |
| Colorant 3 | - | - | - | - | - | - |
| Colorant 4 | 0.002 | 0.005 | - | - | - | - |
| Colorant 5 | - | - | 0.01 | 0.02 | - | - |
| Colorant 6 | - | - | - | - | 0.0002 | 0.0005 |
| L* | 91.80 | 90.28 | 92.36 | 91.92 | 92.49 | 91.77 |
| a* | -0.98 | -2.60 | 0.46 | 0.80 | 0.12 | 0.23 |
| b* | 2.95 | 1.39 | 3.43 | 2.84 | 4.10 | 4.39 |
| YI | 5.01 | 0.73 | 7.05 | 6.16 | 8.04 | 8.70 |

[Table 29]

| | Example 150 | Example 151 | Example 152 | Example 153 | Example 154 | Example 155 | Example 156 | Example 157 |
|---|---|---|---|---|---|---|---|---|
| Compound 2 | 0.1 | 0.1 | 0.1 | 0.1 | 0.1 | 0.1 | 0.1 | 0.1 |
| Colorant 1 | 0.002 | 0.005 | 0.01 | 0.002 | 0.002 | 0.002 | - | - |
| Colorant 2 | - | - | - | - | - | - | 0.0002 | 0.002 |
| Colorant 3 | - | - | - | 0.0002 | 0.001 | 0.0025 | - | - |
| Colorant 4 | - | - | - | - | - | - | - | - |
| Colorant 5 | - | - | - | - | - | - | - | - |
| Colorant 6 | - | - | - | - | - | - | - | - |
| L* | 92.69 | 92.13 | 91.32 | 92.29 | 91.82 | 90.87 | 92.82 | 92.45 |
| a* | 0.07 | -0.15 | -0.45 | -0.39 | -2.05 | -5.38 | 0.07 | -0.02 |
| b* | 3.66 | 2.85 | 1.66 | 3.57 | 3.48 | 3.37 | 3.94 | 3.67 |
| YI | 7.14 | 5.45 | 2.92 | 6.63 | 5.18 | 2.38 | 7.68 | 7.10 |

[Table 30]

| | Example 158 | Example 159 | Example 160 | Example 161 | Example 162 | Example 163 | Example 164 | Example 165 |
|---|---|---|---|---|---|---|---|---|
| Compound 2 | 0.1 | 0.1 | 0.1 | 0.1 | 0.1 | 0.1 | 0.1 | 0.1 |
| Colorant 1 | - | - | - | - | - | - | - | - |
| Colorant 2 | 0.005 | 0.01 | - | - | - | - | - | - |
| Colorant 3 | - | - | 0.0002 | 0.001 | 0.0025 | 0.0002 | 0.001 | 0.001 |
| Colorant 4 | - | - | - | - | - | 0.005 | 0.005 | - |
| Colorant 5 | - | - | - | - | - | - | - | 0.02 |
| Colorant 6 | - | - | - | - | - | - | - | - |
| L* | 91.78 | 90.68 | 92.71 | 92.17 | 91.26 | 90.14 | 89.48 | 91.05 |
| a* | -0.25 | -0.59 | -0.27 | -2.02 | -4.97 | -2.98 | -4.84 | -1.63 |
| b* | 3.22 | 2.55 | 3.99 | 3.97 | 3.93 | 1.48 | 1.30 | 2.69 |
| YI | 6.10 | 4.58 | 7.50 | 6.12 | 3.76 | 0.56 | -1.27 | 4.02 |

[Table 31]

| | Example 166 | Example 167 | Example 168 | Example 169 | Example 170 | Example 171 | Example 172 |
|---|---|---|---|---|---|---|---|
| Compound 2 | 0.1 | 0.1 | 0.1 | 0.1 | 0.1 | 0.1 | 0.1 |
| Colorant 1 | - | - | - | - | - | - | - |
| Colorant 2 | - | - | - | - | - | - | - |
| Colorant 3 | - | - | - | - | - | - | - |
| Colorant 4 | 0.0002 | 0.002 | 0.005 | - | - | - | - |
| Colorant 5 | - | - | - | 0.01 | 0.02 | - | - |
| Colorant 6 | - | - | - | - | - | 0.0002 | 0.0005 |
| L* | 92.71 | 91.77 | 90.24 | 92.39 | 91.83 | 92.40 | 91.77 |
| a* | 0.04 | -0.97 | -2.60 | 0.46 | 0.80 | 0.11 | 0.23 |
| b* | 3.90 | 2.96 | 1.41 | 3.42 | 2.82 | 4.09 | 4.38 |
| YI | 7.58 | 5.03 | 0.73 | 7.02 | 6.18 | 8.00 | 8.70 |

[Table 32]

| | Example 173 | Example 174 | Example 175 | Example 176 | Example 177 | Example 178 | Example 179 |
|---|---|---|---|---|---|---|---|
| Compound 3 | 0.05 | 0.05 | 0.05 | 0.05 | 0.05 | 0.05 | 0.05 |
| Colorant 1 | 0.002 | 0.005 | 0.01 | 0.002 | 0.002 | 0.002 | - |
| Colorant 3 | - | - | - | 0.0002 | 0.001 | 0.0025 | 0.0002 |
| Colorant 5 | - | - | - | - | - | - | - |

(continued)

| | Example 173 | Example 174 | Example 175 | Example 176 | Example 177 | Example 178 | Example 179 |
|---|---|---|---|---|---|---|---|
| Colorant 6 | - | - | - | - | - | - | - |
| L* | 92.72 | 92.10 | 91.34 | 92.39 | 91.90 | 90.90 | 92.63 |
| a* | 0.05 | -0.16 | -0.47 | -0.40 | -2.03 | -5.37 | -0.28 |
| b* | 3.67 | 2.86 | 1.65 | 3.56 | 3.48 | 3.36 | 4.01 |
| Y | 7.15 | 5.41 | 2.89 | 6.65 | 5.20 | 2.38 | 7.54 |

[Table 33]

| | Example 180 | Example 181 | Example 182 | Example 183 | Example 184 | Example 185 | Example 186 |
|---|---|---|---|---|---|---|---|
| Compound 3 | 0.05 | 0.05 | 0.05 | 0.05 | 0.05 | 0.05 | 0.05 |
| Colorant 1 | - | - | - | - | - | - | - |
| Colorant 3 | 0.001 | 0.0025 | 0.001 | - | - | - | - |
| Colorant 5 | - | - | 0.02 | 0.01 | 0.02 | - | - |
| Colorant 6 | - | - | - | - | - | 0.0002 | 0.0005 |
| L* | 92.25 | 91.30 | 91.05 | 92.40 | 91.82 | 92.47 | 91.68 |
| a* | -2.04 | -4.96 | -1.63 | 0.46 | 0.81 | 0.12 | 0.24 |
| b* | 3.98 | 3.94 | 2.69 | 3.44 | 2.81 | 4.08 | 4.37 |
| YI | 6.08 | 3.76 | 3.99 | 7.04 | 6.20 | 7.97 | 8.67 |

[Table 34]

| | Example 187 | Example 188 | Example 189 | Example 190 | Example 191 | Example 192 | Example 193 |
|---|---|---|---|---|---|---|---|
| Compound 4 | 0.05 | 0.05 | 0.05 | 0.05 | 0.05 | 0.05 | 0.05 |
| Colorant 1 | 0.002 | 0.005 | 0.01 | 0.002 | 0.002 | 0.002 | - |
| Colorant 3 | - | - | - | 0.0002 | 0.001 | 0.0025 | 0.0002 |
| Colorant 5 | - | - | - | - | - | - | - |
| Colorant 6 | - | - | - | - | - | - | - |
| L* | 92.76 | 92.11 | 91.31 | 92.20 | 91.82 | 90.86 | 92.73 |
| a* | 0.05 | -0.14 | -0.46 | -0.38 | -2.05 | -5.39 | -0.27 |
| b* | 3.66 | 2.85 | 1.66 | 3.58 | 3.47 | 3.39 | 3.99 |
| YI | 7.11 | 5.46 | 2.95 | 6.67 | 5.18 | 2.35 | 7.51 |

[Table 35]

| | Example 194 | Example 195 | Example 196 | Example 197 | Example 198 | Example 199 | Example 200 |
|---|---|---|---|---|---|---|---|
| Compound 4 | 0.05 | 0.05 | 0.05 | 0.05 | 0.05 | 0.05 | 0.05 |
| Colorant 1 | - | - | - | - | - | - | - |

(continued)

| | Example 194 | Example 195 | Example 196 | Example 197 | Example 198 | Example 199 | Example 200 |
|---|---|---|---|---|---|---|---|
| Colorant 3 | 0.001 | 0.0025 | 0.001 | - | - | - | - |
| Colorant 5 | - | - | 0.02 | 0.01 | 0.02 | - | - |
| Colorant 6 | - | - | - | - | - | 0.0002 | 0.0005 |
| L* | 92.14 | 91.19 | 91.08 | 92.43 | 91.76 | 92.43 | 91.86 |
| a* | -2.03 | -4.97 | -1.62 | 0.45 | 0.81 | 0.10 | 0.24 |
| b* | 3.97 | 3.92 | 2.69 | 3.42 | 2.81 | 4.07 | 4.36 |
| YI | 6.15 | 3.78 | 3.99 | 7.00 | 6.20 | 8.03 | 8.70 |

[Table 36]

| | Example 201 | Example 202 | Example 203 | Example 204 | Example 205 | Example 206 | Example 207 |
|---|---|---|---|---|---|---|---|
| Compound 5 | 0.05 | 0.05 | 0.05 | 0.05 | 0.05 | 0.05 | 0.05 |
| Colorant 1 | 0.002 | 0.005 | 0.01 | 0.002 | 0.002 | 0.002 | - |
| Colorant 2 | - | - | - | - | - | - | 0.002 |
| Colorant 3 | - | - | - | 0.0002 | 0.001 | 0.0025 | - |
| Colorant 4 | - | - | - | - | - | - | - |
| Colorant 5 | - | - | - | - | - | - | - |
| Colorant 6 | - | - | - | - | - | - | - |
| L* | 92.72 | 92.10 | 91.30 | 92.34 | 91.89 | 90.84 | 92.36 |
| a* | 0.07 | -0.17 | -0.45 | -0.40 | -2.06 | -5.38 | -0.02 |
| b* | 3.66 | 2.86 | 1.65 | 3.59 | 3.48 | 3.37 | 3.65 |
| YI | 7.15 | 5.47 | 2.94 | 6.67 | 5.20 | 2.42 | 7.07 |

[Table 37]

| | Example 208 | Example 209 | Example 210 | Example 211 | Example 212 | Example 213 | Example 214 |
|---|---|---|---|---|---|---|---|
| Compound 5 | 0.05 | 0.05 | 0.05 | 0.05 | 0.05 | 0.05 | 0.05 |
| Colorant 1 | - | - | - | - | - | - | - |
| Colorant 2 | 0.005 | 0.01 | - | - | - | - | - |
| Colorant 3 | - | - | 0.0002 | 0.001 | 0.0025 | 0.0002 | 0.001 |
| Colorant 4 | - | - | - | - | - | 0.005 | 0.005 |
| Colorant 5 | - | - | - | - | - | - | - |
| Colorant 6 | - | - | - | - | - | - | - |
| L* | 91.79 | 90.60 | 92.70 | 92.14 | 91.19 | 90.22 | 89.48 |
| a* | -0.24 | -0.61 | -0.29 | -2.01 | -4.98 | -2.99 | -4.84 |
| b* | 3.21 | 2.55 | 4.01 | 3.99 | 3.93 | 1.46 | 1.30 |
| YI | 6.09 | 4.54 | 7.49 | 6.09 | 3.75 | 0.58 | -1.30 |

[Table 38]

| | Example 215 | Example 216 | Example 217 | Example 218 | Example 219 | Example 220 | Example 221 |
|---|---|---|---|---|---|---|---|
| Compound 5 | 0.05 | 0.05 | 0.05 | 0.05 | 0.05 | 0.05 | 0.05 |
| Colorant 1 | - | - | - | - | - | - | - |
| Colorant 2 | - | - | - | - | - | - | - |
| Colorant 3 | 0.001 | - | - | - | - | - | - |
| Colorant 4 | - | 0.002 | 0.005 | - | - | - | - |
| Colorant 5 | 0.02 | - | - | 0.01 | 0.02 | - | - |
| Colorant 6 | - | - | - | - | - | 0.0002 | 0.0005 |
| L* | 91.01 | 91.78 | 90.24 | 92.46 | 91.82 | 92.45 | 91.78 |
| a* | -1.62 | -0.96 | -2.59 | 0.44 | 0.82 | 0.12 | 0.22 |
| b* | 2.70 | 2.97 | 1.42 | 3.40 | 2.81 | 4.08 | 4.37 |
| YI | 4.04 | 5.00 | 0.72 | 7.01 | 6.18 | 7.98 | 8.71 |

[Table 39]

| | Example 222 | Example 223 | Example 224 | Example 225 | Example 226 | Example 227 | Example 228 | Example 229 |
|---|---|---|---|---|---|---|---|---|
| Compound 6 | 0.05 | 0.05 | 0.05 | 0.05 | 0_05 | 0.05 | 0.05 | 0.05 |
| Colorant 1 | 0.002 | 0.005 | 0.01 | 0.002 | 0.002 | 0.002 | - | - |
| Colorant 2 | - | - | - | - | - | - | 0.0002 | 0.002 |
| Colorant 3 | - | - | - | 0.0002 | 0.001 | 0.0025 | - | - |
| Colorant 4 | - | - | - | - | - | - | - | - |
| Colorant 5 | - | - | - | - | - | - | - | - |
| Colorant 6 | - | - | - | - | - | - | - | - |
| L* | 92.70 | 92.18 | 91.31 | 92.21 | 91.77 | 90.89 | 92.79 | 92.51 |
| a* | 0.06 | -0.17 | -0.44 | -0.40 | -2.06 | -5.36 | 0.08 | -0.01 |
| b* | 3.65 | 2.83 | 1.67 | 3.57 | 3.47 | 3.36 | 3.96 | 3.66 |
| YI | 7.14 | 5.45 | 2.91 | 6.65 | 5.16 | 2.39 | 7.65 | 7.11 |

[Table 40]

| | Example 230 | Example 231 | Example 232 | Example 233 | Example 234 | Example 235 | Example 236 | Example 237 |
|---|---|---|---|---|---|---|---|---|
| Compound 6 | 0.05 | 0.05 | 0.05 | 0.05 | 0.05 | 0.05 | 0.05 | 0.05 |
| Colorant 1 | - | - | - | - | - | - | - | - |
| Colorant 2 | 0.005 | 0.01 | - | - | - | - | - | - |
| Colorant 3 | - | - | 0.0002 | 0.001 | 0.0025 | 0.0002 | 0.001 | 0.001 |
| Colorant 4 | - | - | - | - | - | 0.005 | 0.005 | - |
| Colorant 5 | - | - | - | - | - | - | - | 0.02 |
| Colorant 6 | - | - | - | - | - | - | - | - |
| L* | 91.83 | 90.75 | 92.61 | 92.27 | 91.33 | 90.19 | 89.57 | 91.01 |
| a* | -0.24 | -0.60 | -0.29 | -2.02 | -4.98 | -2.98 | -4.83 | -1.64 |
| b* | 3.22 | 2.53 | 4.01 | 3.98 | 3.93 | 1.49 | 1.29 | 2.70 |
| YI | 6.12 | 4.58 | 7.49 | 6.12 | 3.75 | 0.54 | -1.24 | 4.01 |

[Table 41]

| | Example 238 | Example 239 | Example 240 | Example 241 | Example 242 | Example 243 | Example 244 |
|---|---|---|---|---|---|---|---|
| Compound 6 | 0.05 | 0.05 | 0.05 | 0.05 | 0.05 | 0.05 | 0.05 |
| Colorant 1 | - | - | - | - | - | - | - |
| Colorant 2 | - | - | - | - | - | - | - |
| Colorant 3 | - | - | - | - | - | - | - |
| Colorant 4 | 0.0002 | 0.002 | 0.005 | - | - | - | - |
| Colorant 5 | - | - | - | 0.01 | 0.02 | - | - |
| Colorant 6 | - | - | - | - | - | 0.0002 | 0.0005 |
| L* | 92.77 | 91.86 | 90.25 | 92.44 | 91.75 | 92.32 | 91.70 |
| a* | 0.02 | -0.96 | -2.60 | 0.47 | 0.81 | 0.10 | 0.23 |
| b* | 3.91 | 2.95 | 1.40 | 3.42 | 2.83 | 4.09 | 4.38 |
| YI | 7.54 | 5.06 | 0.75 | 7.05 | 6.15 | 7.97 | 8.66 |

The results shown in Tables 1 to 11 indicate that an additive for synthetic resins composed of a compound containing a monovalent group represented by general formula (1) act as a nucleating agent and improves the crystallinity of a synthetic resin or the transparency of a molded article composed of such a synthetic resin.

The results shown in Tables 12 to 19 indicate that an additive composition for synthetic resins containing a compound containing a monovalent group represented by general formula (1) and a nucleating agent composed of a compound other than the compound containing a monovalent group represented by general formula (1) can impart excellent transparency to a molded article composed of such a synthetic resin.

Furthermore, the results shown in Tables 20 to 25 indicate that an additive composition for synthetic resins containing a compound containing a monovalent group represented by general formula (1) and a lubricant can impart excellent transparency to a molded article composed of such a synthetic resin.

Still further, the results shown in Tables 26 to 41 indicate that an additive composition for synthetic resins

containing a compound containing a monovalent group represented by general formula (1) and a colorant can impart an excellent appearance to a molded article composed of such a synthetic resin.

From the above, it was confirmed that the compounds of the present invention can improve the properties of synthetic resins.

## Claims

1. A compound comprising a monovalent group represented by general formula (1):

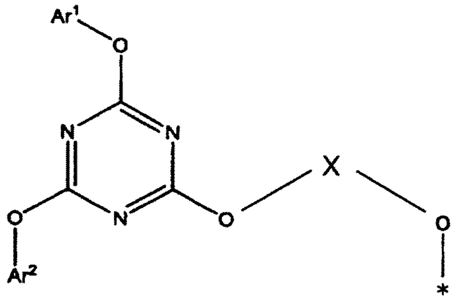

(1)

(where X represents a divalent group, $Ar^1$ and $Ar^2$ each independently represent an unsubstituted or substituted phenyl group, and * represents a site bonding to another atom).

2. The compound according to claim 1, represented by general formula (2):

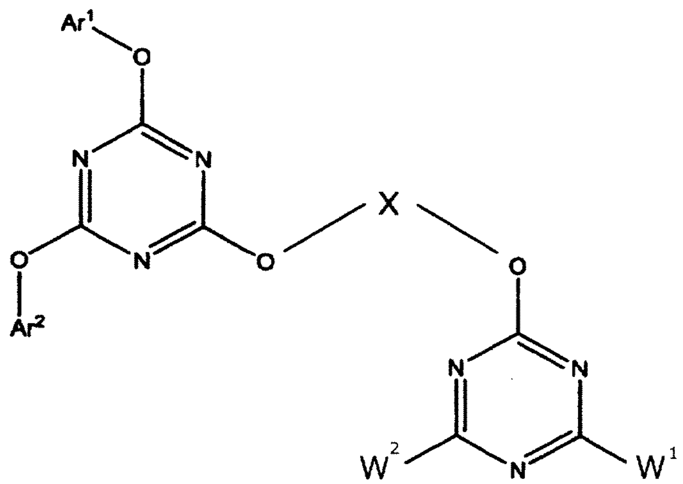

(2)

(where X represents a divalent group, $Ar^1$ and $Ar^2$ each independently represent an unsubstituted or substituent-containing phenyl group, and $W^1$ and $W^2$ each independently represent an unsubstituted or substituted phenyloxy group or a monovalent group represented by general formula (1)).

3. The compound according to claim 1 or 2, represented by general formula (3):

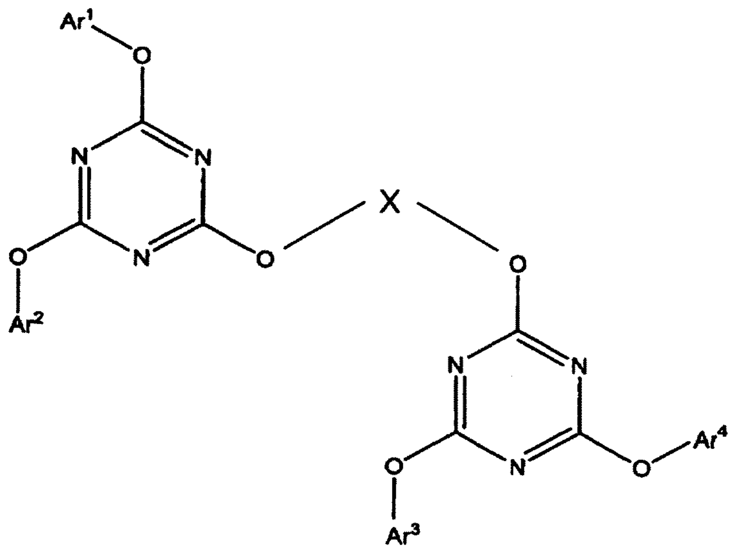

(3)

(where X represents a divalent group, and $Ar^1$, $Ar^2$, $Ar^3$, and $Ar^4$ each independently represent an unsubstituted or substituted phenyl group).

**4.** The compound according to claim 1, represented by general formula (4):

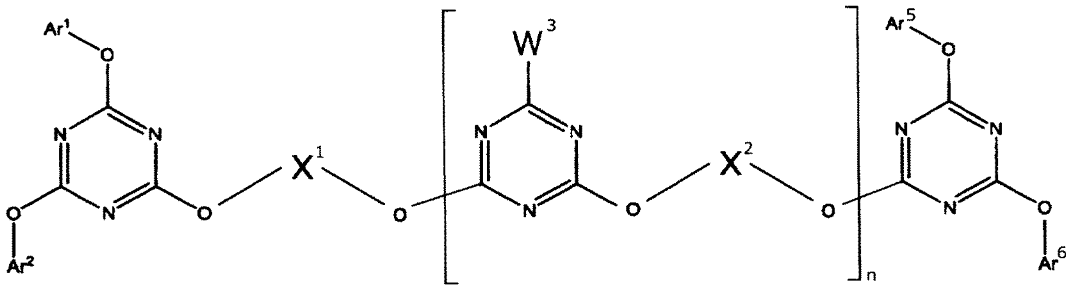

(4)

(where $X^1$ and $X^2$ each independently represent a divalent group, $Ar^1$, $Ar^2$, $Ar^5$, and $Ar^6$ each independently represent an unsubstituted or substituted phenyl group, $W^3$ represents an unsubstituted or substituted phenyloxy group or a monovalent group represented by general formula (1), and n represents an integer of 1 or more).

**5.** The compound according to any one of claims 1 to 3, wherein X is a group represented by general formula (5) or (6):

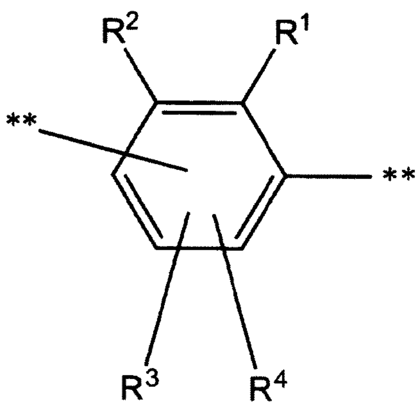

(5)

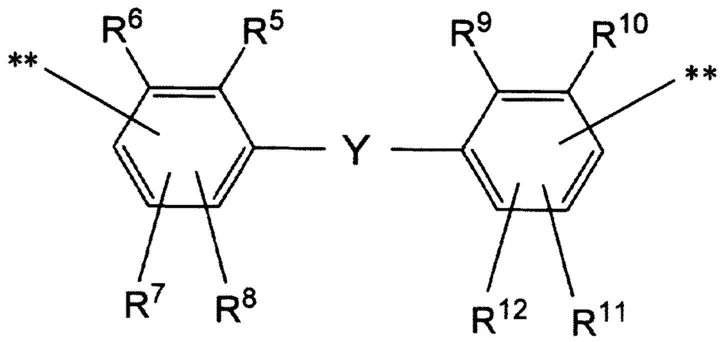

(6)

(where ** represents a site bonding to an oxygen atom, $R^1$ to $R^{12}$ each independently represent a hydrogen atom, an alkyl group having from 1 to 10 carbon atoms, an alkoxy group having from 1 to 10 carbon atoms, or a halogen atom, and Y represents a single bond, an oxygen atom, a sulfur atom, a sulfonyl group, or an unsubstituted or substituted alkanediyl group).

**6.** The compound according to claim 4, wherein $X^1$ and $X^2$ are each independently a group represented by general formula (5) or (6):

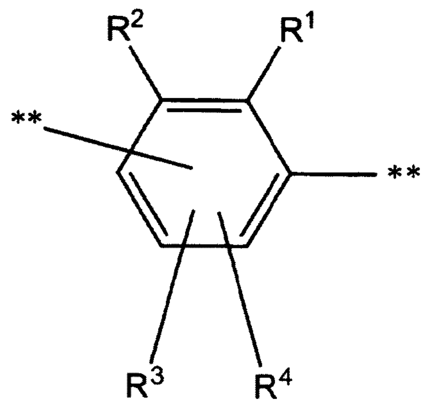

(5)

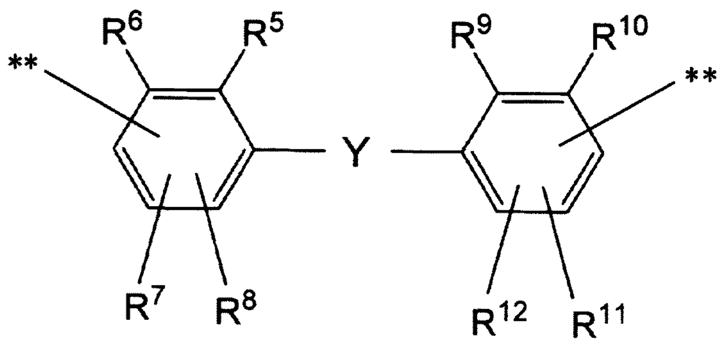

(6)

(where ** represents a site bonding to an oxygen atom, $R^1$ to $R^{12}$ each independently represent a hydrogen atom, an alkyl group having from 1 to 10 carbon atoms, an alkoxy group having from 1 to 10 carbon atoms, or a halogen atom, and Y represents a single bond, an oxygen atom, a sulfur atom, a sulfonyl group, or an unsubstituted or substituted alkanediyl group).

**7.** The compound according to claim 5, wherein X is a group represented by general formula (6), and Y is a single bond.

**8.** The compound according to claim 6, wherein $X^1$ and $X^2$ are each independently a group represented by general formula (6), and Y is a single bond.

**9.** An additive for synthetic resins comprising the compound according to any one of claims 1 to 8.

**10.** The additive for synthetic resins according to claim 9 comprising a nucleating agent.

**11.** An additive composition for synthetic resins comprising the additive for synthetic resins according to claim 9 or 10.

**12.** The additive composition for synthetic resins according to claim 11 comprising another nucleating agent in addition to the additive agent for synthetic resins.

**13.** The additive composition for synthetic resins according to claim 12, wherein the other nucleating agent contains a compound represented by general formula (7):

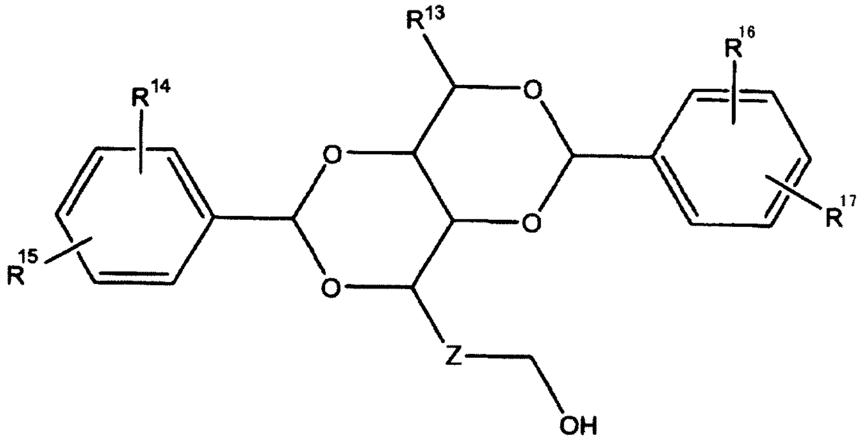

(7)

(where $R^{13}$ represents a hydrogen atom or an alkyl group having from 1 to 10 carbon atoms, $R^{14}$ to $R^{17}$ each independently represent a hydrogen atom, a halogen atom, a cyano group, an alkyl group having from 1 to 10 carbon atoms, or an alkoxy group having from 1 to 10 carbon atoms, or $R^{14}$ and $R^{15}$ or $R^{16}$ and $R^{17}$ are linked together to represent an alkylene group having from 3 to 6 carbon atoms or an alkylenedioxy group having from 1 to 4 carbon atoms, and Z represents a single bond, a -CH(OH)- group, or a -CH(OH)CH(OH)- group.).

**14.** The additive composition for synthetic resins according to any one of claims 11 to 13 comprising a lubricant.

**15.** The additive composition for synthetic resins according to claim 14, wherein the lubricant contains at least one selected from the group consisting of a fatty acid ester and a fatty acid amide.

**16.** The additive composition for synthetic resins according to any one of claims 11 to 15 comprising a colorant.

**17.** A resin composition comprising a synthetic resin and the additive for synthetic resins according to claim 9 or 10.

**18.** A resin composition comprising a synthetic resin and the additive composition for synthetic resins according to any one of claims 11 to 16.

**19.** The resin composition according to claim 17 or 18, wherein the synthetic resin includes a polyolefin resin.

**20.** The resin composition according to claim 19, wherein the polyolefin resin includes at least one selected from the group consisting of a polyethylene resin and a polypropylene resin.

**21.** The resin composition according to any one of claims 17 to 20, wherein the synthetic resin includes an elastomer.

**22.** A molded article obtained by molding the resin composition according to any one of claims 17 to 21.

**23.** The molded article according to claim 22, wherein an a* value and a b* value of a transmitted color, when the molded article is measured with a colorimeter, satisfy-7 $\leq$ a* $\leq$ 1 and -1 $\leq$ b* $\leq$ 5.

**INTERNATIONAL SEARCH REPORT**

International application No.
**PCT/JP2021/030479**

**A. CLASSIFICATION OF SUBJECT MATTER**

***C08K 5/3492***(2006.01)i; ***C08L 23/00***(2006.01)i; ***C08L 101/00***(2006.01)i; ***C07D 251/30***(2006.01)i
FI: C07D251/30 CSP; C08L101/00; C08L23/00; C08K5/3492

According to International Patent Classification (IPC) or to both national classification and IPC

**B. FIELDS SEARCHED**

Minimum documentation searched (classification system followed by classification symbols)

C08K5/3492; C08L23/00; C08L101/00; C07D251/30

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 51-77700 A (BAYER AG) 06 July 1976 (1976-07-06) claims, example 6 | 1-8 |
| X | WO 2019/013261 A1 (FUJIFILM CORPORATION) 17 January 2019 (2019-01-17) claims, compounds C-53, 54, 55 | 1-6 |
| X | JP 63-43918 A (TORAY IND INC) 25 February 1988 (1988-02-25) claims, table 1 | 1-6, 9, 11, 17, 18, 22 |
| X | CN 104725665 A (CHINA THREE GORGES UNIVERSITY) 24 June 2015 (2015-06-24) claims, examples | 1-3, 9, 11, 17, 18, 22 |
| X | LO, W. et al. Tri-substituted s-triazines as mesogenic cores, Molecular Crystals and Liquid Crystals Science and Technology. Section A: Molecular Crystals and Liquid Crystals., 1997, vol. 308, no. 1, pp. 133-146 fig. 1, scheme 1 | 1, 5, 7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2021** | **19 October 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2021/030479**

**C. DOCUMENTS CONSIDERED TO BE RELEVANT**

| Category* | Citation of document, with indication, where appropriate, of the relevant passages | Relevant to claim No. |
|---|---|---|
| X | MORMANN, W. et al. Synthesis and mesomorphic properties of cyanurates and isocyanurates, Branched mesogens as model crosslinks for liquid crystalline thermosets. Liquid Crystals., 1995, vol. 19, no. 4, pp. 481-488<br>schemes 1, 2, tables 2, 3, compound 6c | 1, 5, 7 |
| X<br>Y | WO 2020/067144 A1 (ADEKA CORPORATION) 02 April 2020 (2020-04-02)<br>claims, paragraph [0067], compound no. 1-3, 24, 36-39, 43, 51-54, examples | 1, 5, 9-12, 14-23<br>12-23 |
| Y | JP 2018-95786 A (MITSUI CHEMICALS INC) 21 June 2018 (2018-06-21)<br>claims, paragraphs [0141], [0158], [0160], [0169] | 12-23 |
| Y | JP 2010-526175 A (DOW GLOBAL TECHNOLOGIES INC) 29 July 2010 (2010-07-29)<br>claims, paragraph [0129] | 12-23 |
| Y | JP 2009-256474 A (TOHO KK) 05 November 2009 (2009-11-05)<br>claims, paragraphs [0028], [0030] | 12-23 |

Form PCT/ISA/210 (second sheet) (January 2015)

**INTERNATIONAL SEARCH REPORT**
**Information on patent family members**

International application No.
**PCT/JP2021/030479**

| Patent document cited in search report | Publication date (day/month/year) | Patent family member(s) | Publication date (day/month/year) |
|---|---|---|---|
| JP 51-77700 A | 06 July 1976 | US 4042567 A<br>claims, example 6<br>GB 1505308 A<br>FR 2293453 A | |
| WO 2019/013261 A1 | 17 January 2019 | US 2020/0180957 A1<br>claims, compounds C-53, 54, 55<br>EP 3653572 A1<br>CN 110799455 A<br>KR 10-2020-0016946 A | |
| JP 63-43918 A | 25 February 1988 | (Family: none) | |
| CN 104725665 A | 24 June 2015 | (Family: none) | |
| WO 2020/067144 A1 | 02 April 2020 | EP 3858909 A1<br>claims, paragraph [0066], compounds no. 1-3, 24, 36-39, 44, 51-54, examples<br>CN 112839988 A | |
| JP 2018-95786 A | 21 June 2018 | (Family: none) | |
| JP 2010-526175 A | 29 July 2010 | US 2010/0152361 A1<br>claims, paragraph [0137]<br>WO 2008/134186 A1<br>EP 2147047 A1<br>CN 101679697 A | |
| JP 2009-256474 A | 05 November 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S6114261 A
- WO 2020067144 A
- JP 2010132927 A
- JP H0710989 A
- US 6552131 B1
- JP 2016023254 A